# EUROPEAN PATENT APPLICATION

(11) **EP 4 790 058 A1**
(43) Date of publication of application: **12.08.2026**
(21) Application number: 25305169.2
(22) Date of filing: 05.02.2025
(51) Int. Cl.: C07F 9/24, C07F 9/572, C07F 9/58, C07F 9/6506, C07F 9/6509, C07F 9/6533, A61P 31/18, A61P 35/00, C12N 15/88

(54) **NOVEL BRANCHED IONIZABLE PHOSPHOLIPIDS FOR NUCLEIC ACIDS DELIVERY**

(71) Applicant: Centre National de la Recherche Scientifique, 75016 Paris (FR); Université de Brest, 29200 Brest (FR); Oregon State University, Corvallis, OR 97333 (US)
(72) Inventor: JAFFRÈS, Paul-Alain, 29820 Bohars (FR); BERCHEL, Mathieu, 29460 Logonna-Daoulas (FR); LE ROUX, Chloé, 29200 Brest (FR); SAHAY, Gaurav, Portland, 97229 (US); JOZIC, Antony, West Linn, 97068 (US); KIM, Jeonghwan, 38541 Gyeongsan (KR)
(74) Representative: Icosa

(57) **Abstract**

The present invention relates to branched ionizable phospholipids. In particular, this invention relates to branched ionizable phospholipids of formula (I). This invention also relates to a process for manufacturing said compounds of formula (I). This invention also relates to lipid nanoparticles obtained by formulation of the compounds of general formula (I). This invention also relates to the use of the compounds of formula (I) and of lipid nanoparticles obtained by formulation of the compounds of general formula (I).

## Description

### FIELD OF INVENTION

The present invention relates to branched ionizable phospholipids. In particular, this invention relates to branched ionizable phospholipids of formula (I). This invention also relates to a process for manufacturing said compounds of formula (I). The present invention relates to the use of branched ionizable phospholipids to prepare nucleic acid-based lipid nano particles (LNP) of different composition. In particular, this invention relates to lipid nano particles, liposomes and lipoplexes obtained by formulation of the compounds of general formula (I). The present invention also relates to the use of branched ionizable phospholipid-based LNP for in vitro and in vivo delivery of nucleic acids. In particular, this invention relates to the use of the compounds of formula (I) and LNP obtained by formulation of the compounds of general formula (I).

### BACKGROUND OF INVENTION

The use of DNA and RNA as therapeutics offers promising development, but the difficulties of delivering these nucleic acids derivatives efficiently and selectively are still limiting factors.

Several methods have been investigated to carry nucleic acid constructs into cells, including the use of either modified virus or synthetic vectors as carriers, which are two possible approaches for both in vitro and in vivo applications, as reported by J.W.B. Bainbridge, C. Stephens, K. Parsley, C. Demaison, A. Half ard, A.J. Thrasher and R.R. Alii, Gene Ther. 2001 , 8, 1665-1668.

Other methods including physical methods, such as for example electroporation, as described in E. Neumann, M. Schaefer-Ridder, Y. Wang and P.H. Hofschneider, EMBO J, 1982, 1, 841-5, have been proposed for transfection but so far they are restricted to few in vivo applications (e.g. vaccination, as reported by C.J. Wu, S.C. Lee, H.W. Huang, M.H. Tao Vaccine, 2004, 22, 1457-1464).

Transfection with naked DNA may also be applicable, but this method is mainly limited to the transfection of muscle tissues (E. Gronevik, S. Tollefsen, L.I.B. Sikkeland, T. Haug, T.E. Tjelle, I. Mathiesen, J. Gene Med. 2003, 5, 909-917; S Braun Curr. Op. Mol. Therapeutics 2004 6:499-505).

Due to an easier production control associated with the absence of immune response, the use of synthetic vectors can be a promising method.

The term of synthetic vector is used to indicate a wide variety of synthetic structures including for instance cationic lipids, neutral lipids, ionizable lipids, cationic polymers, dendrimers or neutral polymers. More schematically, the synthetic vectors can be classified in two categories depending on their chemical structures, which can be polymer or lipid derivatives.

Among the lipid derivatives, a wide variety of cationic or neutral lipids have been reported, for example in A.D. Miller, Angew. Chem., Int. Ed. 1998, 37, 1768-1785, I. Tranchant et al., J. Gene Med. 2004, 6, S24-35, B. Martin et al. Curr. Pharm. Des. 2005, 11, 375-394, P. Midoux et al., Br. J. Pharmacol. 2009, 157, 166-178. A common feature between these synthetic vectors is the presence of two lipid chains (e.g. oleyl) or a cholesterol unit, a cationic or polycationic head and a linker between these two parts.

Cationic amphiphilic lipids constituent a vast class of vectors commonly used for the vectorisation of nucleic acids (pDNA, siRNA, mRNA) in vitro or in vivo.

Since the pioneering work of Felgner et al. (Felgner, P.L.G.; Gadek, T.R.; Holm, M.; Roman, R.; Chan, H.W.; Wenz, M.; Northrop, J.P.; Ringold, M.G.; Danielsen, M. Proc. Natl. Acad. Sci. U. S. A., 1987, 84, 7413-7417), efforts have been made to propose novel structures of cationic amphiphilic lipids that make it possible to improve the effectiveness of the transfection and to expand the knowledge of transfection mechanisms.

Transfection is carried out thanks to supramolecular aggregates formed by the association of a cationic amphiphilic lipid with DNA (lipoplexes). After the cellular internalization of these lipoplexes which is produced by endocytosis pathway, the release of the nucleic material from the endosomes to the cytosol is necessary in order to prevent degradation of the loaded material inside the lysosomes.

Different strategies based on a molecular approach have been explored to promote the destabilization of the endosomal membrane or act on the stability of lipoplexes after the cellular internalization thereof.

As such novel cationic amphiphilic lipids that can be protonated in the endosomes (proton sponge effect) or cleaved by an enzymatic or redox reaction in the cytosol have been proposed to destabilize the endosomal membrane.

Another strategy for improving the efficiency of transfection consists in improving the stability and the fusion properties of lipoplexes (a) Ewert, K.; Slack, N.L.; Ahmad, A.; Evans, H.M.; Lin, A.J.; Samuel, C.E.; Safinya, C.R. Curr Med Chem., 2004, 11, 133-49; b) Dan, N.; Danino, D. Adv Colloid Interface Sci., 2014, 205, 230-9). Work in particular has provided an improvement in transfections by associating co-lipids such as 1,2-dioleoyl-sn-glycero-3-phosphoetanolamine (DOPE) with a cationic amphiphilic lipid. This improvement is attributed to the propensity of DOPE to adopt a reversed hexagonal phase which is known to be more fusogenic than the lamellar phases.

Another strategy for producing non-lamellar phase consists in acting on the molecular form of cationic amphiphilic lipids. Ewert et al. reported the synthesis of cationic amphiphilic lipids having a dendritic head group (Ewert, K.K.; Evans, H.M.; Zidovska, A.; Bouxsein N.F.; Ahmad, A.; Safinya, C.R. J. Am. Chem. Soc. 2006, 128, 3998-4006). The shape of this cationic polar head induced the formation of hexagonal phases H₁ when they are included in a binary formulation. High transfection efficiencies were observed on cell lines known to be difficult to transfect. Lindberg et al have shown that the incorporation of two phytanyl chains (methylated C16-alkyl chains) into the cationic lipo-phosphoramidate structure produces a reversed hexagonal phase after the formulation in water (Lindberg, M.; Carmoy, N.; Le Gall, T.; Fraix, A.; Berchel, M.; Lorilleux, C.; Couthon-Gourvès, H.; Bellaud, P.; Fautrel, A.; Jaffrès, P.A.; Lehn, P.; Montier, T. Biomaterials 2012, 33, 6240-6253). Good in vivo transfection efficiencies were obtained with this vector.

Despite all this work there is still a real need for developing novel lipids for nucleic acid delivery.

Regardless the kind of structure of the synthetic vectors, there is a need, on the one side, of ensuring a sufficiently low toxicity, which has led chemists to use natural resources for the design of low toxic synthetic vectors. On the other side, there is a need of tailoring a composition of formulations to be used for nucleic acid delivery, because this has an impact on the fusogenic character of the nano-objects, i.e. of liposomes, lipoplexes and LNPs and thus on the transfection efficacy.

One of the objectives of the present disclosure is therefore to provide synthetic vectors for nucleic acid delivery which can be readily and large-scale produced and easily administrated.

Surprisingly, the inventors have evidenced that these new compounds, i.e. phospholipids containing a protonable nitrogen atom including lipid derivatives featuring a branched structure, are low toxic synthetic vectors and improves the effectiveness of the transfection, the protonation capacity of the lipids being a key element of their efficacies. Moreover, the molecular platform developed is versatile, i.e. facility to modulate the structure of the hydrophobic domain, the structure of the polar head group, and the structure of the phosphorus function, and requires a limited number of steps for the synthesis of the final compounds. This methodology allows the synthesis of branched ionizable phospholipids. Branched phospholipids are molecules possessing a phosphorus function and a shape featuring ramification resulting from the presence of branched lipid chains.

### SUMMARY

This invention thus relates to compound of Formula (I), 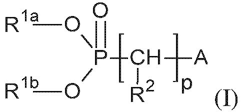 wherein **R^{1a}**, **R^{1b}**, **R²,** p and A are as defined in the claims and hereafter.

Another aspect of the invention is directed to a process for manufacturing a compound of formula (I) as defined above:
comprising the following step:
Reacting a compound of formula (II) : 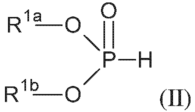
wherein **R^{1a}** and **R^{1b}** are as defined in the claims and hereafter with a compound of formula (III) 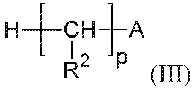
wherein **R², p** and **A** are as defined in the claims and hereafter
   in the presence of an amine.

The invention further relates to lipid nanoparticle (LNP) comprising at least one of the compounds according to the invention.

The invention further relates to a process for manufacturing the lipid nanoparticle according to the invention comprising a step of mixing a nucleic acid sequence or a mixture of nucleic acid sequence and a compound of formulae (I) according to the invention.

The invention further relates to a pharmaceutical composition comprising a compound of formulae (I) according to the invention or a lipid nanoparticle according to the invention and a physiologically acceptable vehicle.

Use of a compound of formulae (I) according to the invention or a lipid nanoparticle according to the invention for transfection *in vitro.*

Compound of formulae (I) according to the invention for the use thereof in transfection *in vivo,* for gene editing, base editing, prime editing, gene therapy, vaccination, cancer immunotherapy, topical treatments, ocular treatment or bactericidal activities.

Lipid nanoparticle according to the invention for the use thereof in transfection *in vivo,* for gene editing, base editing, gene therapy, vaccination, cancer immunotherapy, topical treatments, bactericidal activities, ex-vivo cell transfection or transfection of organoids, ex-vivo and in vitro transfection.

Pharmaceutical composition according to the invention for use in the prevention, treatment or amelioration of viral infection (e.g. Zika, HIV, Cytomegalovirus, rabies virus, COVID, influenza, respiratory syncytial virus, zoster virus), genetic disease including rare diseases, cancer (e.g. melanoma, brain cancer, lung cancer, liver cancer, pancreas cancer, prostate cancer, blood system cancer, ovarian cancer, breast cancer, digestive cancer, bone cancer), immunological diseases, metabolic diseases, ocular diseases.

### DEFINITIONS

In the present invention, the following terms have the following meanings.

### Chemical definitions

Where chemical substituents are combinations of chemical groups, the point of attachment of the substituent to the molecule is by the last chemical group recited. For example, an arylalkyl substituent is linked to the rest of the molecule through the alkyl moiety and it may by represented as follows: "aryl-alkyl-".

**"Alkoxy"** refers to any -O-alkyl. Generally, alkoxy groups of this invention are - O-alkyl groups.

**"Alkyl",** by itself or as part of another group, refers to a hydrocarbyl radical of formula CₙH₂ₙ₊₁ wherein n is a number greater than or equal to 1. Generally, alkyl groups of this invention comprise from 1 to 30 carbon atoms. Alkyl groups may be linear or branched and may be substituted as indicated herein. Non-limiting examples of alkyl groups include methyl, ethyl, propyl (*n*-propyl, *i*-propyl), butyl (*n*-butyl, *i*-butyl, *s*-butyl and t-butyl), pentyl and its isomers (*e.g., n*-pentyl, *iso*-pentyl), and hexyl and its isomers (*e.g., n*-hexyl, *iso-*hexyl).

**"Amino"** refers to -NH₂ group.

**"Aryl"** refers to a polyunsaturated, aromatic hydrocarbyl group having a single ring (*i.e.,* phenyl) or multiple aromatic rings fused together (*e.g.,* naphthyl) or linked covalently, typically containing from 5 to 12 carbon atoms, preferably from 6 to 10 carbon atoms, wherein at least one ring is aromatic. The aromatic ring may optionally include one to two additional rings (either cycloalkyl, heterocycloalkyl or heteroaryl) fused thereto. Aryl is also intended to include the partially hydrogenated derivatives of the carbocyclic systems enumerated herein, as long as at least one ring is aromatic. Non-limiting examples of aryl include phenyl, biphenyl, biphenylenyl, 5- or 6-tetralinyl, naphthalen-1- or -2-yl, 4-, 5-, 6 or 7-indenyl, 1- 2-, 3-, 4- or 5-acenaphthylenyl, 3-, 4- or 5-acenaphthenyl, 1- or 2-pentalenyl, 4- or 5-indanyl, 5-, 6-, 7- or 8-tetrahydronaphthyl, 1,2,3,4-tetrahydronaphthyl, 1,4-dihydronaphthyl, and 1-, 2-, 3-, 4- or 5-pyrenyl.

**"Cycloalkyl"** refers to a cyclic alkyl group, *i.e.,* a monovalent, saturated, or unsaturated hydrocarbyl group having 1 or 2 cyclic structures. Cycloalkyl includes monocyclic or bicyclic hydrocarbyl groups. Cycloalkyl groups may comprise 3 or more carbon atoms in the ring and generally, cycloalkyl groups of this invention comprise from 3 to 10, preferably from 3 to 8 carbon atoms, more preferably from 3 to 6 carbon atoms. This definition of "cycloalkyl" encompasses polycyclic cycloalkyls (*e.g*., bicycles) and bridged cycloalkyl structures, including cycles bound together through one atom ("spiro") or through two atoms. Non-limiting examples of cycloalkyl groups include cyclopropyl, cyclobutyl, cyclopentyl, cyclohexyl, cycoheptyl, cyclooctanyl, cyclononanyl, cyclodecanyl, norbornyl, adamantyl, bicyclo[2.2.2]octanyl, bicyclo[4.4.0]decanyl, bicyclo[3.2.1]octanyl, bicyclo[3.3.1]nonanyl, bicyclo[2.1.1]hexane, 2,3-dihydro-1H-indenyl, 1,2,3,4-tetrahydronaphthalenyl, decahydronaphthalenyl, 1,2,3,4-tetrahydronaphthalenyl, and octahydropentalenyl.

"**Cₓ-C_{y}**" or "(**Cₓ-C_{y}**)" preceding the name of a group means that the group comprises from x to y carbon atoms, in accordance to common terminology in the chemistry field.

**"Heteroalkyl"** refers to an alkyl group wherein one or more carbon atoms are replaced by a heteroatom, for example an oxygen, nitrogen or sulfur atom, and wherein the resulting heteroalkyl group comprises at least one carbon atom. In heteroalkyl groups, the heteroatoms are bound along the alkyl chain only to carbon atoms, *i.e.,* each heteroatom is separated from any other heteroatom by at least one carbon atom, typically by at least two carbon atoms. The nitrogen and sulfur heteroatoms may optionally be oxidized and the nitrogen heteroatoms may optionally be quaternarized (*e.g*., sulfur may be oxidized as SO or SO₂). Heteroalkyl groups may further include one or more =O and/or =S groups. In one embodiment, at least two carbon atoms are replaced by a heteroatom. In one embodiment, the heteroalkyl is bound to another group or molecule through a carbon atom, *i.e.,* the binding atom is not selected among the heteroatoms included therein. In one embodiment, the heteroalkyl is bound to another group or molecule through one of the heteroatoms included therein. When substituted by one or more other group(s), an heteroalkyl may be substituted either through a carbon atom or through a heteroatom (*e.g*., nitrogen), unless otherwise specified. Non-limiting examples of heteroalkyl include alkoxy, ethers and polyethers (*e.g*., polyethylene glycol), secondary and tertiary amines and polyamines, thioethers and polythioethers, and combinations thereof.

**"Heterocyclyl"** refer(s) to non-aromatic, fully saturated or partially unsaturated cyclic groups (for example, 3- to 7-membered monocyclic, 7-to 11-membered bicyclic, or containing a total of 3 to 10 ring atoms) which have at least one heteroatom in at least one carbon atom-containing ring. Heterocyclic groups may in particular be 3- to 7-membered, preferably 5- or 6-membered. Heterocyclic groups may in particular be monocyclic or bicyclic, preferably monocyclic. Each ring of the heterocyclic group containing a heteroatom may have 1, 2, 3 or 4 heteroatoms selected from nitrogen atoms, oxygen atoms and/or sulfur atoms. The heterocyclic group may be attached at any heteroatom or carbon atom of the ring or ring system, where valence allows. The rings of multi-ring heterocyclic groups may be fused, bridged and/or joined through one or more spiro atoms. The heterocyclic group may optionally be substituted by one or more substituent(s) (for example 1 to 4 substituent(s), or for example 1, 2, 3 or 4 substituent(s)), which are for example selected from oxo, halogen, hydroxyl, nitro, amino, cyano, alkyl, alkylamino, dialkylamino, alkoxy, haloalkyl, acyl, carbamoyl, alkylsulfoxide, sulfamoyl, alkylthio, carboxyl, and the like. Non-limiting examples of heterocyclic groups include aziridinyl, oxiranyl, thiiranyl, piperidinyl, azetidinyl, 2-imidazolinyl, pyrazolidinyl imidazolidinyl, isoxazolinyl, oxazolidinyl, isoxazolidinyl, thiazolidinyl, isothiazolidinyl, piperidinyl, succinimidyl, *3H*-indolyl, indolinyl, isoindolinyl, *2H*-pyrrolyl, 1-pyrrolinyl, 2-pyrrolinyl, 3-pyrrolinyl, pyrrolidinyl, *4H*-quinolizinyl, 2-oxopiperazinyl, piperazinyl, homopiperazinyl, 2-pyrazolinyl, 3-pyrazolinyl, tetrahydro-2*H*-pyranyl, 2*H*-pyranyl, *4H-*pyranyl, 3,4-dihydro-*2H*-pyranyl, oxetanyl, thietanyl, 3-dioxolanyl, 1,4-dioxanyl, 2,5-dioximidazolidinyl, 2-oxopiperidinyl, 2-oxopyrrolodinyl, indolinyl, tetrahydropyranyl, tetrahydrofuranyl, tetrahydrothiophenyl, tetrahydroquinolinyl, tetrahydroisoquinolinyl (*e.g*., tetrahydroisoquinolin-1-yl, tetrahydroisoquinolin-2-yl, tetrahydroisoquinolin-3-yl or tetrahydroisoquinolin-4-yl), thiomorpholinyl (*e.g.,* thiomorpholin-4-yl), thiomorpholin-4-ylsulfoxide, thiomorpholin-4-ylsulfone, 1,3-dioxolanyl, 1,4-oxathianyl, 1,4-dithianyl, 1,3,5-trioxanyl, *1H*-pyrrolizinyl, tetrahydro-1,1-dioxothiophenyl, N-formylpiperazinyl, and morpholinyl (*e.g*., morpholin-4-yl).

**"Hydroxyl"** refers to -OH group.

Where at least one carbon atom in an aryl group is replaced with a heteroatom, the resultant ring is referred to herein as a **heteroaryl ring.**

**"Heteroaryl"** as used herein by itself or as part of another group refers but is not limited to 5 to 12 carbon-atom aromatic rings or ring systems containing 1 to 2 rings which are fused together or linked covalently, typically containing 5 to 6 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2, 1 -b] [ 1 ,3] thiazolyl, thieno [3 ,2-b] furanyl, thieno [3 ,2-b] thiophenyl, thieno[2,3-d][l,3]thiazolyl, thieno[2,3-d]imidazolyl, tetrazolo[l,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2- benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1 ,2,3-benzothiadiazolyl, 2, 1 ,3-benzothiadiazolyl, thienopyridinyl, purinyl, imidazo[l,2-a]pyridinyl, 6-oxo-pyridazin-l(6H)-yl, 2- oxopyridin-l(2H)-yl, 6-oxo-pyridazin-l(6H)-yl, 2-oxopyridin-l(2H)-yl, 1,3- benzodioxolyl, quinolinyl, isoquinolinyl, cinnolinyl, quinazolinyl, quinoxalinyl.

The alkyl, aryl or any other group defined hereabove can be optionally substituted, meaning that said group can be substituted, or not, by one or more substituents chosen independently of one another, among linear or branched alkyl group, linear or branched heteroalkyl group, cycloalkyl group, heterocycloalkyl group, aryl group, heteroaryl group, hydroxy group (-OH), amino group (-NH₂), a halogen atom, a cyano group, -COOH, -PO₃H₂, -PO₃RR', -NRR', -COOR, -CONRR'; where R and R' being each independently selected from H, alkyl or aryl or heteroaryl, or heterocyclyl

### Biological definitions

**"barcoding"** or **"DNA barcoding":** relates to a method that uses delivery of a short genetic marker which can act as a marker for delivery of lipid-based nanoparticles, include lipoplex, liposomes, or lipid nanoparticles.

**"Lipoplex":** relates to a nucleic acid - liposome complex.

**"Liposomal nanoparticle":** relates to an artificial vesicle formed by concentric lipid bilayers, trapping aqueous compartments between them.

**"Lipid nanoparticles (LNP)"** relates to supramolecular aggregates including nucleic acid, ionizable lipid, PEG lipids and possibly the addition of helper lipid, sterol and targeting motif.

**"Ionizable lipid"** relates to lipid functionalized by one or several function that can be protonated. In one embodiment the protonable function occurs at a pH ranging from pH = 6 to pH = 7.

**"Branched ionizable lipid"** relates to ionizable lipid that present in the hydrophobic domain a ramification that comes from a ramified carbon chains.

**"branched ionizable phospholipid"** relates to branched ionizable lipids possessing at least one phosphorus function.

**"PEG Lipid"** relates to compounds featuring one or several lipid chains and one or several PEG chains. The PEG chains is a repetition of ethyleneoxide of different length corresponding, for this PEG motif, to a molecular weight ranging from 200 to 5000 g.mol⁻¹.

**"Helper lipid"** relates to any kind of lipid derivatives or amphiphilic compounds that can improve either the stability of the LNP or their efficacy of transfection.

**"Sterol"** relates to a class of lipid compounds featuring a central core composed of sterane with an alcohol function in position 3.

**"Targeting motif**" relates to a molecular structure that contribute to favour the distribution of the LNP selectively in one organ or tissues.

**"Amphiphilic compound"** relates to compounds featuring a hydrophobic moiety covalently linked a polar moiety.

**"Nucleic acid"** or **"polynucleotide"** refers to any polyribonucleotide or polydeoxyribonucleotide, which may be unmodified RNA or DNA or modified RNA or DNA. "Nucleic acid" or "Polynucleotides" include, without limitation single-and double-stranded DNA, DNA that is a mixture of single- and double-stranded regions, single- and double- stranded RNA, and RNA that is a mixture of single- and double-stranded regions, hybrid molecules comprising DNA and RNA that may be single-stranded or, more typically, double-stranded or a mixture of single- and double-stranded regions.

**"mRNA",** stands for "messenger ribonucleic acid", and refers to a single-stranded molecule of RNA that corresponds to the genetic sequence of a gene, and is read by a ribosome in the process of synthesizing a protein.

**"siRNA",** stands for "small interfering ribonucleic acid" or "silencing ribonucleic acid", and refers to a synthetic RNA duplex (generally between 18-30 base pairs) designed to specifically target a particular mRNA for degradation.

**"saRNA"** : stands for self-amplifying RNA.

**"ShRNA"** stands for short hairpin RNA.

**"miRNA":** stands for micro-RNA it is usually a 18 to 25 nucleotides and can regulate gene expression.

**"Guide RNA"** or **"gRNA"** refers to any nucleic acid that promotes the specific association (or "targeting") of an RNA-guided nuclease (such as, for example, Cas9) to a target sequence (*e.g*., a genomic or episomal sequence) in a cell. A gRNA comprises a first part capable of binding the RNA-guided nuclease and a second part, that contains a sequence that may be referred to as a "spacer", capable of binding to the target sequence to be modified, and typically located at the 5' terminus of the gRNA.

**"pDNA",** stands for "plasmid deoxyribonucleic acid" or "plasmid" and refers to a small extra-genomic DNA molecule, most commonly found as circular double stranded DNA molecules that may be used as a cloning vector in molecular biology, to make and/or modify copies of DNA fragments up to about 15 kb (i.e., 15,000 base pairs). Plasmids may also be used as expression vectors to produce large amounts of proteins of interest encoded by a nucleic acid sequence found in the plasmid downstream of a promoter sequence.

**"Organoid",** refers to a miniaturized and simplified version of an organ produced in vitro in three dimensions that mimics the key functional, structural, and biological complexity of that organ.

**"HITI",** stands for "Homology-independent targeted integration" and refers to an alternative method of knocking down DNA that exploits the highly efficient non-homologous end-joining pathway.

**"CRISPR-Cas system",** wherein "CRISPR" stands for "clustered regularly interspaced short palindromic repeats", refers to an engineered system that contains two components: a guide RNA (gRNA) and a CRISPR-associated endonuclease (Cas protein). The CRISPR-Cas system is routinely used to perform genome editing enabling site-specific alterations in a variety of organisms and cellular contexts. The genome editing by the CRISPR-Cas9 system entails three steps: (1) scanning of the genome by the RNA-guided Cas9 nuclease to find the DNA sequence complementary to the gRNA, (2) creation of a DNA double-strand break (DSB) by Cas9, and (3) repair of the lesion by the endogenous DNA repair machinery. The induced DSB is repaired through DNA repair machinery, such as, for example, the efficient but error-prone non-homologous end joining (NHEJ) or the less efficient but high-fidelity homology-directed repair for gene modifications (HDR), and the like. The CRISPR-Cas system may be used to generate insertions or deletions (termed Indels) or frameshift mutations due to error-prone repair pathways. The CRISPR-Cas system may also be used to silence or mutate a gene. The CRISPR-Cas system may furthermore be used to produce knock-out (KO) mutants. Moreover, the CRISPR-Cas system may be used to perform base editing and prime editing, thereby generating precise and on demand nucleotide conversion, allowing, for example, fine-tuning of protein function and generating gain-of-function mutants. The CRISPR-Cas system may be delivered through different Cas and gRNA formats, for example selected in the list comprising, but not limited to, plasmid DNA (pDNA), RNA or proteins (e.g., Cas ribonucleoproteins (RNPs)), and the like.

**"Cas"** stands for "CRISPR-associated protein" and relates in particular to a CRISPR-associated endonuclease, *i.e.,* an enzyme that cleaves both strands of DNA molecule. The Cas proteins can comprise (i) an active DNA cleavage domain and (ii) a guide RNA binding domain. As part of the CRISPR-Cas system, the Cas protein is guided to its specific site of action (*i.e.,* to the target DNA sequence) by a gRNA. The Cas may originate from different protein families selected in the list comprising, but not limited to, Cas3, Cas7, Cas8, Cas9, Cas10, Cas11, Cas12, and Cas14. In some specific embodiments, the present invention relates to Cas9. Classically, a Cas9 comprises two nuclease domains, namely the HNH and the RuvC, each of them cleaving a DNA strand.

**"Transfection"** refers to a process by which exogenous nucleic acid and/or protein are transferred or introduced into a host cell, in particular a host cell of eukaryotic origin. A "transfected" host cell is one which has been manipulated so as to incorporate the exogenous nucleic acid or protein. The cell includes the primary subject cell and its progeny.

### General definitions

**"About"** is used herein to mean approximately, roughly, around, or in the region of. The term "about" preceding a figure means plus or less 10 % of the value of the figure. When the term "about" is used in conjunction with a numerical range, it modifies that range by extending the boundaries above and below the numerical values set forth by 10%.

**"at least"** is equivalent to the expression "one or more".

**"Comprising"** or **"comprise"** is to be construed in an open, inclusive sense, but not limited to.

**"From X to Y"** refers to the range of values between X and Y, the limits X and Y being included in said range.

**"Administration",** or a variant thereof (*e.g*., **"administering"**), means providing the active agent or active ingredient, alone or as part of a pharmaceutically acceptable composition, to the patient in whom/which the condition, symptom, or disease is to be treated or prevented. The active agent of active ingredient is the compound of the invention

**"Human"** refers to a subject of both genders and at any stage of development (*i.e.,* neonate, infant, juvenile, adolescent, adult).

**"Patient"** refers to a warm-blooded animal, more preferably a human, who/which is awaiting the receipt of, or is receiving medical care or is/will be the object of a medical procedure.

**"Pharmaceutically acceptable carrier"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA.

By **"pharmaceutically acceptable"** is meant that the ingredients of a pharmaceutical composition are compatible with each other and not deleterious to the patient thereof.

**"Pharmaceutical vehicle"** refers to a carrier or inert medium used as solvent or diluent in which the pharmaceutically active agent is formulated and/or administered. Non-limiting examples of pharmaceutical vehicles include creams, gels, lotions, solutions and liposomes.

**"Therapeutically effective amount"** (or more simply an **"effective amount")** refers to the amount of active agent or active ingredient that is sufficient to achieve the desired therapeutic or prophylactic effect in the patient to which/whom it is administered.

The terms **"treat", "treating"** and **"treatment",** as used herein, are meant to include alleviating, attenuating or abrogating a condition or disease and/or its attendant symptoms.

The terms **"prevent", "preventing"** and **"prevention",** as used herein, refer to a method of delaying or precluding the onset of a condition or disease and/or its attendant symptoms, barring a patient from acquiring a condition or disease, or reducing a patient's risk of acquiring a condition or disease.

### DETAILED DESCRIPTION

### Compound

### [General formula]

This invention relates to a compound of Formula (I) 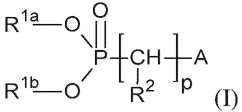 wherein:
**R^{1a}** and **R^{1b}** identical or different, represent, independently from one another, a branched or linear saturated C₅-C₃₅ alkyl chain, optionally substituted by a cyclic or acyclic C₁-C₆ alkyl or by an ester group of formula (C6-C₁₄)-O-C(O)- provided that at least one of **R^{1a}** and **R^{1b}** is a branched saturated C₅-C₃₅ alkyl chain;
**R²** represents H, aryl group, heteroaryl group, heterocyclyl group or hydroxyl group;
**p** represents an integer ranging from 0 to 1;
**A** represent -N(R³)(R⁴), -OR⁵ or an heteroaryl group comprising a nitrogen atom;
**R³** represent a hydrogen atom, unsubstituted or substituted alkyl group, or an unsubstituted or substituted aminoalkyl group;
**R⁴** represent a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- or -O-, and terminated by a group selected from substituted or unsubstituted amine group, substituted or unsubstituted heteroaryl group, substituted or unsubstituted heterocyclyl group and phosphoramidate of formula 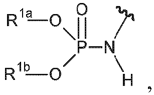 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl; and
**R⁵** represents a ionizable group selected from substituted or unsubstituted heterocyclyl group comprising a nitrogen atom, substituted or unsubstituted heteroaryl group comprising a nitrogen atom, substituted or unsubstituted aryl group, or a linear or branched saturated hydrocarbon chain, the hydrocarbon chain being optionally interrupted by one or more heteroatoms chosen from N, S or O, and terminated by a group selected from substituted or unsubstituted amine group, substituted or unsubstituted heteroaryl group, and substituted or unsubstituted heterocyclyl group.

According to one embodiment, **R^{1a}** and **R^{1b}** identical or different, represent, independently from one another, a branched or linear saturated C₅-C₃₅ alkyl chain, optionally substituted by a cyclic or acyclic C₁-C₆ alkyl. According to one embodiment, **R^{1a}** and **R^{1b}** identical or different, represent, independently from one another, a branched saturated C₅-C₃₅ alkyl chain, optionally substituted by a cyclic or acyclic C₁-C₆ alkyl, preferably a branched saturated C₁₀-C₃₅ alkyl chain, optionally substituted by a cyclic or acyclic C₁-C₆ alkyl, more preferably a branched saturated C₁₄-C₂₈ alkyl chain, optionally substituted by a cyclic or acyclic C₁-C₆ alkyl. According to one embodiment, **R^{1a}** and **R^{1b}** are the same. According to one embodiment, **R^{1a}** and **R^{1b}** are different.

According to a preferred embodiment, **R^{1a}** and **R^{1b}**, identical or different, are independently selected from

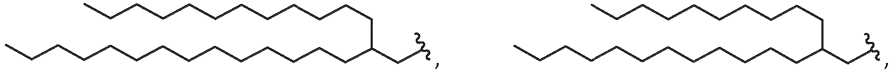

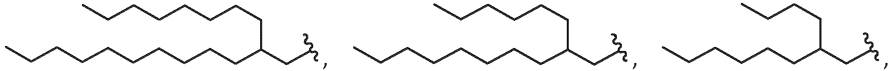

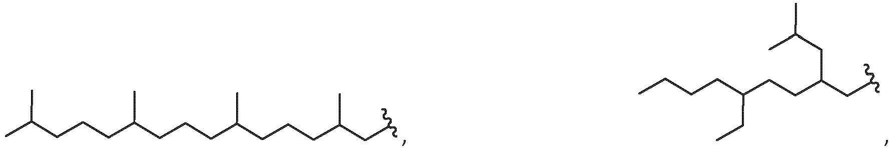

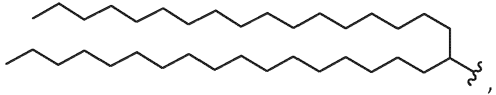

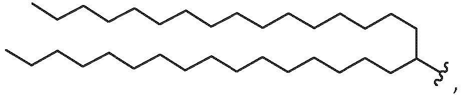

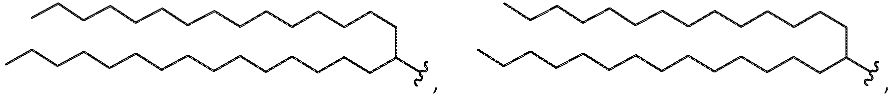

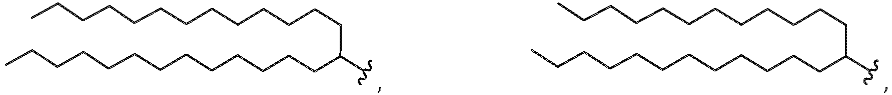

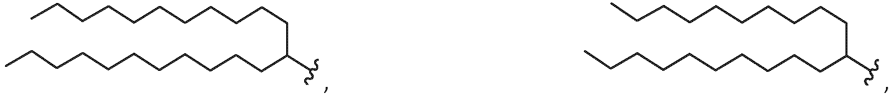

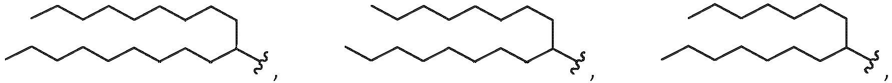

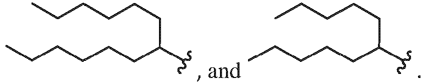

According to a preferred embodiment, **R^{1a}** and **R^{1b}**, identical or different, are independently selected from

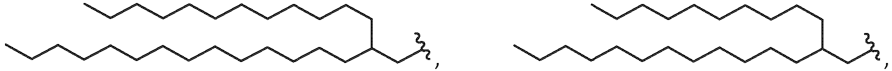

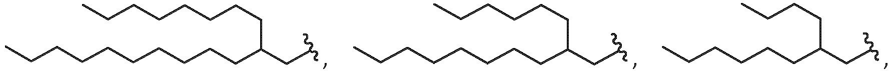

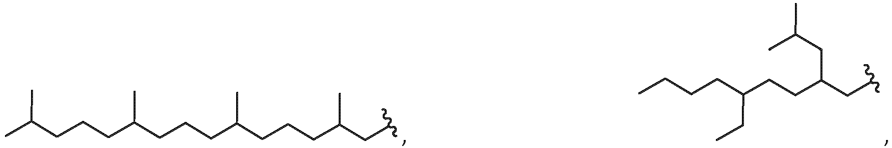

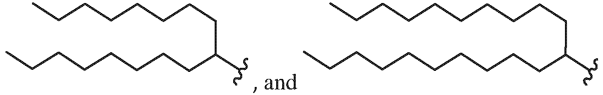

According to one embodiment, **R²** represents H, OH, phenyl group or imidazole.

According to one embodiment, **p** represents 1. According to another embodiment, **p** represents 0.

According to one embodiment, **A** represents -N(R³)(R⁴) or - OR⁵. According to one embodiment, **A** represents -N(R³)(R⁴). According to one embodiment, **A** represents -OR⁵.

According to one embodiment, **A** is selected from 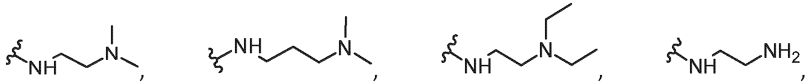 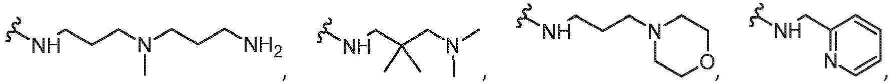 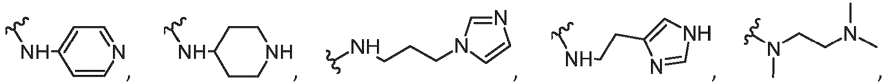 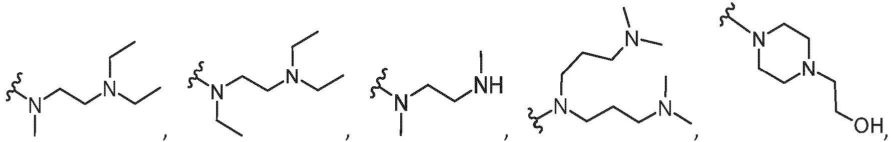 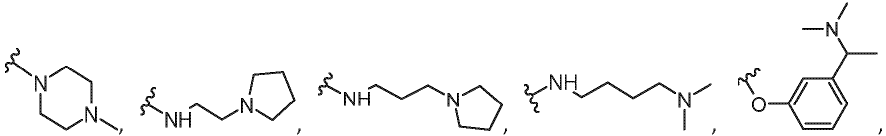 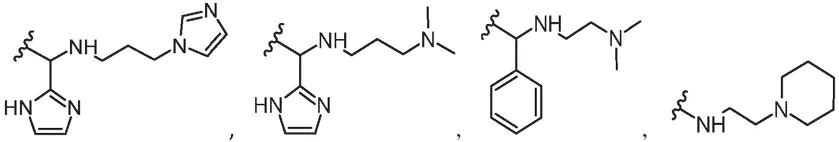 and

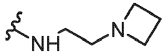

According to one embodiment, A is selected from

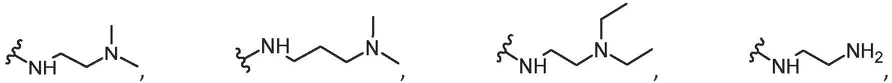

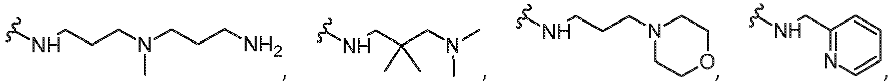

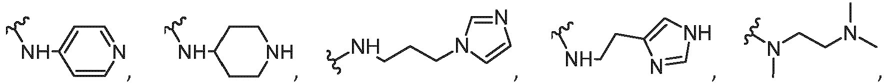

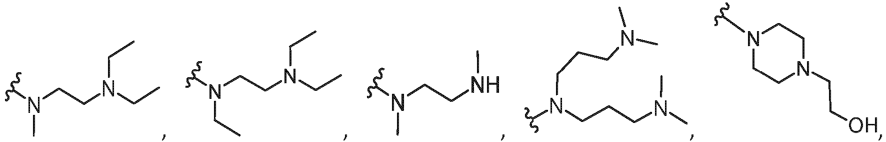

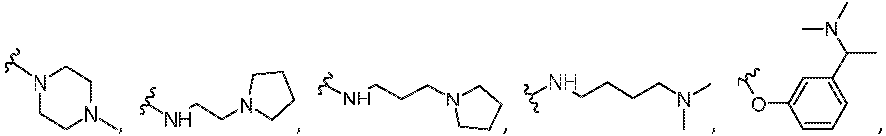

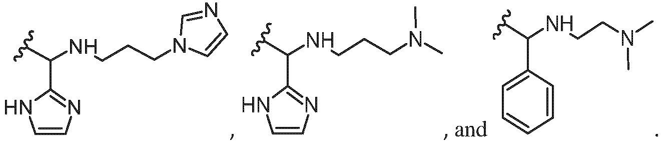

In one embodiment, **R³** represents hydrogen atom, alkyl group, or a substituted aminoalkyl group. In one embodiment, **R³** represents hydrogen atom, methyl, ethyl, or dimethylaminopropyl.

According to one embodiment, **R⁴** represents a ionizable group selected from unsubstituted heterocyclyl group comprising a nitrogen atom, substituted heteroaryl group comprising a nitrogen atom, and a linear or branched saturated hydrocarbon chain, the hydrocarbon chain being optionally interrupted by -N(R)-, and terminated by a group selected from substituted or unsubstituted amine group, unsubstituted heteroaryl group, unsubstituted heterocyclyl group and phosphoramidate of formula 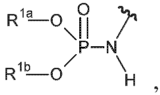 **R** being an alkyl group.

According to one embodiment, **R⁴** represents a ionizable group selected from unsubstituted heterocyclyl group comprising a nitrogen atom, substituted heteroaryl group comprising a nitrogen atom, and a linear or branched saturated hydrocarbon chain, the hydrocarbon chain being optionally interrupted by -N(R)-, and terminated by a group selected from substituted or unsubstituted amine group, unsubstituted heteroaryl group, unsubstituted heterocyclyl group and phosphoramidate of formula 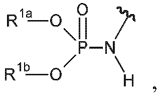 **R** being a methyl.

According to one embodiment, **R⁴** represents a ionizable group selected from unsubstituted heterocyclyl group comprising a nitrogen atom, substituted heteroaryl group comprising a nitrogen atom, and a linear or branched saturated hydrocarbon chain, the hydrocarbon chain being optionally interrupted by -N(R)-, and terminated by a group selected from substituted or unsubstituted amine group, unsubstituted heteroaryl group, unsubstituted heterocyclyl group, **R** being an alkyl group. According to one embodiment **R⁴** is selected from

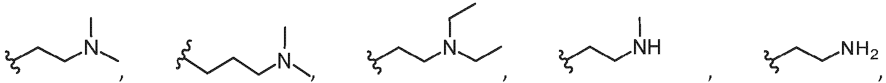

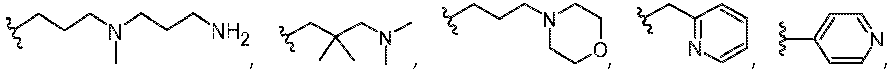

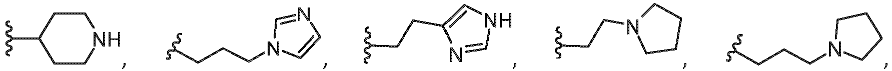

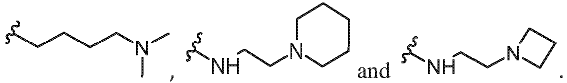

According to one embodiment **R⁴** is selected from 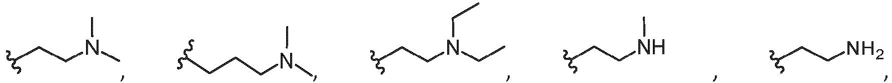 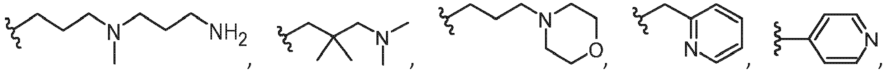 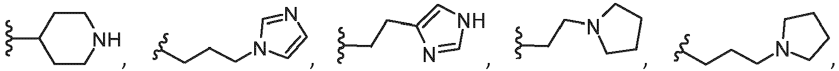 and

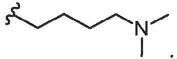

According to one embodiment, **R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl. According to one embodiment, **R³** and **R⁴** represent together, with the nitrogen atom which carries them, a heterocyclyl group, said heterocyclyl group being interrupted by N and optionally substituted by one or more groups chosen from alkyl, hydroxyalkyl. According to one embodiment, **R³** and **R⁴** represent together, with the nitrogen atom which carries them, a heterocyclyl group, said heterocyclyl group being interrupted by N and substituted by one or more groups chosen from alkyl, hydroxyalkyl. According to one embodiment, **R³** and **R⁴** represent together, with the nitrogen atom which carries them, a heterocyclyl group, said heterocyclyl group being interrupted by N and substituted by one or more groups chosen from methyl, hydroxyethyl.

According to one embodiment, **R³** and **R⁴** represent together with the nitrogen atom which carries them, a piperazine group substituted by a methyl or hydroxyethyl group.

According to one embodiment, **R⁵** represent a ionizable group selected from substituted or unsubstituted heterocyclyl group comprising a nitrogen atom, substituted or unsubstituted heteroaryl group comprising a nitrogen atom, substituted or unsubstituted aryl group, or a linear or branched saturated hydrocarbon chain, the hydrocarbon chain being optionally interrupted by one or more heteroatoms chosen from N, S or O, and terminated by a group selected from substituted or unsubstituted amine group, substituted or unsubstituted heteroaryl group, and substituted or unsubstituted heterocyclyl group.

According to one embodiment, **R⁵** is selected from

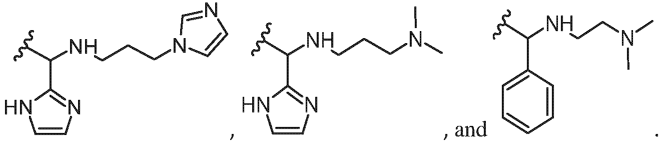

According to a preferred embodiment, the compound of the invention is a compound of Formula (I-A), 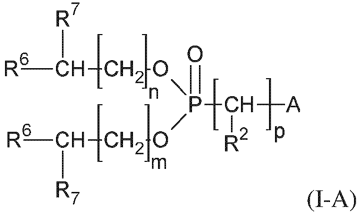 wherein:
**R⁶** and **R⁷**, identical or different, represent, independently from one another, a linear or branched, saturated, C₁-C₁₅ alkyl chain, optionally substituted by a cyclic or acyclic C₁-C₆ alkyl or by an ester group of formula (C₆-C₁₄)-O-C(O)-; and
**n** and **m** represent each independently an integer ranging from 0 to 4;
**R²** represents H, aryl group, heteroaryl group or heterocyclyl group or hydroxyl group;
**p** represents an integer ranging from 0 to 1;
**A** represents -N(R³)(R⁴), -OR⁵ or an heteroaryl group comprising a nitrogen atom ;
**R³** represents a hydrogen atom, unsubstituted or substituted alkyl group, or an unsubstituted or substituted aminoalkyl group;
**R⁴** represents a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- and -O-, and terminated by a group selected from substituted or unsubstituted amine group, substituted or unsubstituted heteroaryl group, substituted or unsubstituted heterocyclyl group and phosphoramidate of formula 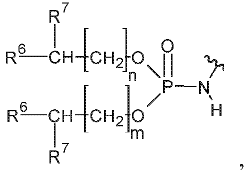 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl; and
**R⁵** represent a ionizable group selected from substituted or unsubstituted heterocyclyl group comprising a nitrogen atom, substituted or unsubstituted heteroaryl group comprising a nitrogen atom, substituted or unsubstituted aryl group, or a linear or branched saturated hydrocarbon chain, the hydrocarbon chain being optionally interrupted by one or more heteroatoms chosen from N, S or O, and terminated by a group selected from substituted or unsubstituted amine group, substituted or unsubstituted heteroaryl group, and substituted or unsubstituted heterocyclyl group.

According to one embodiment, **R⁶** and **R⁷**, identical or different, represent, independently from one another, a linear or branched, saturated, C₁-C₁₅ alkyl chain, optionally substituted by a cyclic or acyclic C₁-C₆ alkyl. According to one embodiment, **R⁶** and **R⁷**, identical or different, represent, independently from one another, a linear or branched, saturated, C₄-C₁₄ alkyl chain, optionally substituted by a cyclic or acyclic C₁-C₆ alkyl. According to one embodiment, **R⁶** and **R⁷**, identical or different, represent, independently from one another, a linear saturated C₄-C₁₄ alkyl chain, optionally substituted by a cyclic or acyclic C₁-C₆ alkyl. According to one embodiment, **R⁶** and **R⁷** are different. According to one embodiment, **R⁶** a linear saturated, C₉-C₁₄ alkyl chain. According to one embodiment, **R⁷** represents a linear saturated, C₈-C₁₂ alkyl chain.

According to a preferred embodiment, the compound of the invention is a compound of Formula (I-B), 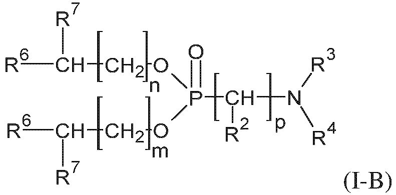
wherein **R², R⁶**, **R⁷**, **n, m** and **p** are such as defined hereinabove, i.e. as defined in formula (I-A), and
**R³** represents a hydrogen atom, unsubstituted or substituted alkyl group, or an unsubstituted or substituted aminoalkyl group;
**R⁴** represents a neutral group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- and -O-, and terminated by a group selected from substituted or unsubstituted amine group, substituted or unsubstituted heteroaryl group, substituted or unsubstituted heterocyclyl group and phosphoramidate of formula 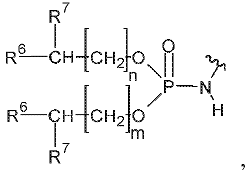 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl; and

According to a preferred embodiment, the compound of the invention is a compound of Formula (I-C), 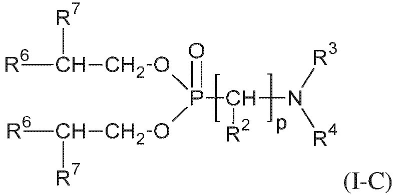
wherein **R², R⁶**, **R⁷** and **p** are such as defined hereinabove, i.e. as defined in formula (I-A), and
**R³** represents a hydrogen atom, unsubstituted or substituted alkyl group, or an unsubstituted or substituted aminoalkyl group;
**R⁴** represents a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- and -O-, and terminated by a group selected from substituted or unsubstituted amine group, substituted or unsubstituted heteroaryl group, substituted or unsubstituted heterocyclyl group and phosphoramidate of formula 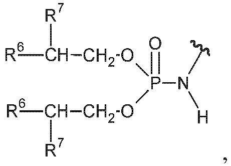 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl.

According to a preferred embodiment, the compound of the invention is a compound of Formula (I-D), 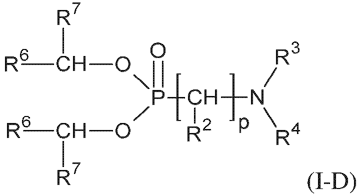
wherein **R², R⁶**, **R⁷** and **p** are such as defined hereinabove, i.e. as defined in formula (I-A), and
**R³** represents a hydrogen atom, unsubstituted or substituted alkyl group, or an unsubstituted or substituted aminoalkyl group;
**R⁴** represents a neutral group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- and -O-, and terminated by a group selected from substituted or unsubstituted amine group, substituted or unsubstituted heteroaryl group, substituted or unsubstituted heterocyclyl group and phosphoramidate of formula 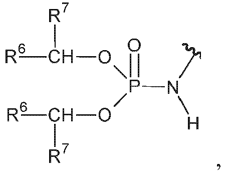 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl.

According to one embodiment, the compound according to the invention is selected from:

**Table 1.**

| | | |
|---|---|---|
| **001** | 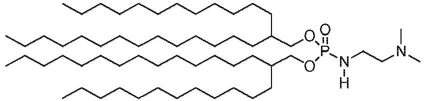 | *O,O*-bis(2-dodecylhexadecyl)-*N*-(2-(dimethylamino)ethyl)phosphoramidate |
| **002** | 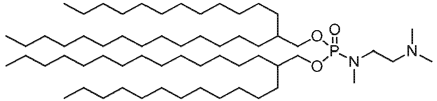 | *O,O*-bis(2-dodecylhexadecyl)-*N*-methyl-*N-(2-*(dimethylamino)ethyl)phosphoramidate |
| **003** | 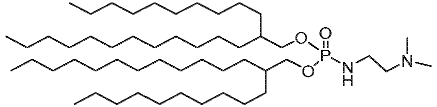 | *O,O*-bis(2-decyltetradecyl)-*N*-(2-(dimethylamino)ethyl)phosphoramidate |
| **004** | 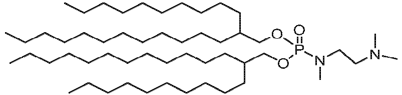 | *O,O*-bis(2-decyltetradecyl)-*N*-methyl-*N-*(2-(dimethylamino)ethyl)phosphoramidate |
| **005** | 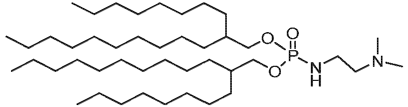 | *O*,*O*-bis(2-octyldodecyl)-*N*-(2-(dimethylamino)ethyl)phosphoramidate |
| **006** | 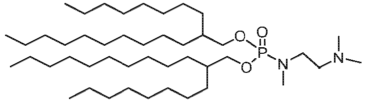 | *O*,*O*-bis(2-octyldodecyl)-*N*-methyl-*N*-(2-(dimethylamino)ethyl)phosphoramidate |
| **007** | 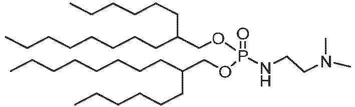 | *O*,*O*-bis(2-hexyldecyl)-*N*-(2-(dimethylamino)ethyl)phosphoramidate |
| **008** | 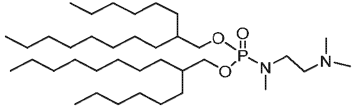 | *O*,*O*-bis(2-hexyldecyl)-*N*-methyl-*N*-(2-(dimethylamino)ethyl)phosphoramidate |
| **009** | 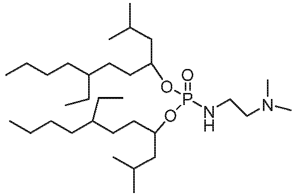 | *O*,*O*-bis(7-ethyl-2-methylundecan-4-yl)-*N-(2-*(dimethylamino)ethyl)phosphoramidate |
| **010** | 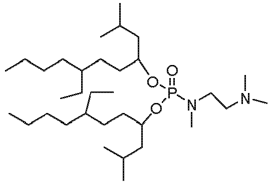 | *O*,*O*-bis(7-ethyl-2-methylundecan-4-yl)-*N* -methyl-*N*-(2-(dimethylamino)ethyl) Phosphoramidate |
| **011** | 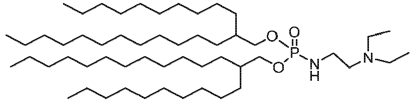 | *O*,*O*-bis(2-decyltetradecyl)-*N*-(2-(dimethylamino)ethyl)phosphoramidate |
| **012** | 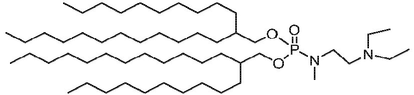 | *O*,*O*-bis(2-decyltetradecyl)-*N*-methyl-*N-*(2-(dimethylamino)ethyl)phosphoramidate |
| **013** | 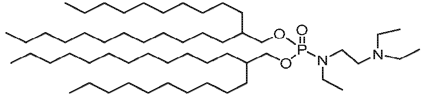 | *O*,*O*-bis(2-decyltetradecyl)-*N*-ethyl-*N-*(2-(dimethylamino)ethyl)phosphoramidate |
| **014** | 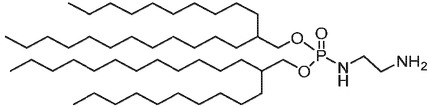 | *O*,*O*-bis(2-decyltetradecyl)-*N*-ethyl-*N-*(2-(dimethylamino)ethyl)phosphoramidate |
| **015** | 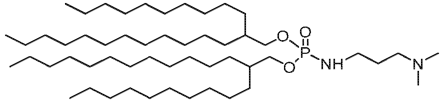 | *O*,*O*-bis(2-decyltetradecyl)-*N*-(3-(dimethylamino)propyl)phosphoramidate |
| **016** | 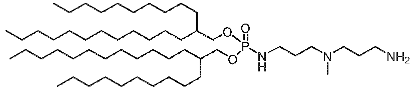 | *O*,*O*-bis(2-decyltetradecyl)-*N*-(3-((3-aminopropyl)(methyl)amino)propyl) Phosphoramidate |
| **017** | 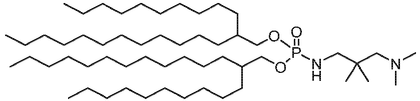 | *O*,*O*-bis(2-decyltetradecyl)-*N*-(3-(dimethylamino)-2,2-dimethylpropyl) Phosphoramidate |
| **018** | 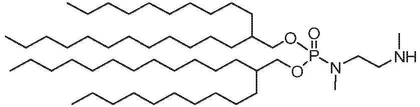 | *O*,*O*-bis(2-decyltetradecyl)-*N*-(2-(methylamino)ethyl)phosphoramidate |
| **019** | 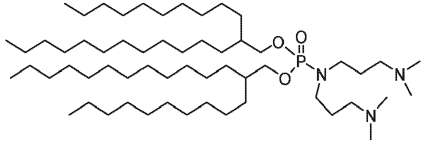 | *O*,*O*-bis(2-decyltetradecyl)-*N*,*N*-bis(3-(dimethylamino)propyl)phosphoramidate |
| **020** | 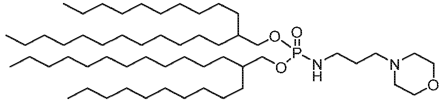 | *O*,*O*-bis(2-decyltetradecyl)-*N*-(3-morpholinopropyl)phosphoramidate |
| **021** | 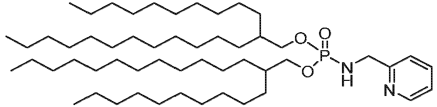 | *O*,*O*-bis(2-decyltetradecyl)-*N*-(pyridin-2-ylmethyl)phosphoramidate |
| **022** | 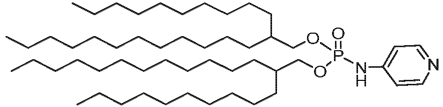 | *O*,*O*-bis(2-decyltetradecyl)-*N*-pyridin-4-ylphosphoramidate |
| **023** | 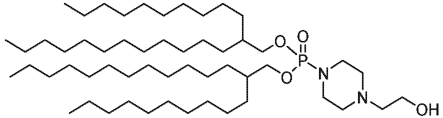 | *O*,*O*-bis(2-decyltetradecyl)-*N*-(4-(2-hydroxyethyl)piperazin-1-yl)phosphoramidate |
| **024** | 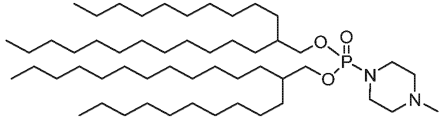 | *O*,*O*-bis(2-decyltetradecyl)-*N*-(4-methyl piperazin-1-yl)phosphoramidate |
| **025** | 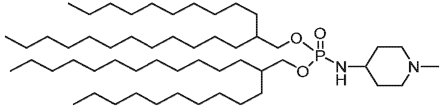 | *O*,*O*-bis(2-decyltetradecyl)-*N*-(1-methyl piperidin-4-yl)phosphoramidate |
| **026** | 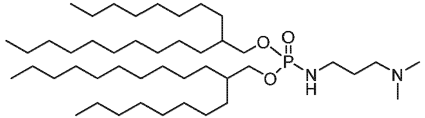 | *O*,*O*-bis(2-octyldodecyl)-*N*-(3-(dimethylamino)propyl)phosphoramidate |
| **027** | 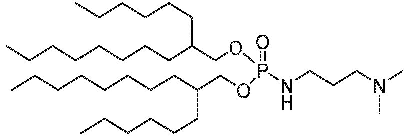 | *O*, *O*-bis(2-hexyldecyl)-*N*-(3-(dimethylamino)propyl)phosphoramidate |
| **028** | 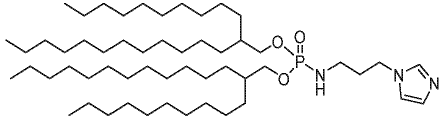 | *O*,*O*-bis(2-decyltetradecyl)-*N*-(3-(1*H-*imidazol -1-yl)propyl)phosphoramidate |
| **029** | 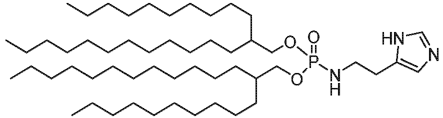 | *O*,*O*-bis(2-decyltetradecyl)-*N*-(2-(1*H-*imidazol -5-yl)ethyl)phosphoramidate |
| **030** | 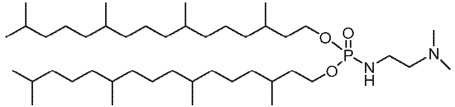 | *O*,*O*-bis(3,7,11,15-tetramethylhexadecyl) -*N*-(2-(dimethylamino)ethyl) phosphoramidate |
| **031** | 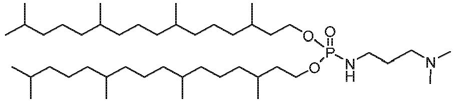 | *O*,*O*-bis(3,7,11,15-tetramethylhexadecyl) -*N*-(3-(dimethylamino)propyl) Phosphoramidate |
| **032** | 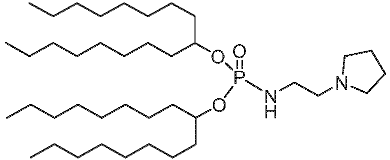 | *O*,*O*-di(heptadecan-9-yl) (2-(pyrrolidin-1-yl)ethyl) Phosphoramidate |
| **033** | 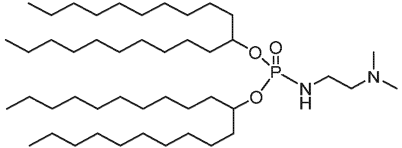 | *O*,*O*-di(henicosan-11-yl) (2-(dimethylamino)ethyl) Phosphoramidate |
| **034** | 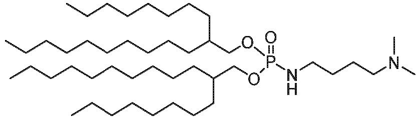 | *O*,*O*-bis(2-octyldodecyl)-*N*-(4-(dimethylamino)butyl)phosphoramidate |
| **035** | 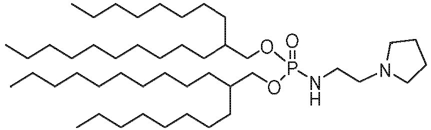 | *O*, *O*-bis(2-octyldodecyl)-*N-*(2-(pyrrolidin-1-yl)ethyl) Phosphoramidate |
| **036** | 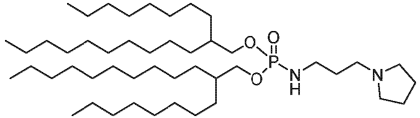 | *O*, *O*-bis(2-octyldodecyl)-*N-*(3-(pyrrolidin-1-yl)propyl) Phosphoramidate |
| **037** | 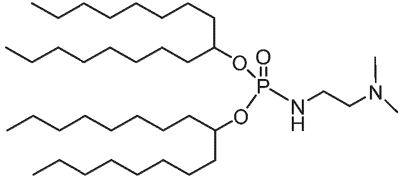 | *O*,*O*-di(heptadecan-9-yl)-*N-*(2-(dimethylamino)ethyl) Phosphoramidate |
| **038** | 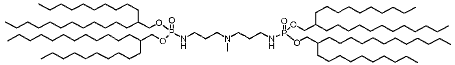 | *O*,*O*-tetrakis(2-decyltetradecyl)-*N-*((methylazanediyl)bis(propane-3,1-diyl))bis(phosphoramidate) |
| **039** | 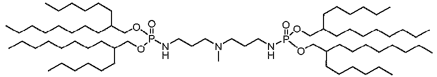 | *O*,*O*-tetrakis(2-hexyldecyl)-*N-*((methylazanediyl)bis(propane-3,1-diyl))bis(phosphoramidate) |
| **040** | 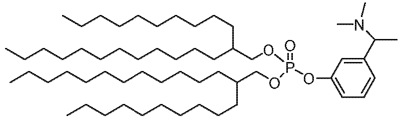 | *O*,*O*-bis(2-decyltetradecyl)-*N-*(3-(1-(dimethylamino)ethyl) phenyl) phosphate |
| **041** | 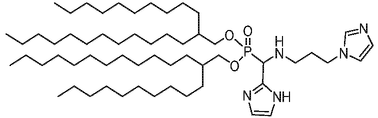 | *O*,*O*-bis(2-decyltetradecyl)-*N*-(((3-(1*H*-imidazol-1-yl)propyl)amino) (1*H*-imidazol-2-yl)methyl)phosphonate |
| **042** | 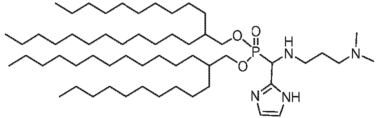 | *O*,*O*-bis(2-decyltetradecyl)-*N*-(((3-(dimethylamino)propyl)amino) (1*H*-imidazol-2-yl)methyl)phosphonate |
| **043** | 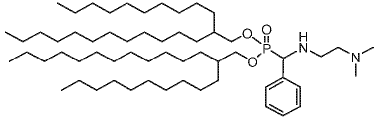 | *O*,*O*-bis(2-decyltetradecyl)-*N*-(((2-(dimethylamino)ethyl)amino) (phenyl)methyl)phosphonate |
| **044** | 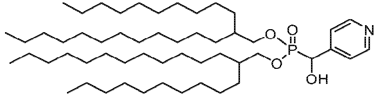 | *O*,*O*-bis(2-decyltetradecyl)-hydroxy-(pyridine-4-yl)phosphonate |
| **045** | 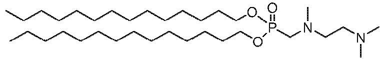 | *O,O*-ditetradecyl-(((2-dimethylamino)ethyl)(methyl) amino)methyl)phosphonate |
| **046** | 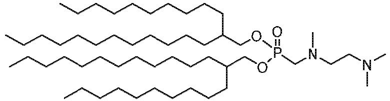 | *O*,*O*-bis(2-decyltetradecyl)-(((2-(dimethylamino)ethyl)(methyl) amino)methyl)phosphonate |
| **047** | 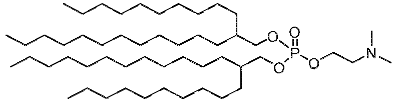 | *O*,*O*-bis(2-decyltetradecyl)-*O*-(*N*,*N-*dimethylaminoeth-2-yl)phosphoramidate |
| **048** | 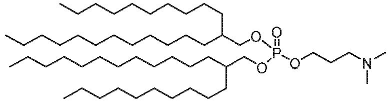 | *O*,*O*-bis(2-decyltetradecyl)-*O*-(*N*,*N-*dimethylaminoprop-3-yl)phosphoramidate |
| **049** | 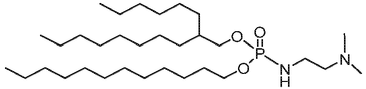 | *O*,*O*-dodecyl(2-hexyldecyl)-*N*-(2-(dimethylamino)ethyl)phosphoramidate |
| **050** | 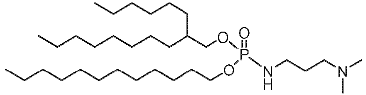 | *O*,*O*-dodecyl(2-hexyldecyl)-*N*-(3-(dimethylamino)propyl) Phosphoramidate |
| **051** | 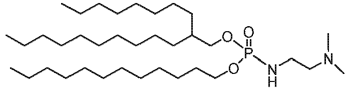 | *O*,*O*-(Z)-octadec-9-en-1-yl(2-octyldodecyl) -*N*-(2-(dimethylamino)ethyl) Phosphoramidate |
| **52** | 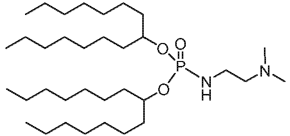 | *O*,*O*-di(pentadecan-8-yl) (2-(dimethylamino)ethyl)phosphoramidate |
| ***53*** | 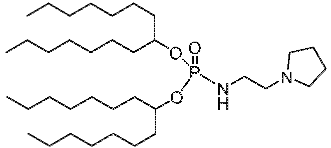 | *O*,*O*-di(pentadecan-8-yl) (2-(pyrrolidin-1-yl)ethyl)phosphoramidate |
| **54** | 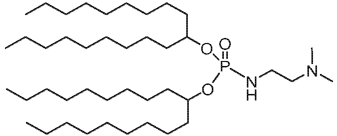 | *O,O*-di(nonadecan-10-yl) (2-(dimethylamino)ethyl)phosphoramidate |
| **55** | 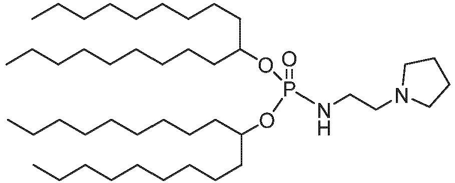 | *O*,*O*-di(nonadecan-10-yl) (2-(pyrrolidin-1-yl)ethyl)phosphoramidate |
| **56** | 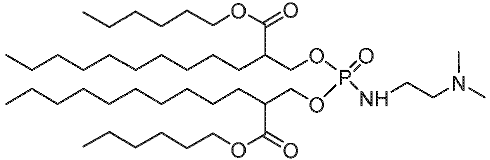 | dihexyl 2,2'-(((((2-(dimethylamino)ethyl)amino) phosphoryl)bis(oxy))bis(methylene)) didodecanoate |

The compounds of **Table 1** were named with the assistance of ChemDraw^{®} Professional 15.1 (PerkinElmer).

This invention also relates to a liposomal nanoparticle comprising at least one compound of the invention such as described hereinabove. In particular, the liposomal nanoparticle comprises at least one of the compounds of formula (I), (I-A), (I-B), (I-C) or (I-D). In a preferred embodiment, the liposomal nanoparticle comprises at least one of the compounds of formula (I-A), (I-B), (I-C) or (I-D). In a preferred embodiment, the liposomal nanoparticle comprises at least one of the compounds of formula (I-B), (I-C) or (I-D). In a preferred embodiment, the liposomal nanoparticle comprises at least one compound of formula (I-C). In a preferred embodiment, the liposomal nanoparticle comprises at least one compound of formula (I-D).

The liposomal nanoparticle of the invention can be obtained according to methods known by the skilled artisan.

This invention also relates to a LNP comprising at least one compound of the invention such as described hereinabove. In particular, the LNP comprises at least one of the compounds of formula (I), (I-A), (I-B), (I-C) or (I-D). In a preferred embodiment, the LNP comprises at least one of the compounds of formula (I-A), (I-B), (I-C) or (I-D). In a preferred embodiment, the LNP comprises at least one of the compounds of formula (I-B), (I-C) or (I-D). In a preferred embodiment, the LNP comprises at least one compound of formula (I-C). In a preferred embodiment, the LNP comprises at least one compound of formula (I-D). According to one embodiment, the LNP comprises at least one of the compounds of formula (I), (I-A), (I-B), (I-C) or (I-D), at least one nucleic acid sequence and at least one PEG lipid. According to one embodiment, the LNP further comprises at least one sterol and/or at least one helper lipid.

According to one embodiment, at least one nucleic acid sequence means 1, 2, 3, 4 or 5 different nucleic acid sequences. According to one embodiment, the nucleic acid sequence is a ribonucleic acid (RNA) sequence or a deoxyribonucleic acid (DNA) sequence. According to one embodiment, the nucleic acid sequence is selected from mRNA, pDNA, siRNA, gRNA saRNA, shRNA, microRNA and a combination thereof.

According to one embodiment, at least one PEG lipid means 1, 2 or 3 different PEG lipids, preferably 1 or 2 different PEG lipids, more preferably 1 PEG lipids. According to one embodiment, PEG lipid is 1,2-dimyristoyl-sn-glycero-3-phosphoethanolamine-N-[methoxy(polyethylene glycol)-2000] (DMPE-PEG-2000).

According to one embodiment, at least one sterol means 1, 2 or 3 different sterols, preferably 1 or 2 different sterols, more preferably 1 sterol. According to one embodiment, the sterol is cholesterol.

According to one embodiment, at least one helper lipid means 1, 2 or 3 different helper lipids, preferably 1 or 2 different helper lipids, more preferably 1 helper lipid. According to one embodiment, the helper lipid is (delta9-Cis)-1,2-Dioleoyl-sn-glycero-3-phosphoethanolamine (DOPE).

In one of the embodiment: LNP are constituted with mRNA, branched ionizable phospholipid 50%, DOPE used as helper lipid 10 %, cholesterol: 38.5 % and PEG lipid (DMPE-PEG-2000 : 1.5%). In a second embodiment LNP are constituted with mRNA, 35.0% of branched ionizable phospholipid, 16.0% helper lipid (DOPE), 46.5% Cholesterol, and 2.5% C14-PEG2K (DMPE-PEG2000).

The LNP of the invention can be obtained according to methods known by the skilled artisan.

According to an embodiment, the LNP can be prepared by evaporation of the organic solvent wherein the compounds of formula (I), (I-A), (I-B), (I-C) or (I-D) of the invention are dissolved eventually with other lipids (helper lipids, sterols, PEG lipids, targeting lipids), then by putting said compounds in suspension in an aqueous solvent. This operation is preferentially carried out at a temperature greater than the phase transition temperature of the compounds of the invention.

LNP solution can be prepared by microfluidics. It consists to mix in a microfluidic ship two solutions : one solution is constituted by an aqueous solution or buffer solution and the second solution include one or a mix of the of the compounds of formula (I), (I-A), (I-B), (I-C) or (I-D) of the invention and one or a mixture of helper lipid, and one or a mixture of sterol and one or a mixture of PEG lipid that are dissolved in an organic solvent (e.g. ethanol). At the end the liposomal solution is dialyzed to remove the organic solvent.

According to one embodiment LNP can be prepared by microfluidic devices consisting to mix in a microfluidic ship two solutions : one solution is constituted by a nucleic acid in an aqueous solution or buffer solution and the second solution include one or a mix of the of the compounds of formula (I), (I-A), (I-B), (I-C) or (I-D) of the invention and one or a mixture of helper lipid, and one or a mixture of sterol and one or a mixture of PEG lipid that are dissolved in an organic solvent (e.g. ethanol). At the end the LNP solution is dialyzed to remove the organic solvent.

According to one embodiment, LNP can be prepared by ethanolic injection consisting to place one or a mix of the of the compounds of formula (I), (I-A), (I-B), (I-C) or (I-D) of the invention and one or a mixture of helper lipid, and one or a mixture of sterol and one or a mixture of PEG lipid that are dissolved in an organic solvent (e.g. ethanol). This solution is then injected in an aqueous solution containing the nucleic acid. The final LNP solution is dialyzed to remove the organic solvent.

According to one embodiment, the size of the LNP of the invention is ranging from 50 nm to 250 nm, preferably ranging from50 nm to 120 nm, preferably is about 100 nm.

This invention also relates to a lipoplex comprising at least one compound of the invention such as described hereinabove. In particular, the lipoplex comprises at least one of the compounds of formula (I), (I-A), (I-B), (I-C) or (I-D). In a preferred embodiment, the lipoplex comprises at least one of the compounds of formula (I-A), (I-B), (I-C) or (I-D). In a preferred embodiment, the lipoplex comprises at least one of the compounds of formula (I-B), (I-C) or (I-D). In a preferred embodiment, the lipoplex comprises at least one compound of formula (I-C). In a preferred embodiment, the lipoplex comprises at least one compound of formula (I-D).

The lipoplexes of the invention can be obtained according to methods known by the skilled artisan. Mixture of liposomal solution with a solution including a nucleic acid.

### Method

The invention also relates to a process for manufacturing a compound of formula (I) as defined hereabove:
comprising the following step:
Reacting a compound of formula (II): 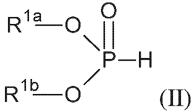
wherein **R^{1a}** and **R^{1b}** identical or different, represent, independently from one another, a branched or linear saturated C₅-C₃₅ alkyl chain, optionally substituted by a cyclic or acyclic C₁-C₆ alkyl or by an ester group of formula (C₆-C₁₄)-O-C(O)-; provided that at least one of **R^{1a}** and **R^{1b}** is a branched saturated C₅-C₃₅ alkyl chain;
with a compound of formula (III) 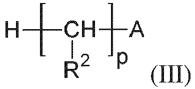 wherein
**R²** represents H, aryl group, heteroaryl group or heterocyclyl group;
**p** represents an integer ranging from 0 to 1;
**A** represent -N(R³)(R⁴), -OR⁵ or an heteroaryl group comprising a nitrogen atom;
**R³** represent a hydrogen atom, unsubstituted or substituted alkyl group, or an unsubstituted or substituted aminoalkyl group;
**R⁴** represent a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- or -O-, and terminated by a group selected from substituted or unsubstituted amine group, substituted or unsubstituted heteroaryl group, substituted or unsubstituted heterocyclyl group and phosphoramidate of formula 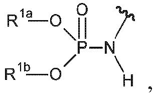 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl; and
**R⁵** represent a ionizable group selected from substituted or unsubstituted heterocyclyl group comprising a nitrogen atom, substituted or unsubstituted heteroaryl group comprising a nitrogen atom, substituted or unsubstituted aryl group, or a linear or branched saturated hydrocarbon chain, the hydrocarbon chain being optionally interrupted by one or more heteroatoms chosen from N, S or O, and terminated by a group selected from substituted or unsubstituted amine group, substituted or unsubstituted heteroaryl group, and substituted or unsubstituted heterocyclyl group,
in the presence of an amine.

According to one embodiment, the compound of formula (I) is a compound of Formula (I-A). According to one embodiment, the compound of formula (I) is a compound of Formula (I-B). According to a preferred embodiment, the compound of formula (I) is a compound of Formula (I-C). According to another preferred embodiment, the compound of formula (I) is a compound of Formula (I-D).

According to one embodiment, this reaction step is carried out in the presence of 1 equivalent of compound of formula (II) and 1 to 1.5 equivalent of compound of formula (III), preferably from 1.4 to 1.5 equivalent of compound of formula (III), more preferably around 1.3 equivalent of compound of formula (III).

According to one embodiment, this reaction step is carried out in the presence of 1 to 1.5 equivalent of amine, preferably from 1.2 to 1.5 equivalent of amine, more preferably around 1.3 equivalent of amine.

The amine may be advantageously chosen from a tertiary amine or aromatic amine like diisopropylethylamine, triethylamine, pyridine, dimethylaminopyridine, preferably is diisopropylethylamine.

According to one embodiment, the reaction is carried out in presence of bromotricholomethane.

According to one embodiment, the solvent used in this reaction step is selected from the group comprising dichloromethane, chloroform, tetrachloromethane, methanol, ethanol, acetonitrile, ethyl acetate, dimethylformamide and a mixture thereof. According to a preferred embodiment, the solvent used in this reaction step is dichloromethane.

According to one embodiment, this reaction step is carried out under cooling/heating conditions, at a temperature ranging from 0°C to 30°C, preferably 4°C then 20°C after stirring 30 minutes.

According to one embodiment, this reaction step is carried out under inert atmosphere.

According to one embodiment, compound of formula (I) is purified by using chromatographic techniques.

The invention also relates to a process for manufacturing the lipoplex of the invention comprising a step of mixing a nucleic acid sequence and a compound according to the invention.

According to one embodiment, the nucleic acid sequence is a ribonucleic acid (RNA) sequence or a deoxyribonucleic acid (DNA) sequence. According to one embodiment, the nucleic acid sequence is selected from mRNA, pDNA, siRNA, gRNA saRNA, shRNA, microRNA and a combination thereof.

According to one embodiment, the formulation process of the lipoplex or LNP can be done by microfluidic device, hydration of a lipid film followed by the addition of nucleic acid or any other methods producing either liposomal solution, loaded liposomal solution or nucleic acid based LNP. According to a preferred embodiment, the formulation process of the LNP is done by microfluidic device. According to one embodiment, the branched ionizable phospholipid are formulated with DOPE, cholesterol, C14-PEG₂ₖ PE. Using micro-fluidics allows more reproducible results and produces smaller LNPs.

### Pharmaceutical composition

This invention also relates to a pharmaceutical composition comprising a compound according to the invention, a liposomal nanoparticle according to the invention and/or a lipoplex or LNP according to the invention and/or a LNP according to the invention as described hereinabove, and at least one pharmaceutically acceptable carrier.

According to an embodiment, the composition of the invention is a pharmaceutical composition comprising a compound of Formula I in combination with a pharmaceutically acceptable vehicle. According to an embodiment the compound of formula I is a compound of formula I-C. According to another embodiment the compound of formula I is a compound of formula I-D.

### Medical use and methods of treatment

This invention also relates to a compound according to the invention, a liposomal nanoparticle according to the invention and/or a lipoplex according to the invention and/or a LNP according to the invention, as described hereinabove, for use as a medicament.

This invention also relates to the use of a compound according to the invention, as described hereinabove, in the manufacture of a medicament.

This invention also relates to the use of a compound according to the invention, as described hereinabove, in the manufacture of a medicament for the treatment and/or prevention of viral infection (e.g. Zika, HIV, Cytomegalovirus, rabies virus, COVID, influenza, respiratory syncytial virus, zoster virus), genetic disease including rare diseases, cancer (e.g. melanoma, brain cancer, lung cancer, liver cancer, pancreas cancer, prostate cancer, blood system cancer, ovarian cancer, breast cancer, digestive cancer, bone cancer), immunological diseases, metabolic diseases, ocular diseases.

This invention also relates to a method for the treatment and/or prevention of viral infection (e.g. Zika, HIV, Cytomegalovirus, rabies virus, COVID, influenza, respiratory syncytial virus, zoster virus), genetic disease including rare diseases, cancer (e.g. melanoma, brain cancer, lung cancer, liver cancer, pancreas cancer, prostate cancer, blood system cancer, ovarian cancer, breast cancer, digestive cancer, bone cancer), immunological diseases, metabolic diseases, ocular diseases in a subject in need thereof, comprising a step of administrating to said subject a therapeutically effective amount of a compound according to the invention, as described hereinabove.

According to a first embodiment, the compound according to the invention is administrated to the subject as sole therapeutic agent.

According to a second embodiment, the compound according to the invention is administrated to the subject in combination with at least another therapeutic agent.

The invention relates to a medicament comprising a compound, a liposomal nanoparticle and/or a lipoplex according to the invention. The invention relates to a medicament comprising a compound of Formula I. According to an embodiment the compound of formula I is a compound of formula I-C. According to another embodiment the compound of formula I is a compound of formula I-D.

The uses described hereinbelow relate to the use of a compound of Formula I, of a pharmaceutical composition or of a medicament according to this invention.

This invention also relates to a compound according to the invention for the use thereof in transfection in vivo, for gene editing, base editing, prime editing, gene therapy, vaccination, cancer immunotherapy, topical treatments or bactericidal activities.

Liposomal nanoparticle according to the invention or lipoplex according to the invention or LNP according to the invention for the use thereof in transfection in vivo, for gene editing, base editing, gene therapy, vaccination, cancer immunotherapy, topical treatments, bactericidal activities, ex-vivo cell transfection or transfection of organoids and in vitro transfection.

Lipoplexes and LNP including lipid of this invention can be formulated with nucleic acid including one DNA barecode to optimize the formulations. Barcoding technology.

. Lipoplexes and LNP including lipid of this invention can be formulated with nucleic acid including one DNA barecode.

According to one embodiment, gene editing may be CRISPR-CAS9, HITI-mediated genome editing.

According to an embodiment, the subject is an animal, more preferably a mammal, more preferably a human.

### Methods of administration

The compounds of the invention may be administered by oral, parenteral (e.g., intramuscular, intraperitoneal, intravenous, intraocular, ICV, intracisternal injection or infusion, subcutaneous injection, or implant), by inhalation spray, nasal, vaginal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals such as mice, rats, horses, cattle, sheep, dogs, cats, monkeys, etc., the compounds of the invention are effective for use in humans. The pharmaceutical compositions for the administration of the compounds of this invention may conveniently be presented in dosage unit form and may be prepared by any of the methods well known in the art of pharmacy. All methods include the step of bringing the compound of the invention into association with the carrier which constitutes one or more accessory ingredients. In general, the pharmaceutical compositions are prepared by uniformly and intimately bringing the compound of the invention into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts. The pharmaceutical compositions containing the compound of the invention may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the compound of the invention in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patents 4,256,108; 4,166,452; and 4,265,874 to form osmotic therapeutic tablets for control release. Formulations for oral use may also be presented as hard gelatin capsules wherein the compound of the invention is mixed with an inert solid diluent, for example, calcium carbonate, calcium phosphate or kaolin, or as soft gelatin capsules wherein the compound of the invention is mixed with water or an oil medium, for example peanut oil, liquid paraffin, or olive oil.

Aqueous suspensions that can be suitable according to the present invention preferably contain the compound of the invention in admixture with excipients suitable for the manufacture of aqueous suspensions. Such excipients are preferably suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions that can be suitable according to the invention may be formulated by suspending the compound of the invention in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid. Dispersible powders and granules suitable for preparation of an aqueous suspension by the addition of water provide the compound of the invention in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Suitable syrups and elixirs may be formulated with sweetening agents, for example glycerol, propylene glycol, sorbitol or sucrose. Such formulations may also contain a demulcent, a preservative and flavoring and coloring agents.

The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables. The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols. For topical use, creams, ointments, jellies, solutions or suspensions, etc., containing the compounds of the present invention are employed. (For purposes of this application, topical application shall include mouthwashes and gargles.)

### BRIEF DESCRIPTION OF THE DRAWINGS

**Figure 1** is a histogram showing size, and polydispersity of mRNA-based LNP obtained with compounds of the invention.
**Figure 2** is a histogram showing the encapsulation of mRNA in LNPs prepared from the ionizable lipids of the invention.
**Figure 3A** is a histogram showing the in-vitro results of the mRNA-based LNP obtained with compounds **003** and compounds CA, CB, and CC, DA, DB
**Figure 3B** is a histogram showing the in-vivo results of the m-RNA-based LNP obtained with compounds **003** and compounds CA, CB, and CC .
**Figure 4A** is a histogram showing the efficacy of mRNA delivery of LNPs (003, 005, 006, 015, 048, 046, 008, 007, 026, 027, 043) in vivo, liver ROI (Region of Interest) (3 µg of mRNA injected and formulated as LNP including 35% of ionizable lipids indicated by its label number) and analyzed 4h and 24h post-transfection
**Figure 4B** is a histogram showing the efficacy of mRNA delivery of LNPs in vivo (046, 017, 048, 047, 016, 019, 045, 020), liver ROI (Region Of Interest) (3 µg of mRNA injected and formulated as LNP including 35% of the ionizable lipids indicated by its label number) and analyzed 4h or 24h post-transfection.
**Figure 4C** is a histogram showing the efficacy of mRNA delivery of LNPs in vivo (010, 007, 001, 004, 002, 014, 025, 011, 005, 006, 015, 046, 048), liver ROI (Region of Interest) (3 µg of mRNA injected and formulated as LNP including 35% of the ionizable lipids indicated by its label number) and analyzed 4h or 24h post-transfection.
**Figure 4D** is a histogram showing the efficacy of mRNA delivery of LNPs in vivo (005, 033, 032), liver ROI (Region of Interest) (2 µg of mRNA injected and formulated as LNP including 50% of the ionizable lipids indicated by its label number) and analyzed 4h or 24h post-transfection.
**Figure 4E** is a histogram showing the efficacy of mRNA delivery of LNPs in vivo (003, 035, 036, 037), liver ROI (Region of Interest) (2 µg of mRNA injected and formulated as LNP including 50% of the ionizable lipids indicated by its label number) and analyzed 4h or 24h post-transfection.

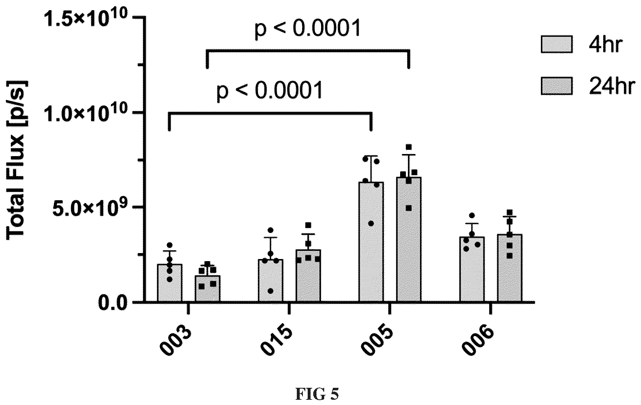

**Figure 5** is a histogram showing the in-vivo results of the LNP obtained with compounds **003, 015, 005** and **006.**

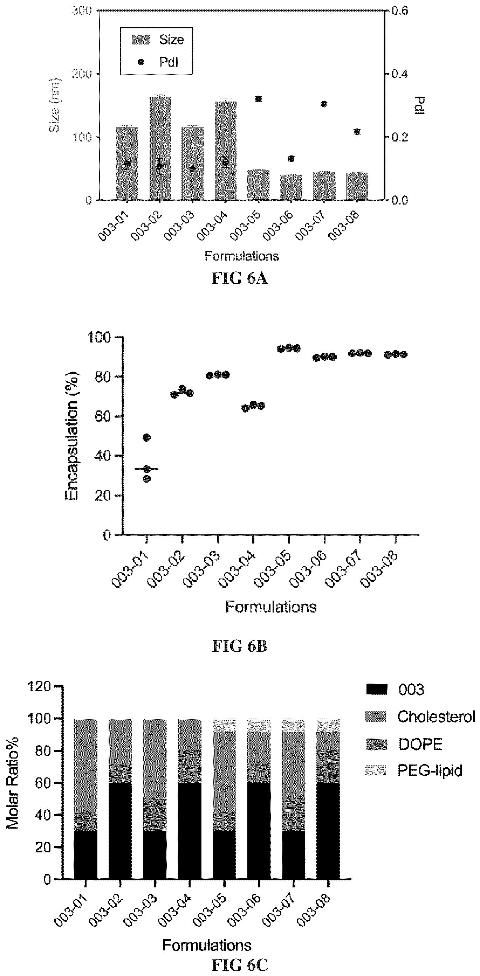

**Figure 6** is a histogram showing the results of the experiment determining lipid ratio extremes for barcoding experiment. Ionizable lipid 003 was used at 30% or 60% of lipid mix, DOPE was used at 12% or 20% of lipid mix, Cholesterol ranged from 11.5% to 57.5%, and PEG-lipid was used at 0.5% or 8.5% of total lipid mix. **Figure 6A** is a histogram showing size, and polydispersity of the different formulations. **Figure 6B** is a histogram showing encapsulation of mRNA of the different formulations. **Figure 6C** is a histogram showing the lipid composition for LNP formulation.

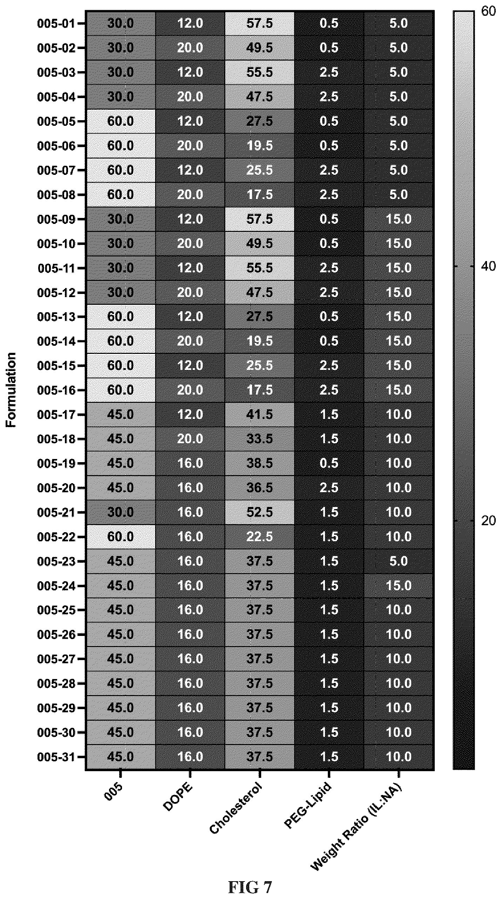

**Figure 7** is a table showing the Formulation library used for DNA barcoding. Molar ratio of 005, DOPE, Cholesterol, and PEG-lipid are shown. Weight ratio of Ionizable lipid (005) to nucleic acid (DNA barcode) is shown.
**Figure 8** is a histogram showing DNA barcoding library characteristics for LNP formulation screening of compound 005.

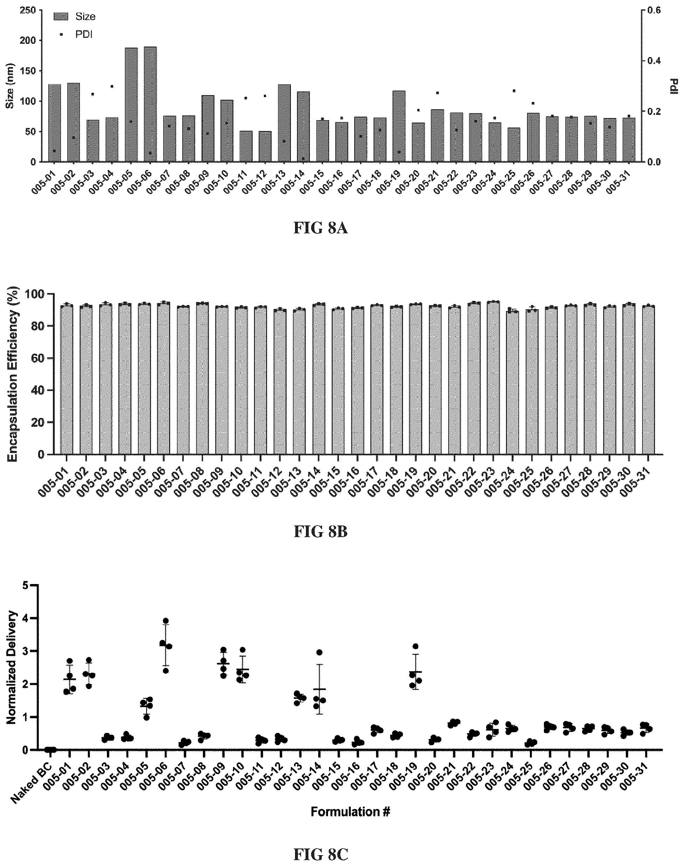

   **Figure 8**A is a histogram showing size, and polydispersity of LNPs encapsulating DNA barcodes.
   **Figure 8B** is a histogram showing barcode encapsulation efficiency.
   **Figure 8C** is a histogram showing the delivery of DNA barcodes to the liver.

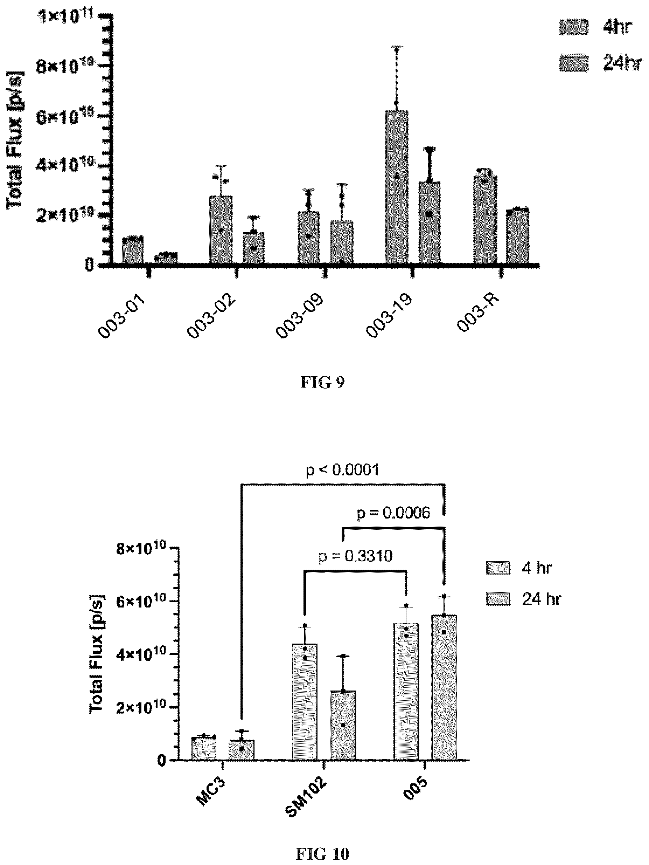

**Figure 9** is a histogram showing validation of barcoding lead candidates by mRNA delivery. 005-R formulation is a standard LNP formulation (50% ionizable lipid, 38.5% cholesterol, 10% structural lipid, 1.5% PEG).
**Figure 10** is a histogram showing in vivo comparison of FDA approved LNP formulations and LNP obtained with compound **005** formulation at 4 hours and 24 hours post-injection.

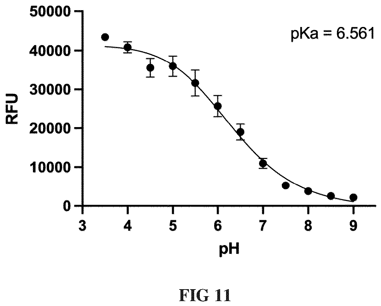

**Figure 11** is a graph showing the TNS assay of LNPs containing compound 005 as the ionizable lipid. The apparent pKa of the LNPs using this lipid is 6.56, within ideal range for endosomal escape.

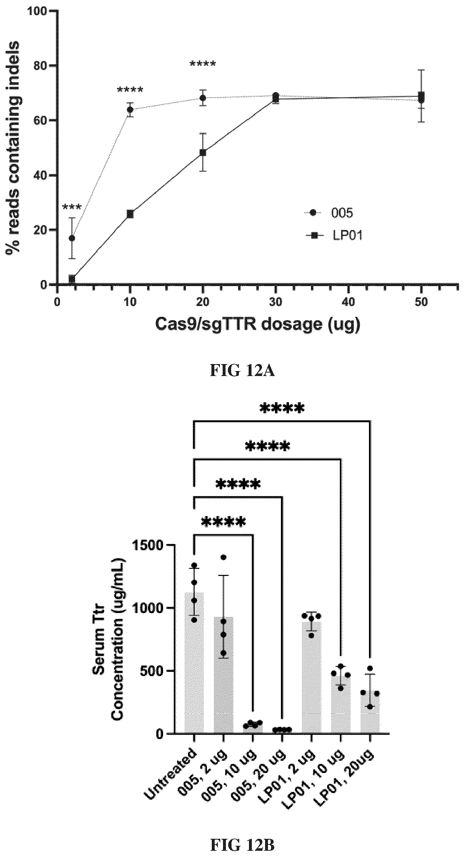

**Figure 12A** is graph showing genomic editing in terms of insertions and deletions of CRISPR-Cas9 knockdown of mouse Transthyretin (TTR). **Figure 12B** is a histogram showing reduction of TTR protein in the serum as a result of the genomic editing of CRISPR-Cas9 knockdown of mouse Transthyretin (TTR) Cas9 mRNA and an sgRNA targeting mouse TTR or a non-targeting guide specific to GFP protein were co-encapsulated with compound 005 or LP01. Mice were injected with 2, 10, 20 µg of total RNA (50% Cas9, 50% sgRNA).

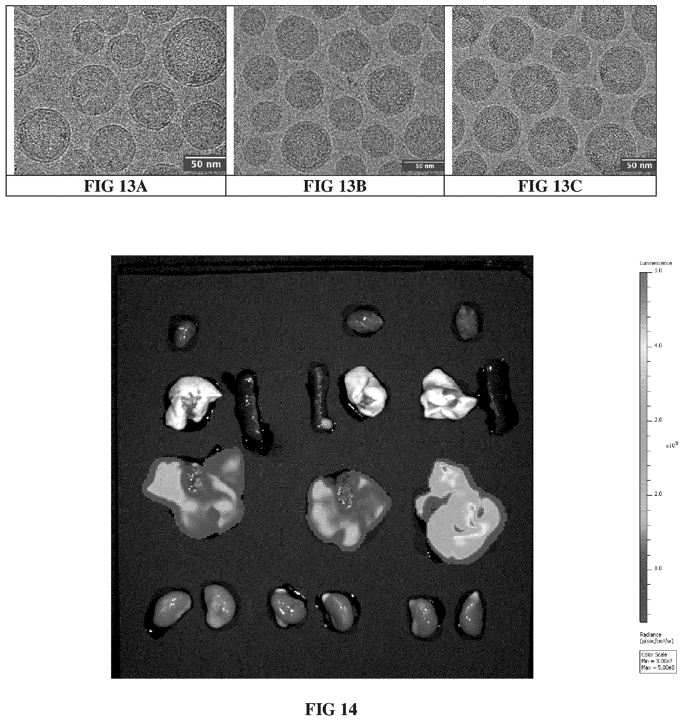

**Figure 13** is CryoEM imaging of LNPs containing compound 003, compound 005.
   **Figure 13A** is CryoEM imaging of 003 encapsulating FLuc mRNA.
   **Figure 13B** is CryoEM imaging of lipid 005 encapsulating FLuc mRNA.
   **Figure 13C** is CryoEM imaging of LNPs containing 005 co-encapsulating Cas9 mRNA & TTR sgRNA.
**Figure 14** is an ex-vivo imaging of mouse organs after administration of 005-LNPs encapsulating FLuc mRNA. Expression of FLuc is localized to the liver.

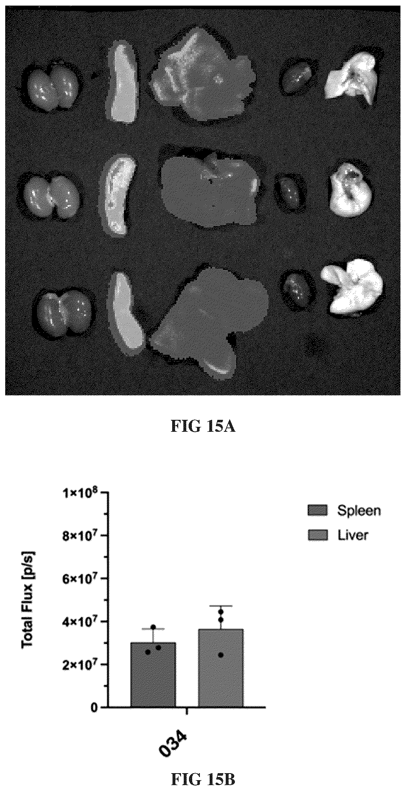

**Figure 15A** is an ex-vivo imaging of mouse organs after administration of 034-LNPs encapsulating FLuc mRNA. Expression of FLuc is localized to spleen and liver. **Figure 15B** is histogram showing the localization of the expression of FLuc in spleen and liver.

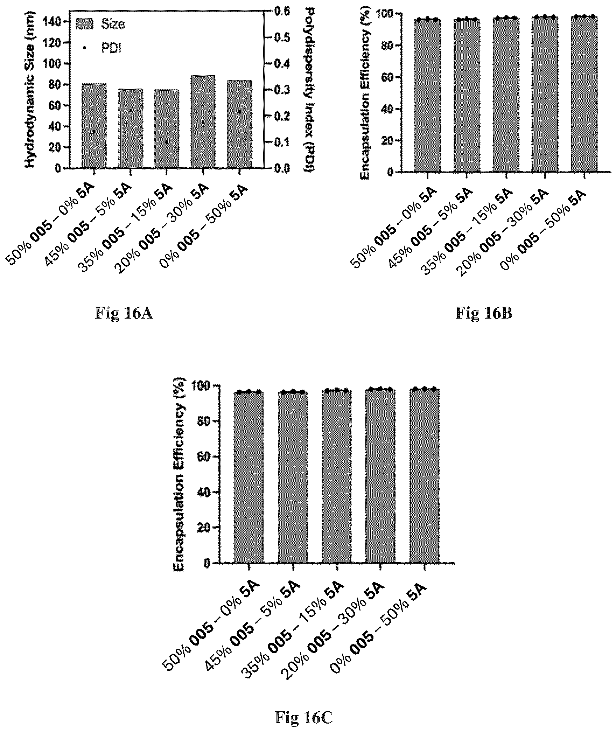

**Figure 16A** is an histogram showing the Size and PdI of LNP featuring different ratio of ionizable lipids 005/cationic lipid 5A ; **Figure 16B****:** is an histogram howing the encapsulation efficacies of LNP with different ratios of ionizable lipids 005/cationic lipid 5A; **Figure 16C** is an histogram showing the Zeta potential of LNP with different ratios of ionizable lipids 005/cationic lipid 5A.

### EXAMPLES

The present invention is further illustrated by the following examples which are provided by way of illustration only and should not be considered to limit the scope of the invention.

The following abbreviations are used:
ATR : Attenuated Total Reflectance,
Cas9mRNA: mRNA coding for the endonuclease CAS9
cm⁻¹: per centimeter,
ELSD: evaporative light scattering detector,
DIPEA: diisopropylethylamine,
DOPE: 1,2-Dioleoyl-sn-glycero-3-phosphoethanolamine,
eq.: equivalent,
ESI: Electrospray Ionisation,
ESI-qTOF: Electrospray Ionisation - Quadrupole Time-of-Flight,
g: gram,
GFP protein: Green Fluorescent Protein,
h: hour,
HPLC: high pressure liquid chromatography,
HRMS: high-resolution mass spectrometry,
K: Kelvin,
M: mol/liter,
MC3: D-Lin-MC3-DMA, (6Z,9Z,28Z,31Z)-heptatriacont-6,9,28,31-tetraene-19-yl 4-(dimethylamino)butanoate, CAS number : 1224606-06-7
MHz : megahertz,
mg: milligram,
min: minute,
mL: milliliter,
µL : microliter,
mmol: millimole,
NMR: Nuclear Magnetic Resonance,
PEG: polyethylene glycol
ppm: parts-per-million,
Rf: retention factor
RT: room temperature,
SM-102: Heptadecan-9-yl8-((2-hydroxyethyl)(6-oxo-6-(undecyloxy)hexyl)amino)
Octanoate, CAS number : 2089251-47-6
t: time,
TTR: transthyretin

| | |
|---|---|
| Cationic lipid 5A : | 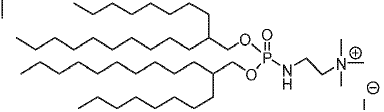 |
| LP01 | 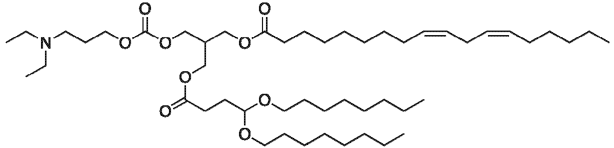 J. D. Finn, A. R. Smith, M. C. Patel, L. Shaw, M. R. Youniss, J. van Heteren, T. Dirstine, C. Ciullo, R. Lescarbeau, J. Seitzer, R. R. Shah, A. Shah, D. Ling, J. Growe, M. Pink, E. Rohde, K. M. Wood, W. E. Salomon, W. F. Harrington, C. Dombrowski, W. R. Strapps, Y. Chang, D. V. Morrissey, Cell Reports 2018, 22, 2227-2235 |

### Material and Methods

Reagents and chemicals were purchased from commercial suppliers and used without further purification.

**Nuclear Magnetic Resonance.** ¹H, ¹³C and ³¹P NMR spectra were recorded at 298 K using Bruker Avance 500 (500 MHz), or Bruker Avance 400 (400 MHz) spectrometers. The chemical shifts (in ppm) were measured versus the residual peak of the solvent as an internal standard. Data are reported as s = singlet, d = doublet, t = triplet, q = quartet, m = multiplet, brs = broad singlet, brd = broad doublet, coupling constant(s) in Hertz.

**HRMS spectra** were performed on a Bruker maXis mass spectrometer.

**IR** - **Infra Red Spectrometry.** IR spectra were recorded on a FTIR Bruker ATR Vertex 70 spectrometer in the range of wavelength of 4000-200 cm⁻¹

### Synthesis of dialkylphosphite

General protocollipid alcohol and diphenylphosphite (1 eq.) were placed in a Kugelroch distillation apparatus and heating at 130 °C under vacuum (2.4 10⁻⁴ mbar). The reaction was monitored by ¹H NMR and ³¹P and stopped when the reaction is over and all traces of phenol have been eliminated. If needed the temperature is increased to remove the excess of alcohol.

### O,O-bis(2-dodecylhexadecyl)phosphite

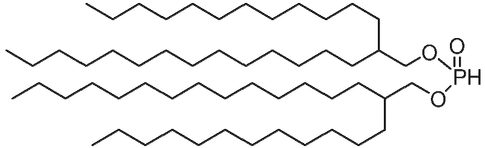

2-dodecylhexadecanol (3.64 g, 8.86 mmol, 2.05 eq.), diphenylphosphite (1.00 g, 4.2 mmol, 1.0 eq.). The undistilled product is collected as a colorless oil (3.2 g, 87 % yield; purity 95%, 5 % of alcohol). ¹H NMR (CDCl₃, 500.0 MHz): δ = 0.87 (t, ³J_{HH} = 6.8 Hz, CH₃, 12H), 1.25 (broad, 96H), 1.61 (broad, CH, 2H), 3.95 (t, ³J_{HH} = ³J_{HP} = 6.2 Hz, CH₂-O, 4H), 6.78 (d, ¹J_{HP} = 690.6 Hz, P-H, 1H); ³¹P{¹H} (CDCl₃, 202.4 MHz): δ = 8.74; ¹³C (CDCl₃, 125.7 MHz): δ = 16.81 (CH₃), 25.40 (CH₂), 29.37 (CH₂), 32.09 (CH₂), 32.34 (CH₂), 32.42 (CH₂), 32.67 (CH₂), 33.44 (CH₂), 34.65 (CH₂), 41.41 (d, ³J_{CP} = 6.4 Hz, CH), 70.82 (d, ²J_{CP} = 6.0 Hz, CH₂-O); IR (ATR, neat): 2916.09, 2849.63, 1468.14, 1254.55 (v P=O), 968.98

### O,O-bis(2-decyltetradecyl)phosphite

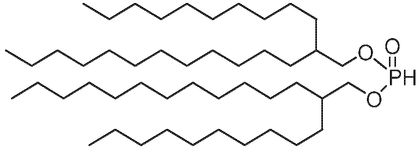

2-decyltetradecanol (8.33 g, 23.5 mmol, 2.2 eq.), diphenylphosphite (2.5 g, 10.6 mmol, 1.0 eq.). The undistilled product is collected as a colorless oil (7.76 g, 96 % yield). ¹H NMR (CDCl₃, 500.0 MHz): δ = 0.87 (t, ³J_{HH}= 6.8 Hz, CH₃, 12H), 1.25 (broad, 84H), 1.61 (broad, CH, 2H), 3.95 (t, ³J_{HH} = ³J_{HP} = 6.3 Hz, CH₂-O, 4H), 6.78 (d, ¹J_{HP} = 690.6 Hz, P-H, 1H); ³¹P{¹H} (CDCl₃, 202.4 MHz): δ = 8.74 ; ¹³C (CDCl₃, 125.7 MHz): δ = 16.80 (CH₃), 25.40 (CH₂), 29.36 (CH₂), 32.07 (CH₂), 32.33 (CH₂), 32.38 (CH₂), 32.66 (CH₂), 33.43 (CH₂), 34.64 (CH₂), 41.41 (d, ³J_{CP} = 6.5 Hz, CH), 70.81 (d, ²J_{CP} = 6.1 Hz, CH₂-O); IR (ATR, neat): 2920.77, 2852.11, 1465.45, 1261.32 (v P=O), 967.76, 721.13

### O,O-bis(2-octyldodecyl)phosphite

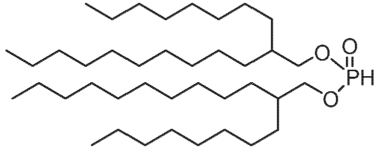

2-octyldodecanol (4.14 g, 13.0 mmol, 2.5 eq.), diphenylphosphite (1.3 g, 5.5 mmol, 1.0 eq.). The undistilled product is collected as a colorless oil (3.34 g, 90 % yield). ¹H NMR (CDCl₃, 500.0 MHz): δ = 0.87 (t, ³J_{HH} = 6.8 Hz, CH₃, 12H), 1.25 (broad, 64H), 1.61 (broad, CH, 2H), 3.95 (t, ³J_{HH} = ³J_{HP} = 6.2 Hz, CH₂-O, 4H), 6.78 (d, ¹J_{HP} = 690.8 Hz, P-H, 1H); ³¹P{¹H} (CDCl₃, 202.4 MHz): δ = 8.74 ; ¹³C (CDCl₃, 125.7 MHz): δ = 16.81 (CH₃), 25.39 (CH₂), 29.36 (CH₂), 32.04 (CH₂), 32.06 (CH₂), 32.28 (CH₂), 32.33 (CH₂), 32.35 (CH₂), 32.38 (CH₂), 32.66 (CH₂), 33.43 (CH₂), 34.63 (CH₂), 41.41 (d, ³J_{CP} = 6.5 Hz, CH), 70.84 (d, ²J_{CP} = 6.0 Hz, CH₂-O); IR (ATR, neat): 2921.27, 2852.75, 1465.42, 1260.65 (v P=O), 966.87, 721.45

### O,O-bis(2-hexyldecanyl)phosphite

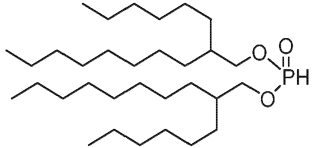

2-hexyldecanol (2.59 g, 10.6 mmol, 2.5 eq.), diphenylphosphite (1.0 g, 4.27 mmol, 1.0 eq.). The undistilled product is collected as a colorless oil (2.11 g, 93 % yield). ¹H NMR (CDCl₃, 500.0 MHz): δ = 0.87 (t, ³J_{HH} = 6.8 Hz, CH₃, 12H), 1.25 (broad, 48H), 1.61 (broad, CH, 2H), 3.95 (t, ³J_{HH} = ³J_{HP} = 6.1 Hz, CH₂-O, 4H), 6.78 (d, ¹J_{HP} = 690.9 Hz, P-H, 1H); ³¹P{¹H} (CDCl₃, 202.4 MHz): δ = 8.76; ¹³C (CDCl₃, 125.7 MHz): δ = 16.77 (CH₃), 25.35 (CH₂), 29.31 (CH₂), 29.34 (CH₂), 32.02 (CH₂), 32.26 (CH₂), 32.30 (CH₂), 32.64 (CH₂), 33.42 (CH₂), 34.50 (CH₂), 34.59 (CH₂), 41.40 (d, ³J_{CP} = 6.5 Hz, CH), 70.83 (d, ²J_{CP} = 6.0 Hz, CH₂-O); IR (ATR, neat): 2922.56, 2854.09, 1465.26, 1259.84 (v P=O), 967.76, 722.53.

### O,O-bis(2-butyloctyl)phosphite

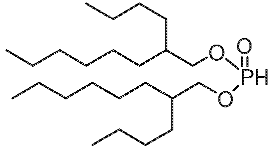

2-butyloctanol (7.95 g, 42.7 mmol, 2.5 eq.), diphenylphosphite (4.0 g, 17.0 mmol, 1.0 eq.). The undistilled product is collected as a colorless oil (6.66 g, 94 % yield). ¹H NMR (CDCl₃, 500.0 MHz): δ = 0.86 (m, CH₃, 12H), 1.25 (broad, 32H), 1.61 (broad, CH, 2H), 3.93 (m, CH₂-O, 4H), 6.77 (d, ¹J_{HP} = 690.9 Hz, P-H, 1H); ³¹P{¹H} (CDCl₃, 202.4 MHz): δ = 8.74; ¹³C (CDCl₃, 125.7 MHz): δ = 16.71 (CH₃), 16.76 (CH₃), 25.34 (CH₂), 25.64 (CH₂), 29.30 (CH₂), 31.53 (CH₂), 32.29 (CH₂), 33.10 (CH₂), 33.42 (CH₂), 34.50 (CH₂), 41.38 (d, ³J_{CP} = 6.5 Hz, CH), 70.83 (d, ²J_{CP} = 6.0 Hz, CH₂-O); IR (ATR, neat): 2924.91, 2856.89, 1466.01, 1259.45 (v P=O), 964.33, 725.68.

### Bis-(7-ethyl-2-methylundecan-4-yl)phosphite

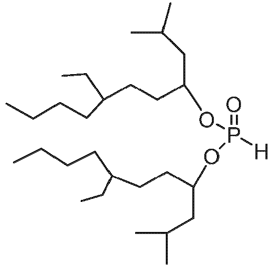

7-ethyl-2-methylundecan-4-ol (4.81 g, 22.4 mmol, 2.1 eq.), diphenylphosphite (2.5 g, 10.6 mmol, 1.0 eq.). The unstilled product is collected as a colorless oil as a mixture of diastereoisomers (4.75 g, 94 % yield). ¹H NMR (CDCl₃, 500.0 MHz): δ = 0.84-0.97 (m, CH₃, 24H), 1.25-1.31 (m, CH, CH₂, 24H), 1.60-1.64 (m, CH₂ β-O, 6H), 1.73-1.80 (?), 4.48-4.54 (m, CH₂ α-O, 4H), 6.93 (d, ¹J_{HP} = 681.18 Hz, P-H, 1H); ³¹P{¹H} (CDCl₃, 202.4 MHz): δ = 6.12; ¹³C (CDCl₃, 125.7 MHz): δ = 10.66, 13.99, 21.85, 21.96, 22.95, 23.08, 24.23, 24.37, 25.52, 25.62, 28.19, 28.26, 28.29, 28.79, 32.52, 32.56, 32.63, 32.89, 33.22, 33.23, 38.58, 38.62, 38.70, 44.36, 44.40, 44.56, 77.12, 77.27; IR (ATR, neat): 2955.75, 2870.48, 1462.46, 1258.32 (v P=O), 963.26.

### O,O-bis(3,7,11,15-tetramethylhexadecyl) phosphite

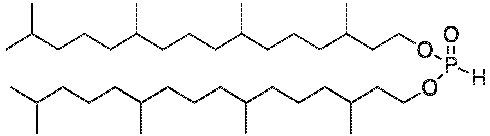

3,7,11,15-tetramethylhexadecan-1-ol (2.03 g, 6.80 mmol, 2.1 eq.) and diphenylphosphite (0.758 g, 3.24 mmol, 1.0 eq.). The undistilled product was collected as a colorless oil (1.66 g, 80 % yield). ¹H NMR (CDCl₃, 400.0 MHz): δ = 0.82-0.90 (m, CH₃, 30H), 1.03-1.35 (broad, 40H), 1.46-1.58 (broad, CH, 6H), 1.70-1.73 (broad, CH, 2H), 4.07-4.12 (m, CH₂-O, 4H), 6.79 (d, ¹J_{HP} = 691.81 Hz, P-H, 1H); ³¹P{¹H} (CDCl₃, 202.4 MHz): δ = 8.19; ¹³C (CDCl₃, 125.7 MHz): δ = 19.31 (CH₃), 19.37 (CH₃), 19.69 (CH₃), 19.76 (CH₃), 22.64 (CH₃), 22.73 (CH₃), 24.30 (CH₂), 24.48 (CH₂), 24.81 (CH₂), 27.98 (CH), 29.36 (CH), 32.78 (CH), 37.21 (CH₂), 37.30 (CH₂), 37.35 (CH₂), 37.40 (CH₂), 37.46 (CH₂), 39.38 (CH₂ β-O), 64.25 (d, ²J_{CP} = 5.9 Hz, CH₂ α-O); IR (ATR, neat): 2924.23, 2867.76, 1461.69, 1378.01, 1262.00 (v P=O), 966.32.

### O,O-bis(pentadecan-8-yl) phosphite

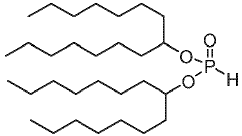

Diphenylphosphite (3.29 mmol; 0.77 g) ; diheptylcarbinol (6.58 mmol; 1.50 g), Yield: 62% (1.02 g). ¹H NMR (CDCl₃, 400.0 MHz): δ = 0.81 (t, ³J_{HH} = 6.9 Hz, CH₃, 12H), 1.18-1.36 (broad, CH₂, 40H), 1.53 (m broad, CH₂, 8H), 4.37 (m, CH, 2H), 6.81 (d, ¹J_{PH} = 686.3 Hz, PH, 1H); ³¹P{¹H} (CDCl₃, 161.9 MHz): δ = 5.93 (s, P); IR (ATR, neat) : 2915, 2849, 1463, 1376, 1256 (v P=O), 961 (P-O), 722.

### O,O-bis(heptadecan-9-yl) phosphite

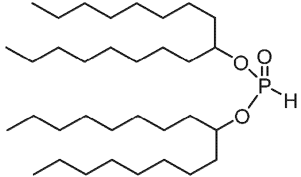

Dioctylcarbinol (4.0 g, 15.6 mmol, 2.03 eq.), diphenylphosphite (1.8 g, 7.68 mmol, 1.0 eq.). The undistilled product was collected as a colorless oil (4.29 g, 100 % yield). ¹H NMR (CDCl₃, 400.0 MHz): δ = 0.79 (t, ³J_{HH}= 6.7 Hz; CH₃, 12H), 1.17-1.30 (broad, 64H), 1.46-1.58 (broad, CH, 6H), 1.47-1.57 (broad, 8H), 4.33 (m, CH-O, 2H), 6.90 (d, ¹J_{HP} = 685.9 Hz, P-H, 1H) ; ³¹P{¹H} (CDCl₃, 100.6 MHz): δ = 6.33; IR (ATR, neat) : 2916, 2848, 1463, 1256 (v P=O), 967, 721.

### O,O-bis(heneincosan-11-yl) phosphite

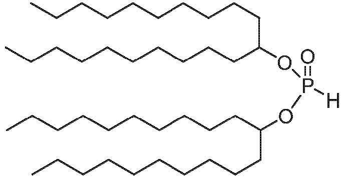

Decanoylcarbinol (1.5 g, 4.80 mmol, 2.0 eq.), diphenylphosphite (0.56 g, 2.40 mmol, 1.0 eq.). The undistilled product was collected as a colorless oil (1.41 g, 88 % yield). ¹H NMR (CDCl₃, 400.0 MHz) : δ = 0.79 (t, ³J_{HH}= 6.7 Hz; CH₃, 12H), 1.17-1.30 (broad, 64H), 1.46-1.58 (broad, CH, 6H), 1.47-1.57 (broad, 8H), 4.33 (m, CH-O, 2H), 6.90 (d, ¹J_{HP} = 685.9 Hz, P-H, 1H) ; ³¹P{¹H} (CDCl₃, 161.92 MHz): δ = 5.96; ¹³C (CDCl₃, 100.6 MHz): δ = 13.97 (CH₃), 22.55 (CH₂), 25.02 (CH₂), 25.16 (CH₂), 29.20 (CH₂), 29.22 (CH₂), 29.36 (CH₂), 29.41 (CH₂), 29.43 (CH₂), 29.46(CH₂), 29.51 (CH₂), 31.78 (CH), 35.20 (d, ³J_{CP} = 4.2Hz, CH₂), 35.49 (d, ³J_{CP} = 3.6 Hz, CH₂), 78.38 (d, ²J_{CP} = 6.4 Hz, CH-O); IR (neat) : 2915, 2847, 1463, 1257 (v P=O), 960, 720.

### O,O-bis(nonadecan-10-yl) phosphite

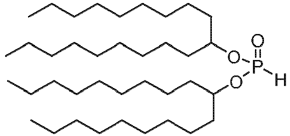

Diphenylphosphite (2.63 mmol; 617 mg); dinonylcarbinol (5.27 mmol; 1.5 g), Yield: 88% (1.42g). ¹H NMR (CDCl₃, 400.0 MHz) : δ = 0.89 (t, ³J_{HH}= 6.8 Hz, CH₃, 12H), 1.27-1.42 (broad, CH₂, 56H), 1.60 (broad, CH₂, 8H), 4.44 (m, CH, 2H), 6.88 (d, ¹J_{PH} = 686.5 Hz, PH, 1H); ³¹P{¹H} (CDCl₃, 161.92 MHz): δ = 6.30 (s, P); ¹³C (CDCl₃, 100.6 MHz): δ = 13.93 (CH₃), 22.53 (CH₂), 25.00 (CH₂), 25.14 (CH₂), 29.19 (CH₂), 29.34 (CH₂), 29.40 (CH₂), 29.44(CH₂), 29.58 (CH₂), 31.75 (CH₂), 35.19 and 35.47 (d, ³J_{CP} = 4.2 and 3.8 Hz, CH₂), 78.38 (d, ²J_{CP} = 6.4 Hz, CH-O); IR (ATR, neat) : 2916, 2847, 1463, 1375, 1256 (v P=O), 958 (P-O), 720.

### O,O-bis(heneincosan-11-yl) phosphite

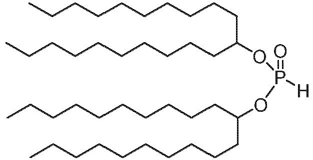

didecanoylcarbinol (1.5 g, 4.80 mmol, 2.0 eq.), diphenylphosphite (0.56 g, 2.40 mmol, 1.0 eq.). The undistilled product was collected as a colorless oil (1.41 g, 88 % yield). ¹H NMR (CDCl₃, 400.0 MHz): δ = 0.79 (t, ³J_{HH}= 6.7 Hz; CH₃, 12H), 1.17-1.30 (broad, 64H), 1.46-1.58 (broad, CH, 6H), 1.47-1.57 (broad, 8H), 4.33 (m, CH-O, 2H), 6.90 (d, ¹J_{HP} = 685.9 Hz, P-H, 1H) ; ³¹P{¹H} (CDCl₃, 161.9 MHz): δ = 5.96; ¹³C (CDCl₃, 100.6 MHz): δ = 13.97 (CH₃), 22.55 (CH₂), 25.02 (CH₂), 25.16 (CH₂), 29.22 (CH₂), 29.36 (CH₂), 29.46(CH₂), 29.51 (CH₂), 31.78 (CH₂), 35.20 and 35.49 (2d, ³J_{CP} = 4.2 and 3.6 Hz, CH₂), 78.38 (d, ²J_{CP} = 6.4 Hz, CH-O); IR (ATR, neat) : 2915, 2847, 1463, 1257 (v P=O), 960, 720.

### O,O-bis(heneincosan-11-yl) phosphite

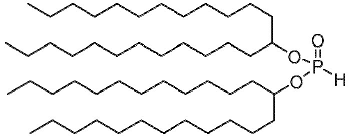

Diphenylphosphite (2.04 mmol; 0.48g); didodecylcarbinol (4.08 mmol; 1.5 g), yield: 88% (1.41g). ¹H NMR (CDCl₃, 400.0 MHz): δ = 0.90 (t, ³J_{HH}= 6.8 Hz, CH₃, 12H), 1.28-1.44 (broad, CH₂, 80H), 1.62 (broad, CH₂, 8H), 4.44 (m, CH, 2H), 6.90 (d, ¹J_{PH} = 686.3 Hz, PH, 1H); ³¹P{¹H} (CDCl₃, 161.9 MHz): δ = 6.34 (s, P), ¹³C (CDCl₃, 100.6 MHz): δ = 13.97 (CH₃), 22.55 (CH₂), 25.02 (CH₂), 26.16 (CH₂), 29.23 (CH₂), 29.36 (CH₂), 29.42 (CH₂), 29.46 (CH₂), 29.52 (CH₂), 29.56 (CH₂), 31.79 (CH₂), 35.20 and 35.49 (d, ³J_{HP} = 4.2 and 3.4 Hz, CH₂-CH-O), 78.40 (d, ²J_{CP} = 6.4 Hz, CH-O).

### General protocol for Atherton-Todd reaction.

In a 50 mL round-bottom flask are place the dialkylphosphite (1 eq.), amine of formula (III) (1.3 eq.) and diisopropylethylamine (DIPEA; 1.3 eq.). The flask was placed under nitrogen atmosphere and cooled with ice. Then 5 mL of dichloromethane was added. Then, bromotrichloromethane (1.3 eq.) in solution in dichloromethane (2 mL) was added dropwise. At the end of the addition the solution was stirred at 4°C for 30 min and then 1h30 at 20°C. Solvent and the excess of volatile reagents were removed under vacuum. Diethylether was added to the residue, mixed and filtered on celite. The filtrate was evaporated. Dichloromethane (50 mL) was added and the solution was washed with water (3*20 mL), dried over MgSO₄, filtered and concentrated. The final product was purified by chromatography on silica gel.

### O,O-bis(2-dodecylhexadecyl)-N-(2-(dimethylamino)ethyl)phosphoramidate (compound 001)

Bis(2-dodecylhexadecyl)phosphite (0.6 g, 0.69 mmol, 1 eq.), *N*,*N-*dimethylethylenediamine (0.08 g, 0.90 mmol, 1.3 eq.). Chromatography on silica gel CH₂Cl₂/MeOH from 100/0.5 to 100/7 to produce 0.57 g of colorless viscous oil (Yield: 87%). R_{f} (CH₂Cl₂/MeOH: 100/10 (v/v)): 0.43; ¹H NMR (CDCl₃, 500.0 MHz): δ = 0.87 (t, ³J_{HH} = 6.9 Hz, CH₃, 12H), 1.24 (broad, 96H), 1.59 (broad, CH, 2H), 2.21 (s, N-CH₃, 6H), 2.38 (t, ³J_{HH} = 5.6 Hz, CH₂-NMe₂, 2H), 2.94 (q, NH-CH₂, 2H), 3.23 (s broad, NH, 1H), 3.86 (m, CH₂-O, 4H); ³¹P{¹H} (CDCl₃, 202.4 MHz): δ = 10.0; ¹³C (CDCl₃, 125.7 MHz): δ = 16.82 (CH₃), 25.41 (CH₂), 29.43 (CH₂), 32.09 (CH₂), 32.39 (CH₂), 32.44 (CH₂), 32.73 (CH₂), 33.58 (CH₂), 33.63 (CH₂), 34.65 (CH₂), 41.37 (CH₂-NH), 41.38 (d, ³J_{CP} = 8.1 Hz, CH), 47.79 (NMe₂), 62.37 (d, ³J_{CP} = 6.8 Hz, CH₂-NMe₂), 71.42 (d, ²J_{CP} = 5.6 Hz, CH₂-O); IR (ATR, neat): 3207.28; 2920.58; 2851.90; 1464.73; 1235.27 (v P=O); 1004.16; 720.89; HRMS (ESI-qTOF), m/z calcd for C₆₀H₁₂₅N₂O₃P+H [M+H]⁺= 953.9506; observed [M+H]⁺= 953.9576;

### O,O-bis(2-dodecylhexadecyl)-N-methyl-N-(2-(dimethylamino)ethyl)phosphoramidate (compound 002)

Bis(2-dodecylhexadecyl)phosphite (0.62 g, 0.72 mmol, 1 eq.), *N*,*N*,*N*'-trimethylethylenediamine (0.10 g, 0.94 mmol, 1.3 eq.). Chromatography on silica gel CH₂Cl₂/MeOH from 100/0.5 to 100/10 to produce 0.57 g of colorless viscous oil (Yield: 81%). R_{f} (CH₂Cl₂/MeOH: 100/10 (v/v)): 0.40; ¹H NMR (CDCl₃, 500.0 MHz): 0.86 (t, ³J_{HH} = 6.9 Hz, CH₃, 12H), 1.28 (broad, 96H), 1.58 (broad, CH, 2H), 2.25 (s, N-CH₃, 6H), 2.43 (t, ³J_{HH}= 7.2 Hz, CH₂-NMe₂, 2H), 2.66 (d, ³J_{HH}= 9.7 Hz, CH₃-N, 3H), 3.11 (q, NMe-CH₂, 2H), 3.79 and 3.85 (2m, CH₂-O, 4H); ³¹P{¹H} (CDCl₃, 202.4 MHz): 10.9 ; ¹³C (CDCl₃, 125.7 MHz): 16.82 (CH₃), 25.40 (CH₂), 29.42 (CH₂), 32.09 (CH₂), 32.39 (CH₂), 32.43 (CH₂), 32.77 (CH₂), 33.58 (CH₂), 33.66 (CH₂), 34.64 (CH₂), 36.73 (d, ²J_{CP} = 3.1 Hz, N-CH₃), 41.36 (d, ³J_{CP} = 7.5 Hz, CH), 48.37 (NMe₂), 49.93 (CH₂-NMe), 60.32 (CH₂-NMe₂), 71.24 (d, ²J_{CP} = 5.7 Hz, CH₂-O); IR (ATR, neat): 2920.82; 2851.49; 1466.69; 1259.41 (v P=O); 996.63; 721.03;

### O,O-bis(2-decyltetradecyl)-N-(2-(dimethylamino)ethyl)phosphoramidate (compound 003)

Bis(2-dodecylhexadecyl)phosphite (1.5 g, 1.99 mmol, 1 eq.), *N*,*N-*dimethylethylenediamine (194 mg, 2.20 mmol, 1.1 eq.). Chromatography on silica gel CH₂Cl₂/MeOH from 100/0.5 to 100/7 to produce 1.57 g of colorless viscous oil (Yield: 80%). R_{f} (CH₂Cl₂/MeOH: 100/10 (v/v)): 0.42; ¹H NMR (CDCl₃, 400.0 MHz): δ = 0.87 (t, ³J_{HH} = 6.6 Hz, CH₃, 12H), 1.24 (broad, 80H), 1.59 (broad, CH, 2H), 2.21 (s, N-CH₃, 6H), 2.37 (t, ³J_{HH} = 5.7 Hz, CH₂-NMe₂, 2H), 2.94 (q, NH-CH₂, 2H), 3.21 (s broad, NH, 1H), 3.86 (m, CH₂-O, 4H); ³¹P{¹H} (CDCl₃, 202.4 MHz): δ = 9.95; ¹³C (CDCl₃, 125.7 MHz): δ = 13.91 (CH₃), 22.51 (CH₂), 26.53 (CH₂), 29.19 (CH₂), 29.49 (CH₂), 29.53 (CH₂), 29.86 (CH₂), 30.69 (CH₂), 30.83 (CH₂), 31.76 (CH₂-NH), 38.48 (d, ³J_{CP} = 8.1 Hz, CH), 45.02 (NMe₂), 59.40 (d, ³J_{CP} = 8.4 Hz, CH₂-NMe₂), 68.44 (d, ²J_{CP} = 7.1 Hz, CH₂-O); IR (ATR, neat): 3205 ; 2915 ; 2847 ; 1462 ; 1232 (v P=O); 1002 ; 870.

### O,O-bis(2-decyltetradecyl)-N-methyl-N-(2-(dimethylamino)ethyl)phosphoramidate (compound 004)

Bis(2-decyltetradecyl)phosphite (0.5 g, 0.66 mmol, 1 eq.), *N*,*N*,*N*'-trimethylethylenediamine (0.09 g, 0.86 mmol, 1.3 eq.). Chromatography on silica gel CH₂Cl₂/MeOH from 100/0.5 to 100/6 to produce 0.48 g of colorless viscous oil (Yield: 85%). R_{f} (CH₂Cl₂/MeOH: 100/10 (v/v)): 0.60; ¹H NMR (CDCl₃, 500.0 MHz): 0.87 (t, ³J_{HH} = 6.9 Hz, CH₃, 12H), 1.24 (broad, 80H), 1.59 (broad, CH, 2H), 2.25 (s, N-CH₃, 6H), 2.44 (t, ³J_{HH} = 7.2 Hz, CH₂-NMe₂, 2H), 2.66 (d, ³J_{HH} = 9.7 Hz, CH₃-N, 3H), 3.11 (m, NMe-CH₂, 2H), 3.79 and 3.85 (2m, CH₂-O, 4H); ³¹P{¹H} (CDCl₃, 202.4 MHz): 10.9; ¹³C (CDCl₃, 125.7 MHz): 14.10 (CH₃), 22.69 (CH₂), 26.71 (CH₂), 29.37 (CH₂), 29.67 (CH₂), 29.71 (CH₂), 30.05 (CH₂), 30.87(CH₂), 30.95 (CH₂), 31.93 (CH₂), 34.00 (d, ²J_{CP} = 3.4 Hz, N-CH₃), 38.64 (d, ³J_{CP} = 7.5 Hz, CH), 45.65 (NMe₂), 47.21 (d, ²J_{CP} = 2.9 Hz, CH₂-NMe), 57.61 (CH₂-NMe₂), 68.52 (d, ²J_{CP} = 5.8 Hz, CH₂-O); IR (ATR, neat): 2921.20; 2851.85; 1466.66; 1259.28 (v P=O); 999.85; 721.27; HRMS (ESI-qTOF), m/z calcd for C₅₃H₁₁₁N₂O₃P+H [M+H]⁺= 855.8411; observed [M+H]⁺=855.8476;

### O,O-bis(2-octyldodecyl)-N-(2-(dimethylamino)ethyl)phosphoramidate (compound 005)

Bis(2-octyldodecyl)phosphite (0.5 g, 0.74 mmol, 1 eq.), *N*,*N*-dimethylethylenediamine (0.08 g, 0.97 mmol, 1.3 eq.). Chromatography on silica gel CH₂Cl₂/MeOH from 100/0.5 to 100/7 to produce 0.52 g of colorless viscous oil (Yield: 96%). R_{f} (CH₂Cl₂/MeOH: 100/7 (v/v)): 0.31; ¹H NMR (CDCl₃, 500.0 MHz): 0.87 (t, ³J_{HH}= 6.9 Hz, CH₃, 12H), 1.25 (broad, 64H), 1.60 (broad, CH, 2H), 2.22 (s, N-CH₃, 6H), 2.39 (t, ³J_{HH} = 5.6 Hz, CH₂-NMe₂, 2H), 2.95 (m, NH-CH₂, 2H), 3.24 (broad, NH, 1H), 3.86 (m, CH₂-O, 4H); ³¹P{¹H} (CDCl₃, 202.4 MHz): 10.0; ¹³C (CDCl₃, 125.7 MHz): 16.81 (CH₃), 25.39 (CH₂), 29.42 (CH₂), 32.07 (CH₂), 32.32 (CH₂), 32.37 (CH₂), 32.41 (CH₂), 32.74 (CH₂), 33.57 (CH₂), 33.62 (CH₂), 34.63 (CH₂), 41.37 (d, ³J_{CP} = 8.0 Hz, CH), 41.38 (CH₂-NH), 47.79 (NMe₂), 62.34 (d, ³J_{CP} = 6.8 Hz, CH₂-NMe₂), 71.40 (d, ²J_{CP} = 5.7 Hz, CH₂-O); IR (ATR,neat): 3204.80, 2921.63, 2852.90, 1463.98, 1234.81 (v P=O), 1000.56, 854.38, 721.41; HRMS (ESI-qTOF), m/z calcd for C₄₅H₉₃N₂O₃P+H [M+H]⁺= 729.7002; observed [M+H]⁺=729.7041;

### O,O-bis(2-octyldodecyl)-N-methyl-N-(2-(dimethylaminoethyl)phosphoramidate (compound 006)

Bis(2-octyldodecyl)phosphite (0.5 g, 0.74 mmol, 1 eq.), *N*,*N*,*N*'-trimethylethylenediamine (0.10 g, 0.97 mmol, 1.3 eq.). Chromatography on silica gel CH₂Cl₂/MeOH from 100/0.5 to 100/7 to produce 0.43 g of colorless viscous oil (Yield: 78%). R_{f} (CH₂Cl₂/MeOH: 100/7 (v/v)): 0.21; ¹H NMR (CDCl₃, 500.0 MHz): 0.87 (t, ³J_{HH} = 6.9 Hz, CH₃, 12H), 1.25 (broad, 64H), 1.59 (broad, CH, 2H), 2.26 (s, N-CH₃, 6H), 2.44 (t, ³J_{HH} = 7.2 Hz, CH₂-NMe₂, 2H), 2.66 (d, ³J_{HH} = 9.7 Hz, CH₃-N, 3H), 3.12 (m, NMe-CH₂, 2H), 3.79 and 3.85 (2m, CH₂-O, 4H); ³¹P{¹H} (CDCl₃, 202.4 MHz): 10.9; ¹³C (CDCl₃, 125.7 MHz): 16.81 (CH₃), 25.39 (CH₂), 29.41 (CH₂), 32.07 (CH₂), 32.33 (CH₂), 32.37 (CH₂), 32.41 (CH₂), 32.75 (CH₂), 33.57 (CH₂), 33.65 (CH₂), 34.63 (CH₂), 36.72 (d, ²J_{CP} = 3.3 Hz, N-CH₃), 41.35 (d, ³J_{CP} = 7.5 Hz, CH), 48.37 (N-Me₂), 49.93 (d, ²J_{CP} = 2.8 Hz, CH₂-N), 60.33 (CH₂-NMe₂), 71.23 (d, ²J_{CP} = 5.7 Hz, CH₂-O); IR (ATR, neat): 2921.65, 2852.84, 1463.23, 1258.60 (v P=O), 996.34, 721.50; HRMS (ESI-qTOF), m/z calcd for C₄₅H₉₅N₂O₃P+H [M+H]⁺= 743.7159; observed [M+H]⁺=743.7195;

### O,O-bis(2-hexyldecyl)-N-(2-(dimethylamino)ethyl)phosphoramidate (compound 007)

Bis(2-hexyldecyl)phosphite (0.50 g, 0.94 mmol, 1 eq.), *N*,*N*-dimethylethylenediamine (0.11 g, 1.2 mmol, 1.3 eq.). Chromatography on silica gel CH₂Cl₂/MeOH from 100/0.5 to 100/7 to produce 0.49 g of colorless viscous oil (Yield: 83%). R_{f} (CH₂Cl₂/MeOH: 100/10 (v/v)): 0.47; ¹H NMR (CDCl₃, 500.0 MHz): 0.86 (t, ³J_{HH} = 6.8 Hz, CH₃, 12H), 1.24 (broad, 48H), 1.59 (broad, CH, 2H), 2.21 (s, N-CH₃, 6H), 2.37 (t, ³J_{HH} = 5.8 Hz, CH₂-NMe₂, 2H), 2.94 (m, NH-CH₂, 2H), 3.22 (broad, NH, 1H), 3.85 (m, CH₂-O, 4H); ³¹P{¹H} (CDCl₃, 202.4 MHz): 10.0; ¹³C (CDCl₃, 125.7 MHz): 16.78 (CH₃), 25.36 (CH₂), 29.36 (CH₂), 29.40 (CH₂), 32.04 (CH₂), 32.30 (CH₂), 32.37 (CH₂), 32.72 (CH₂), 33.56 (CH₂), 33.61 (CH₂), 34.54 (CH₂), 34.61 (CH₂), 41.37 (CH₂-NH), 41.36 (d, ³J_{CP} = 8.0 Hz, CH), 41.37 (CH₂-NH), 47.77 (NMe₂), 62.31 (d, ³J_{CP} = 6.9 Hz, CH₂-NMe₂), 71.37 (d, ²J_{CP} = 5.8 Hz, CH₂-O); IR (ATR, neat): 3212.17, 2922.55, 2854.08, 1459.04, 1234.80 (v P=O), 1004.20, 856.70, 722.45; HRMS (ESI-qTOF), m/z calcd for C₃₆H₇₇N₂O₃P+H [M+H]⁺= 617.5750; observed [M+H]⁺=617.5765;

### O,O-bis(2-hexyldecyl)-N-methyl-N-(2-(dimethylamino)ethyl)phosphoramidate (compound 008)

Bis(2-hexyldecyl)phosphite (0.5 g, 0.94 mmol, 1 eq.), *N*,*N*,*N'*-trimethylethylenediamine (0.12 g, 1.22 mmol, 1.3 eq.). Chromatography on silica gel CH₂Cl₂/MeOH from 100/0.5 to 100/7 to produce 0.32 g of colorless viscous oil (Yield: 54%). R_{f} (CH₂Cl₂/MeOH: 100/10 (v/v)): 0.43; ¹H NMR (CDCl₃, 500.0 MHz): 0.86 (t, ³J_{HH} = 6.8 Hz, CH₃, 12H), 1.24 (broad, 48H), 1.58 (broad, CH, 2H), 2.24 (s, N-CH₃, 6H), 2.43 (t, ³J_{HH} = 7.2 Hz, CH₂-NMe₂, 2H), 2.66 (d, ³J_{HH} = 9.7 Hz, CH₃-N, 3H), 3.10 (m, NMe-CH₂, 2H), 3.78 and 3.84 (2m, CH₂-O, 4H); ³¹P{¹H} (CDCl₃, 202.4 MHz): 10.9; ¹³C (CDCl₃, 125.7 MHz): 14.07 (CH₃), 22.66 (CH₂), 26.64 (CH₂), 26.68 (CH₂), 29.34 (CH₂), 29.60 (CH₂), 29.67 (CH₂), 30.02 (CH₂), 30.86 (CH₂), 30.94 (CH₂), 31.84 (CH₂), 31.89 (CH₂), 33.98 (d, ²J_{CP} = 3.3 Hz, N-CH₃), 38.63 (d, ³J_{CP} = 7.6 Hz, CH), 45.64 (N-Me₂), 47.20 (d, ²J_{CP} = 3.1 Hz, CH₂-N), 57.60 (CH₂-NMe₂), 68.50 (d, ²J_{CP} = 5.8 Hz, CH₂-O); IR (ATR, neat): 2922.58, 2853.26, 1466.79, 1258.59 (v P=O), 993.91, 722.54; HRMS (ESI-qTOF), m/z calcd for C₃₇H₇₉N₂O₃P+H [M+H]⁺= 631.5907; observed [M+H]⁺=631.5906;

### O,O-bis(7-ethyl-2-methylundecan-4-yl)-N-(2-(dimethylamino)ethyl)phosphoramidate (compound 009)

Bis-(7-ethyl-2-methylundecan-4-yl)phosphite (0.50 g, 1.05 mmol, 1 eq.), *N*,*N-*dimethylethylenediamine (0.11 g, 1.2 mmol, 1.2 eq.). Chromatography on silica gel CH₂Cl₂/MeOH from 100/0.5 to 100/7 to produce 0.30 g of colorless viscous oil as a mixture of diastereoisomers (Yield: 55%). R_{f} (CH₂Cl₂/MeOH: 100/4 (v/v)): 0.10; ¹H NMR (CDCl₃, 500.0 MHz): 0.81-0.93 (m, CH₃, 24H), 1.21-1.36 (m, 24H), 1.52-1.60 (m, 6H), 1.73 (m, 2H), 2.21 (s, N-CH₃, 6H), 2.38 (s broad, CH₂-NMe₂, 2H), 2.96 (s broad, NH-CH₂, 2H), 3.13 (broad, NH, 1H), 4.35 (m, CH-O, 2H); ³¹P{¹H} (CDCl₃, 202.4 MHz): 7.66, 7.78 and 7.91; ¹³C (CDCl₃, 125.7 MHz): 13.50 (CH₃), 16.84 (CH₃), 25.16, 25.20, 25.24, 25.74, 25.81 (CH₂), 27.08, 27.18, 28.42 (CH₂), 30.65-30.69 (m, CH₂), 31.65 (CH₂), 35.30-35.51 (m, CH₂), 41.49 (CH₂), 41.57, 41.64, 46.92-46.94 (m, CH₂), 47.75 (N-Me₂), 62.32 (d, ³J_{CP} = 7.5 Hz, CH₂-NMe₂), 79.24-79.75 (m, CH-O); IR (ATR, neat): 3195.12, 2955.09, 2926.94, 2860.18, 1459.28, 1379.34, 1231.98 (v P=O), 964.72, 727.45;

### O,O-bis(7-ethyl-2-methylundecan-4-yl)-N-methyl-N-(2-(dimethylamino)ethyl)phosphoramidate (compound 010)

Bis-(7-ethyl-2-methylundecan-4-yl)phosphite (0.50 g, 1.05 mmol, 1 eq.), *N*,*N*,*N*'-trimethylethylenediamine (0.14 g, 1.4 mmol, 1.3 eq.). Chromatography on silica gel CH₂Cl₂/MeOH from 100/0.5 to 100/7 to produce 0.38 g of colorless viscous oil as a mixture of diastereoisomers (Yield: 63%). R_{f}(CH₂Cl₂/MeOH: 100/4 (v/v)): 0.09-0.11 (3 spots); ¹H NMR (CDCl₃, 500.0 MHz): 0.80-0.93 (m, CH₃, 24H), 1.21-1.31 (m, 24H), 1.51-1.59 (m, 6H), 1.70-1.74 (m, 2H), 2.25 (s, N-CH₃, 6H), 2.43 (t, ³J_{HH} = 7.2 Hz, CH₂-NMe₂, 2H), 2.62 (d, ³J_{HP} = 9.9 Hz, N-CH₃, 3H), 3.09-3.15 (m, NMe-CH₂, 2H), 4.28-4.33 (m broad, CH-O, 2H); ³¹P{¹H} (CDCl₃, 202.4 MHz): 8.23, 8.48, 8.74; ¹³C (CDCl₃, 125.7 MHz): 13.51 (CH₃), 16.64 (CH₃), 25.02 (CH₃), 25.07 (CH₃), 25.29 (CH₃), 25.69, 25.81 (CH₂), 25.90, 27.07 (CH₃), 27.11 (CH₃), 28.42 (CH₂), 28.47 (CH₂), 30.61 (CH₂), 30.66 (CH₂), 31.65 (CH₂), 35.21-35.27 (m, CH₂), 31.45-35.52 (m, CH₂), 36.83 (d, ²J_{CP} = 2.9 Hz, N-CH₃), 41.57 (CH₃), 41.65 (CH₃), 46.88 (CH₂), 48.33 (N-Me₂),50.12 (m, NMe-CH₂), 60.29 (CH₂-NMe₂), 79.13 (m, CH-O); IR (ATR, neat): 2955.18, 2927.61, 2860.54, 1479.70, 1256.50 (v P=O), 961.24, 778.84; HRMS (ESI-qTOF), m/z calcd for C₃₃H₇₁N₂O₃P+H [M+H]⁺=575.5281; observed [M+H]⁺=575.5274;

### O,O-bis(2-decyltetradecyl)-N-(2-(dimethylamino)ethyl)phosphoramidate (compound 011)

Bis(2-decyltetradecyl)phosphite (0.5 g, 0.66 mmol, 1 eq.), *N*,*N*-diethylethylenediamine (0.10 g, 0.86 mmol, 1.3 eq.). Chromatography on silica gel CH₂Cl₂/MeOH from 100/0.5 to 100/7 to produce 0.51 g of colorless viscous oil (Yield: 89%). R_{f} (CH₂Cl₂/MeOH: 100/7 (v/v)): 0.25; ¹H NMR (CDCl₃, 500.0 MHz): δ = 0.87 (t, ³J_{HH}= 6.9 Hz, CH₃, 12H), 1.00 (t, ³J_{HH} = 6.7 Hz, CH₃, 6H), 1.24 (broad, 80H), 1.59 (broad, CH, 2H), 2.52 (broad, N-CH₂, 6H), 2.92 (broad, NH-CH₂, 2H), 3.34 (s broad, NH, 1H), 3.86 (m, CH₂-O, 4H); ³¹P{¹H} (CDCl₃, 202.4 MHz): δ = 10.17; ¹³C (CDCl₃, 125.7 MHz): δ = 14.50 (CH₃), 16.80 (CH₃), 25.39 (CH₂), 29.43 (CH₂), 32.07 (CH₂), 32.37 (CH₂), 32.42 (CH₂), 32.74 (CH₂), 33.58 (CH₂), 33.64 (CH₂), 34.63 (CH₂), 41.37 (d, ³J_{CP} = 7.4 Hz, CH), 41.49 (CH₂-NH), 49.43 (N-CH₂), 56.15 (d, ³J_{CP} = 6.7 Hz, CH₂-NEt₂), 71.33 (d, ²J_{CP} = 5.6 Hz, CH₂-O); IR (ATR, neat): 3213.42, 2921.16, 2852.37, 1465.27, 1234.14 (v P=O), 1003.72, 721.19;

### O,O-bis(2-decyltetradecyl)-N-methyl-N-(2-(dimethylamino)ethyl)phosphoramidate (compound 012)

Bis(2-decyltetradecyl)phosphite (0.5 g, 0.66 mmol, 1 eq.), *N*,*N*-diethyl-*N*'-methylethylenediamine (0.11 g, 0.86 mmol, 1.3 eq.). Chromatography on silica gel CH₂Cl₂/MeOH from 100/0.5 to 100/7 to produce 0.54 g of colorless viscous oil (Yield: 92%). R_{f} (CH₂Cl₂/MeOH: 100/7 (v/v)): 0.37; ¹H NMR (CDCl₃, 500.0 MHz): δ = 0.87 (t, ³J_{HH}= 6.8 Hz, CH₃, 12H), 1.03 (t, ³J_{HH}= 7.1 Hz, CH₃, 6H), 1.24 (broad, 80H), 1.58 (broad, CH, 2H), 2.56 (m, N-CH₂, 6H), 2.67 (d, ³J_{HP} = 9.7 Hz, N-CH₃, 3H), 3.09 (m, NMe-CH₂, 2H), 3.79 and 3.86 (2m, CH₂-O, 4H); ³¹P{¹H} (CDCl₃, 202.4 MHz): δ = 11.01; ¹³C (CDCl₃, 125.7 MHz): δ = 14.48 (CH₃), 16.82 (CH₃), 25.40 (CH₂), 29.43 (CH₂), 32.08 (CH₂), 32.39 (CH₂), 32.43 (CH₂), 32.77 (CH₂), 33.58 (CH₂), 33.66 (CH₂), 34.64 (CH₂), 36.92 (d, ²J_{CP} = 3.2 Hz, CH₃), 41.37 (d, ³J_{CP} = 7.6 Hz, CH), 49.97 (CH₂-N), 50.07 (CH₂), 53.99 (CH₂-NEt₂), 71.26 (d, ²J_{CP} = 5.7 Hz, CH₂-O); IR (ATR, neat): 2921.26, 2852.39, 1465.45, 1258.05 (v P=O), 994.63, 721.49;

### O,O-bis(2-decyltetradecyl)-N-ethyl-N-(2-(dimethylamino)ethyl)phosphoramidate (compound 013)

Bis(2-decyltetradecyl)phosphite (0.5 g, 0.66 mmol, 1 eq.), *N*,*N*-diethyl-*N*'-methylethylenediamine (0.12 g, 0.86 mmol, 1.3 eq.). Chromatography on silica gel CH₂Cl₂/MeOH from 100/0.5 to 100/7 to produce 0.48 g of colorless viscous oil (Yield: 81%). R_{f} (CH₂Cl₂/MeOH: 100/7 (v/v)): 0.33; ¹H NMR (CDCl₃, 500.0 MHz): δ = 0.87 (t, ³J_{HH}= 6.9 Hz, CH₃, 12H), 1.04 (t, ³J_{HH}= 6.8 Hz, CH₃, 6H), 1.10 (t, ³J_{HH}= 7.1 Hz, CH₃, 3H), 1.24 (broad, 80H), 1.58 (broad, CH, 2H), 2.57 (m, N-CH₃, 6H), 3.06 (m, N-CH₂, 4H), 3.77 and 3.85 (2m, CH₂-O, 4H); ³¹P{¹H} (CDCl₃, 202.4 MHz): δ = 10.90; ¹³C (CDCl₃, 125.7 MHz): δ = 16.82 (CH₃), 17.10 (CH₃), 25.40 (CH₂), 29.43 (CH₂), 29.46 (CH₂), 32.09 (CH₂), 32.39 (CH₂), 32.43 (CH₂), 32.77 (CH₂), 33.61 (CH₂), 33.70 (CH₂), 34.64 (CH₂), 41.37 (d, ³J_{CP} = 7.7 Hz, CH), 43.69 (N-CH₂), 46.03 (N-CH₂), 50.17 (N-CH₂), 54.74 (CH₂-N), 71.15 (d, ²J_{CP} = 5.8 Hz, CH₂-O); IR (ATR, neat): 2921.37, 2852.52, 1465.38, 1377.86, 1256.12 (v P=O), 1004.74, 720.73.

### O,O-bis(2-decyltetradecyl)-N-ethyl-N-(2-(dimethylamino)ethyl)phosphoramidate (compound 014)

Bis(2-decyltetradecyl)phosphite (0.6 g, 0.80 mmol, 1 eq.), ethylenediamine (0.48 g, 7.9 mmol, 10 eq.). Chromatography on silica gel CH₂Cl₂/MeOH from 100/0.5 to 100/7 to produce 0.42 g of colorless viscous oil (Yield: 65%). R_{f} (CH₂Cl₂/MeOH: 100/10 (v/v)): 0.30; ¹H NMR (CDCl₃, 500.0 MHz): δ = 0.86 (t, ³J_{HH}= 6.9 Hz, CH₃, 12H), 1.24 (broad, 80H), 1.59 (broad, CH, 2H), 2.21 (broad, NH₂, 2H), 2.79 (t, ³J_{HH} = 5.6 Hz, H₂N-CH₂, 2H), 2.94 (m, NH-CH₂, 2H), 3.18 (broad, NH, 1H), 3.86 (m, CH₂-O, 4H); ³¹P{¹H} (CDCl₃, 202.4 MHz): δ = 10.06; ¹³C (CDCl₃, 125.7 MHz): δ = 16.82 (CH₃), 25.40 (CH₂), 29.42 (CH₂), 32.09 (CH₂), 32.39 (CH₂), 32.43 (CH₂), 32.75 (CH₂), 33.53 (CH₂), 33.59 (CH₂), 34.64 (CH₂), 41.37 (d, ³J_{CP} = 7.4 Hz, CH), 45.60 (d, ²J_{CP} = 5.1 Hz, HN-CH₂), 46.44 (H₂N-CH₂), 71.59 (d, ²J_{CP} = 5.6 Hz, CH₂-O); IR (ATR, neat): 3300.00, 2920.79, 2851.60, 1466.95, 1229.45 (v P=O), 1004.36, 720.89;

### O,O-bis(2-decyltetradecyl)-N-(3-(dimethylamino)propyl)phosphoramidate (compound 015)

Bis(2-decyltetradecyl)phosphite (0.5 g, 0.66 mmol, 1 eq.), *N,N-*dimethylpropylenediamine (0.088 g, 0.86 mmol, 1.3 eq.). Chromatography on silica gel CH₂Cl₂/MeOH from 100/0.5 to 100/7 to produce 0.41 g of colorless viscous oil (Yield: 72%). R_{f} (CH₂Cl₂/MeOH: 100/7 (v/v)): 0.31; ¹H NMR (CDCl₃, 500.0 MHz): δ = 0.87 (t, ³J_{HH} = 6.9 Hz, CH₃, 12H), 1.25 (broad, 80H), 1.59 (broad, CH, 2H), 1.67 (q, ³J_{HH} = 6.3 Hz, , CH₂, 2H), 2.25 (s, NMe₂, 6H), 2.41 (s broad, NMe₂-CH₂, 2H), 2.97 (m, CH₂-NH, 2H), 3.48 (s broad, NH, 1H), 3.84 (m, CH₂-O, 4H); ³¹P{¹H} (CDCl₃, 202.4 MHz): δ = 9.80 ; ¹³C (CDCl₃, 125.7 MHz): δ = 16.80 (CH₃), 25.39 (CH₂), 29.42 (CH₂), 31.12 (d, ³J_{CP} = 5.2 Hz, CH₂), 32.08 (CH₂), 32.38 (CH₂), 32.42 (CH₂), 32.75 (CH₂), 33.57 (CH₂), 33.63 (CH₂), 34.63 (CH₂), 41.37 (d, ³J_{CP} = 7.4 Hz, CH), 43.37 (N-CH₂), 48.02 (NMe₂), 60.73, CH₂-NMe₂), 71.35 (d, ²J_{CP} = 5.7 Hz, CH₂-O); IR (ATR, neat): 2920.96, 2852.26, 1463.79, 1236.54 (v P=O), 1005.03, 721.06; HRMS (ESI-qTOF), m/z calcd for C₅₃H₁₁₁N₂O₃P+H [M+H]⁺= 855.8411; observed [M+H]⁺=855.8477;

### O,O-bis(2-decyltetradecyl)-N-(3-((3-aminopropyl)(methyl)amino)propyl)phosphoramidate (compound 016)

Bis(2-decyltetradecyl)phosphite (0.5 g, 0.66 mmol, 1 eq.), 3,3'-Diamino-*N-*methyldipropylamine (0.96 g, 6.61 mmol, 10 eq.). Chromatography on silica gel CH₂Cl₂/MeOH/NH₄OH (25% in water) from 100/10/0.5 to 100/10/1 to produce 0.47 g of colorless viscous oil (Yield: 79%). R_{f} (CH₂Cl₂/MeOH/NH₄OH (25% in water): 100/10/1 (v/v/v)): 0.21; ¹H NMR (CDCl₃, 500.0 MHz): δ = 0.86 (t, ³J_{HH}= 6.9 Hz, CH₃, 12H), 1.24 (broad, 80H), 1.62 (m, 6H), 1.99 (broad, NH₂, 2H), 2.18 (s, NMe, 3H), 2.39 (m, 4H), 2.75 (t, ³J_{HH} = 6.8 Hz, CH₂-NH₂, 2H), 2.94 (m, CH₂-NH, 2H), 3.61(broad, NH, 1H), 3.84 (m, CH₂-O, 4H); ³¹P{¹H} (CDCl₃, 202.4 MHz): δ = 9.84; ¹³C (CDCl₃, 125.7 MHz): δ = 16.82 (CH₃), 25.40 (CH₂), 29.43 (CH₂), 31.00 (d, ³J_{CP} = 5.6 Hz, CH₂), 32.09 (CH₂), 32.39 (CH₂), 32.44 (CH₂), 32.77 (CH₂), 33.11 (CH₂), 33.57 (CH₂), 34.63 (CH₂), 34.64 (CH₂), 41.37 (d, ³J_{CP} = 7.4 Hz, CH), 43.23 (CH₂), 43.48 (CH₂), 44.75 (N-CH₃), 58.64 (CH₂), 58.99 (CH₂), 71.35 (d, ²J_{CP} = 5.6 Hz, CH₂-O); IR (ATR, neat): 3210.12, 2920.95, 1464.28, 1235.02 (v P=O), 1003.80, 721.19.

### O,O-bis(2-decyltetradecyl)-N-(3-(dimethylamino)-2,2-dimethylpropyl)phosphoramidate (compound 017)

Bis(2-decyltetradecyl)phosphite (0.5 g, 0.66 mmol, 1 eq.), *N,N*-dimethyl-2,2-dimethylpropylenediamine (0.12 g, 0.86 mmol, 1.3 eq.). Chromatography on silica gel CH₂Cl₂/MeOH 100/4 to produce 0.56 g of colorless viscous oil (Yield: 96%). R_{f} (CH₂Cl₂/MeOH : 100/10 (v/v)): 0.49; ¹H NMR (CDCl₃, 500.0 MHz): δ = 0.85-0.90 (m, 18H), 1.24 (broad, 80H), 1.60 (broad, 2H), 2.18 (broad, 2H), 2.25 (s, NMe₂, 6H), 2.76 (broad, 2H), 3.85 (m, CH₂-O, 4H), 4.50 (s broad, NH, 1H); ³¹P{¹H} (CDCl₃, 202.4 MHz): δ = 9.92; ¹³C (CDCl₃, 125.7 MHz): δ = 16.82 (CH₃), 25.40 (CH₂), 27.46 (CH₃), 29.45 (CH₂), 32.09 (CH₂), 32.44 (CH₂), 32.78 (CH₂), 33.63 (CH₂), 33.69 (CH₂), 34.64 (CH₂), 37.87 (d, ³J_{CP} = 6.6 Hz, C_{quat.}), 41.40 (d, ³J_{CP} = 7.6 Hz, CH), 51.19 (NMe₂), 55.11 (N-CH₂), 71.20 (d, ²J_{CP} = 5.5 Hz, CH₂-O), 73.53 (CH₂-NMe₂); IR (ATR, neat): 3217.94, 2921.17, 2852.38, 1465.11, 1236.75 (v P=O), 1042.11, 1006.93, 721.01.

### O,O-bis(2-decyltetradecyl)-N-(2-(methylamino)ethyl)phosphoramidate (compound 018)

Bis(2-decyltetradecyl)phosphite (0.6 g, 0.79 mmol, 1 eq.), *N,N'-*dimethylethylenediamine (0.70 g, 7.94 mmol, 10 eq.). Chromatography on silica gel with CH₂Cl₂/MeOH 100/0.5 to 100/10 to produce 0.43 g of colorless viscous oil (Yield: 79%). R_{f} (CH₂Cl₂/MeOH : 100/10 (v/v)): 0.43; ¹H NMR (CDCl₃, 500.0 MHz): δ = 0.86 (m, 12H), 1.24 (broad, 80H), 1.59 (broad, 2H), 2.46 (s, NMe, 3H), 2.65 (d, ³J_{HP} = 9.7 Hz, N-CH₃, 3H), 2.71 (broad, NH, 1H), 2.76 (t, ³J_{HH} = 6.4 Hz, CH₂-N, 2H), 3.15 (m, CH₂-N, 2H), 3.79 and 3.86 (2m, CH₂-O, 4H); ³¹P{¹H} (CDCl₃, 202.4 MHz): δ = 11.23; ¹³C (CDCl₃, 125.7 MHz): δ = 16.82 (CH₃), 25.40 (CH₂), 29.41 (CH₂), 32.08 (CH₂), 32.38 (CH₂), 32.42 (CH₂), 32.75 (CH₂), 33.54 (CH₂), 33.63 (CH₂), 34.64 (CH₂), 36.82 (d, ²J_{CP} = 2.5 Hz, N-CH₃), 38.60 (N-CH₃), 41.36 (d, ³J_{CP} = 7.5 Hz, CH), 51.07 (d, ²J_{CP} = 2.9 Hz, CH₂-NMe), 52.31 (N-CH₂), 71.50 (d, ²J_{CP} = 5.9 Hz, CH₂-O); IR (ATR, neat): 2921.04, 2851.73, 1467.03, 1256.31 (v P=O), 996.01, 720.96;

### O,O-bis(2-decyltetradecyl)-N,N-bis(3-(dimethylamino)propyl)phosphoramidate (compound 019)

Bis(2-decyltetradecyl)phosphite (0.5 g, 0.66 mmol, 1 eq.), N,N-bis(3-dimethylaminopropyl)amine (0.16 g, 0.86 mmol, 1.3 eq.). Chromatography on silica gel CH₂Cl₂/MeOH/NH₄OH (25% in water) from 100/10/0.3 to 100/10/1.5 to produce 0.34 g of colorless viscous oil (Yield: 55%). R_{f} (CH₂Cl₂/MeOH/NH₄OH (25% in water): 100/10/2 (v/v/v)): 0.50; ¹H NMR (CDCl₃, 500.0 MHz): δ = 0.86 (t, ³J_{HH} = 6.8 Hz, CH₃, 12H), 1.24 (broad, 80H), 1.57 (broad, CH, 2H), 1.69 (q, ³J_{HH} = 7.2 Hz, 4H), 2.21 (s, NMe₂, 12H), 2.26 (m, CH₂-NMe₂, 4H), 3.00 (m, CH₂-N, 4H), 3.76 and 3.83 (2m, CH₂-O, 4H); ³¹P{¹H} (CDCl₃, 202.4 MHz): δ = 10.88; ¹³C (CDCl₃, 125.7 MHz): δ = 16.82 (CH₃), 25.40 (CH₂), 29.46 (CH₂), 32.09 (CH₂), 32.40 (CH₂), 32.44 (CH₂), 32.79 (CH₂), 33.59 (CH₂), 33.68 (CH₂), 34.64 (CH₂), 41.39 (d, ³J_{CP} = 7.5 Hz, CH), 46.61 (d, ²J_{CP} = 3.4 Hz, CH₂-NMe), 48.11 (NMe₂), 59.82 (Me₂N-CH₂), 71.22 (d, ²J_{CP} = 5.8 Hz, CH₂-O); IR (ATR, neat): 2921.71, 2852.72, 1460.61, 1256.21 (v P=O), 1001.11, 730.47.

### O,O-bis(2-decyltetradecyl)-N-(3-morpholinopropyl)phosphoramidate (compound 020)

Bis(2-decyltetradecyl)phosphite (0.5 g, 0.66 mmol, 1 eq.), 3-morpholinopropan-1-amine (0.12 g, 0.86 mmol, 1.3 eq.). Chromatography on silica gel with CH₂Cl₂/MeOH 100/3 to produce 0.39 g of colorless viscous oil (Yield: 66%). R_{f} (CH₂Cl₂/MeOH : 100/10 (v/v)): 0.45; ¹H NMR (CDCl₃, 500.0 MHz): δ = 0.90 (t, ³J_{HH} = 6.9 Hz, CH₃, 12H), 1.27 (broad, 80H), 1.62 (broad, CH, 2H), 1.69 (q, ³J_{HH} = 6.2 Hz, 2H), 2.45 (broad, CH₂-N, 6H), 3.00 (m, CH₂-NH, 2H), 3.47 (Broad, NH, 1H), 3.72 (t, CH₂-O, 4H), 3.87 (m, CH₂-O, 4H); ³¹P{¹H} (CDCl₃, 202.4 MHz): δ = 9.88; ¹³C (CDCl₃, 125.7 MHz): δ = 16.82 (CH₃), 25.40 (CH₂), 29.43 (CH₂), 30.06 (d, ³J_{CP} = 5.2 Hz, CH₂), 32.08 (CH₂), 32.39 (CH₂), 32.43 (CH₂), 32.76 (CH₂), 33.58 (CH₂), 33.64 (CH₂), 34.64 (CH₂), 41.38 (d, ³J_{CP} = 7.5 Hz, CH), 43.52 (CH₂-NH), 56.47 (CH₂-N), 60.06 (CH₂N), 69.66 (CH₂-O), 71.38 (d, ²J_{CP} = 5.7 Hz, CH₂-O); IR (ATR, neat): 3208.88, 2921.04, 2852.11, 1464.27, 1234.28 (v P=O), 1003.96, 721.35.

### O,O-bis(2-decyltetradecyl)-N-(pyridin-2-ylmethyl)phosphoramidate (compound 021)

Bis(2-decyltetradecyl)phosphite (0.5 g, 0.66 mmol, 1 eq.), pyridine-2-ylmethanamine (0.09 g, 0.86 mmol, 1.3 eq.). Chromatography on silica gel with CH₂Cl₂/MeOH 100/3 to produce 0.46 g of colorless viscous oil (Yield: 81%). R_{f} (CH₂Cl₂/MeOH : 100/4 (v/v)): 0.46; ¹H NMR (CDCl₃, 500.0 MHz): δ = 0.87 (t, ³J_{HH} = 6.8 Hz, CH₃, 12H), 1.24 (broad, 80H), 1.57 (broad, CH, 2H), 3.69 (m, NH, 1H), 3.85 and 3.89 (2m, CH₂-O, 4H), 4.22 (m, CH₂-NH, 2H), 7.17 (t, C_{Ar}-H, 1H), 7.32 (d, J = 7.8 Hz, C_{Ar}-H, 1H), 7.64 (t, J = 7.6 Hz, C_{Ar}-H, 1H), 8.52 (d, J = 4.0 Hz, C_{Ar}-H, 1H); ³¹P{¹H} (CDCl₃, 202.4 MHz): δ = 9.45; ¹³C (CDCl₃, 125.7 MHz): δ = 16.83 (CH₃), 25.41 (CH₂), 29.41 (CH₂), 32.09 (CH₂), 32.38 (CH₂), 32.43 (CH₂), 32.74 (CH₂), 33.50 (CH₂), 33.56 (CH₂), 34.65 (CH₂), 41.35 (d, ³J_{CP} = 7.5 Hz, CH), 48.99 (CH₂-NH), 71.60 (d, ²J_{CP} = 5.6 Hz, CH₂-O), 124.04 (C_{Ar}-H), 124.90 (C_{Ar}-H), 139.34 (C_{Ar}-H), 151.58 (C_{Ar}-H), 160.85 (d, ³J_{CH} = 6.7 Hz, C_{Ar}); IR (ATR,neat): 3201.63, 2920.90, 2852.12, 1465.04, 1233.86 (v P=O), 1004.97, 900.17, 879.38, 751.05, 721.25

### O,O-bis(2-decyltetradecyl)-N-pyridin-4-ylphosphoramidate (compound 022)

Bis(2-decyltetradecyl)phosphite (0.5 g, 0.66 mmol, 1 eq.), 4-aminopyridine (0.08 g, 0.86 mmol, 1.3 eq.). Chromatography on silica gel with CH₂Cl₂/MeOH 100/0.5 to 100/5 to produce 0.38 g of colorless viscous oil (Yield: 68%). R_{f} (CH₂Cl₂/MeOH : 100/7 (v/v)): 0.60; ¹H NMR (CDCl₃, 500.0 MHz): δ = 0.87 (t, ³J_{HH} = 6.8 Hz, CH₃, 12H), 1.24 (broad, 80H), 1.59 (m broad, CH, 2H), 3.87 and 4.02 (2m, CH₂-O, 4H), 6.88 (d, ³J_{HH} = 5.7 Hz, C_{Ar}-H, 2H), 7.33 (s broad, NH, 1H), 8.33 (d, ³J_{HH} = 5.7 Hz, C_{Ar}-H, 2H); ³¹P{¹H} (CDCl₃, 202.4 MHz): δ = 2.23; ¹³C (CDCl₃, 125.7 MHz): δ = 16.82 (CH₃), 25.41 (CH₂), 29.31 (CH₂), 29.37 (CH₂), 32.09 (CH₂), 32.38 (CH₂), 32.42 (CH₂), 32.66 (CH₂), 33.43 (CH₂), 33.46 (CH₂), 34.64 (CH₂), 41.26 (d, ³J_{CP} = 7.5 Hz, CH), 72.23 (d, ²J_{CP} = 5.0 Hz, CH₂-O), 114.86 (d, ³J_{CP} = 7.3 Hz, C_{Ar}-H), 150.28 (C_{Ar}-N), 152.86 (C_{Ar}-H); IR (ATR, neat): 3179.40, 3136.59, 3034.84, 2920.99, 2852.18, 1597.76, 1502.82, 1466.15, 1241.89 (v P=O), 992.71, 721.16;

### O,O-bis(2-decyltetradecyl)-N-(4-(2-hydroxyethyl)piperazin-1-yl)phosphoramidate (compound 023)

Bis(2-decyltetradecyl)phosphite (0.5 g, 0.66 mmol, 1 eq.), N-(2-hydroxyethyl)piperazine (0.11 g, 0.86 mmol, 1.3 eq.). Chromatography on silica gel with CH₂Cl₂/MeOH 100/0.5 to 100/7 to produce 0.50 g of colorless viscous oil (Yield: 86%). R_{f} (CH₂Cl₂/MeOH : 100/7 (v/v)): 0.34; ¹H NMR (CDCl₃, 500.0 MHz): δ = 0.87 (t, ³J_{HH} = 6.8 Hz, CH₃, 12H), 1.25 (broad, 80H), 1.59 (m broad, CH, 2H), 2.49 (s broad, CH₂-N, 4H), 2.56 (t, ³J_{HH} = 5.1 Hz, CH₂-N, 2H), 2.77 (broad, OH, 1H), 3.18 (s broad, CH₂-N, 4H), 3.63 (t, ³J_{HH} = 5.1 Hz, CH₂-O, 2H), 3.81 and 3.87 (2m, CH₂-O, 4H); ³¹P{¹H} (CDCl₃, 202.4 MHz): δ = 8.83; ¹³C (CDCl₃, 125.7 MHz): δ = 16.82 (CH₃), 25.40 (CH₂), 29.41 (CH₂), 32.08 (CH₂), 32.38 (CH₂), 32.42 (CH₂), 32.73 (CH₂), 33.58 (CH₂), 33.66 (CH₂), 34.64 (CH₂), 41.32 (d, ³J_{CP} = 7.5 Hz, CH), 47.24 (CH₂-N), 55.81 (d, ³J_{CP} = 5.1 Hz, CH₂-N), 60.23 (CH₂), 62.40 (CH₂), 71.47 (d, ²J_{CP} = 6.1 Hz, CH₂-O); IR (ATR, neat): 3415.53, 2920.29, 2851.84, 1464.33, 1258.06 & 1241.60 (v P=O), 1146.47, 979.70, 720.65;

### O,O-bis(2-decyltetradecyl)-N-(4-methylpiperazin-1-yl)phosphoramidate (compound 024)

Bis(2-decyltetradecyl)phosphite (0.5 g, 0.66 mmol, 1 eq.), N-methylpiperazine (0.086 g, 0.86 mmol, 1.3 eq.). Chromatography on silica gel with CH₂Cl₂/MeOH 100/0.5 to 100/7 to produce 0.49 g of colorless viscous oil (Yield: 87%). R_{f} (CH₂Cl₂/MeOH : 100/7 (v/v)): 0.30; ¹H NMR (CDCl₃, 500.0 MHz): δ = 0.87 (t, ³J_{HH} = 6.9 Hz, CH₃, 12H), 1.25 (broad, 80H), 2.01 (m broad, CH, 2H), 2.30 (s, CH₃-N, 3H), 2.38 (broad, CH₂-N, 4H), 3.18 (s broad, CH₂-N, 4H), 3.81 and 3.87 (2m, CH₂-O, 4H); ³¹P{¹H} (CDCl₃, 202.4 MHz): δ = 8.98; ¹³C (CDCl₃, 125.7 MHz): δ = 16.81 (CH₃), 25.40 (CH₂), 29.41 (CH₂), 32.08 (CH₂), 32.37 (CH₂), 32.42 (CH₂), 32.73 (CH₂), 33.59 (CH₂), 33.66 (CH₂), 34.64 (CH₂), 41.32 (d, ³J_{CP} = 7.5 Hz, CH), 47.13 (CH₂-N), 49.11 (CH₃-N), 57.83 (d, ³J_{CP} = 5.1 Hz, N-CH₂), 71.41 (d, ²J_{CP} = 6.0 Hz, CH₂-O); IR (ATR, neat): 2920.87, 2852.06, 1463.96, 1261.01 (v P=O), 976.91, 720.66;

### O,O-bis(2-decyltetradecyl)-N-(1-methylpiperidin-4-yl)phosphoramidate (compound 025)

Bis(2-decyltetradecyl)phosphite (0.5 g, 0.66 mmol, 1 eq.), 4-amino-1-methylpiperidine (0.098 g, 0.86 mmol, 1.3 eq.). Chromatography on silica gel with CH₂Cl₂/MeOH 100/0.5 to 100/7 to produce 0.43 g of colorless viscous oil (Yield: 75%). R_{f} (CH₂Cl₂/MeOH : 100/10 (v/v)): 0.33; ¹H NMR (CDCl₃, 500.0 MHz): δ = 0.87 (t, ³J_{HH} = 6.9 Hz, CH₃, 12H), 1.24 (broad, 80H), 1.50-1.59 (m, 4H), 1.91 (m, 2H), 2.07 (s broad, 2H), 2.28 (s, CH₃-N, 3H), 2.45 (m broad, 1H), 2.77 (s broad, 2H), 2.97 (s broad, NH, 1H), 3.82 and 3.88 (2m, CH₂-O, 4H); ³¹P{¹H} (CDCl₃, 202.4 MHz): δ = 8.64; ¹³C (CDCl₃, 125.7 MHz): δ = 16.80 (CH₃), 25.39 (CH₂), 29.42 (CH₂), 32.08 (CH₂), 32.38 (CH₂), 32.43 (CH₂), 32.75 (CH₂), 33.59 (CH₂), 33.67 (CH₂), 34.63 (CH₂), 37.31 (CH₂), 41.34 (d, ³J_{CP} = 7.5 Hz, CH), 48.69(CH₃-N), 50.93 (CH-N), 57.10 (CH₂-N), 71.44 (d, ²J_{CP} = 5.7 Hz, CH₂-O); IR (ATR, neat): 3196.93, 2920.82, 2852.06, 1464.97, 1230.09 (v P=O), 1003.04, 720.99;

### O,O-bis(2-octyldodecyl)-N-(3-(dimethylamino)propyl)phosphoramidate (compound 026)

*O,O*-bis(2-ocyldodecyl)phosphite (0.5 g, 0.78 mmol, 1 eq.), *N*,*N*-dimethylpropane-1,3-diamine (0.103 g, 1.01 mmol, 1.3 eq). The crude compound was purified by chromatography on silica gel CH₂Cl₂/MeOH from 100/0.5 to 100/7 to produce 388 mg of colorless viscous oil (Yield: 67%). R_{f} (CH₂Cl₂/MeOH 100/7 (v/v)): 0.45; ¹H NMR (CDCl₃, 400.0 MHz): δ = 0.89-0.93 (t, ³J_{HH}= 6.7 Hz, CH₃, 12H), 1.29 (broad, 64H), 1.62-1.65 (s broad, CH, 2H), 1.66-1.73 (quin, ³J_{HH} = 6.5 Hz, CH₂, 2H), 2.28 (s, CH₃-N, 6H), 2.42-2.45 (t, ³J_{HH} = 6.7 Hz, CH₂-NMe₂, 2H), 2.97-3.04 (m, CH₂-NH, 2H), 3.49-3.54 (s broad, NH, 1H), 3.86-3.94 (m, CH₂-O, 4H); ³¹P{¹H} (CDCl₃, 202.4 MHz): δ = 9.81; ¹³C (CDCl₃, 125.7 MHz): δ = 15.05 (CH₃), 23.63 (CH₂), 27.66 (CH₂), 29.38 (d, ³J_{CP} = 5.5 Hz, CH₂), 30.32 (CH₂), 30.57 (CH₂), 30.62 (CH₂), 30.66 (CH₂), 30.99 (CH₂), 31.85 (d, ³J_{CP} = 7.0 Hz, CH₂), 32.87 (CH₂), 39.61 (d, ³J_{CP} = 7.5 Hz, CH), 41.60 (CH₂-NMe₂), 46.27 (CH₃-N), 58.97 (CH₂-NH), 69.59 (d, ²J_{CP} = 5.7 Hz, CH₂-O); IR (ATR, neat): 3207.92 (v N-H), 2921.65, 2852.98, 1460.75, 1236.39 (v P=O), 1002.68, 721.52; HRMS m/z calcd for [C₄₅H₉₆N₂O₃P]⁺: 743.7153, found: 743.7152, [M + H], 463.4020 = fragmentation (-C24).

### O,O-bis(2-hexyldecyl)-N-(3-(dimethylamino)propyl)phosphoramidate (compound 027)

*O,O*-bis(2-hexyldecyl)phosphite (0.5 g, 0.94 mmol, 1 eq.), *N*,*N*-dimethylpropane-1,3-diamine (0.125 g, 1.22 mmol, 1.3 eq.). The crude compound was purified by chromatography on silica gel CH₂Cl₂/MeOH from 100/0.5 to 100/7 to produce 406 mg of colorless viscous oil (Yield: 68%). R_{f} (CH₂Cl₂/MeOH 100/7 (v/v)): 0.30; ¹H NMR (CDCl₃, 400.0 MHz): δ = 0.89-0.92 (t, ³J_{HH}= 6.7 Hz, CH₃, 12H), 1.29 (broad, 48H), 1.61-1.65 (broad, CH, 2H), 1.66-1.73 (quin, ³J_{HH} = 6.5 Hz, CH₂, 2H), 2.28 (s, CH₃-N, 6H), 2.42-2.46 (t, ³J_{HH} = 6.7 Hz, CH₂-NMe₂, 2H), 2.97-3.04 (m, CH₂-NH, 2H), 3.48-3.54 (s broad, NH, 1H), 3.87-3.93 (m, CH₂-O, 4H); ³¹P{¹H} (CDCl₃, 202.4 MHz): δ = 9.81; ¹³C (CDCl₃, 125.7 MHz): δ = 15.04 (CH₃), 23.62 (CH₂), 27.61 (CH₂), 27.66 (CH₂), 29.34 (CH₂), 29.38 (CH₂), 30.30 (CH₂), 30.56 (CH₂), 30.63 (CH₂), 30.99 (CH₂), 31.81 (CH₂), 31.87 (CH₂), 32.80 (CH₂), 32.86 (CH₂), 39.62 (d, ³J_{CP} = 7.5 Hz, CH), 41.56 (CH₂-NMe₂), 46.24 (CH₃-N), 58.93 (CH₂-NH), 69.62 (d, ²J_{CP} = 5.6 Hz, CH₂-O); IR (ATR, neat): 3211.96 (v N-H), 2922.73, 2854.15, 1459.61, 1235.89 (v P=O), 1005.23, 722.45; HRMS m/z calcd for [C₃₇H₈₀N₂O₃P]⁺: 631.5901, found: 631.5901, [M + H].

### O,O-bis(2-decyltetradecyl)-N-(3-(1H-imidazol-1-yl)propyl)phosphoramidate (compound 028)

*O*,*O*-bis(2-decyltetradecyl)phosphite (0.5 g, 0.64 mmol, 1 eq.), *N*-(3-Aminopropyl)imidazole (0.104 g, 0.83 mmol, 1.3 eq.). The crude compound was purified by chromatography on silica gel CH₂Cl₂/MeOH from 100/0.5 to 100/2 to produce 322 mg of colorless viscous oil (Yield: 58%). R_{f} (CH₂Cl₂/MeOH: 100/7 (v/v)): 0.38; ¹H NMR (CDCl₃, 400.0 MHz): δ = 0.89-0.92 (t, ³J_{HH}= 6.8 Hz, CH₃, 12H), 1.28 (broad, 80H), 1.63 (broad, CH, 2H), 1.94-2.03 (quin, ³J_{HH} = 6.8 Hz, CH₂, 2H), 2.74 (s broad, NH, 1H), 2.91-2.99 (m, CH₂-NH, 2H), 3.90 (m, CH₂-O, 4H), 4.04-4.08 (t, ³J_{HH} = 7.0 Hz, CH₂-N_{Ar}, 2H), 6.95 (s, CH_{Ar}, 1H), 7.09 (s, CH_{Ar}, 1H), 7.51 (s, CH_{Ar}, 1H); ³¹P{¹H} (CDCl₃, 202.4 MHz): δ = 9.60; ¹³C (CDCl₃, 125.7 MHz): δ = 15.04 (CH₃), 23.67 (CH₂), 26.69 (CH₂), 29.36 (CH₂), 29.66 (CH₂), 29.70 (CH₂), 30.02 (CH₂), 30.85 (d, ³J_{CP} = 8.1 Hz, CH₂), 31.91 (CH₂), 33.06 (CH₂), 33.10 (CH₂), 38.21 (CH₂-NH), 38.66 (d, ³J_{CP} = 7.3 Hz, CH), 43.96 (CH₂ α-N_{Ar}), 68.88 (d, ²J_{CP} = 6.1 Hz, CH₂-O), 118.75 (CH_{Ar}), 129.57 (CH_{Ar}), 137.10 (CH_{Ar}); IR (ATR, neat): 3215.06 (v N-H), 2921.00, 2852.22, 1464.65, 1231.15 (v P=O), 1004.19, 721.92; HRMS m/z calcd for [C₅₄H₁₀₉N₃O₃P]⁺: 878.8201, found: 878.8195, [M + H], 542.4445 = fragmentation (-C24).

### O,O-bis(2-decyltetradecyl)-N-(2-(1H-imidazol-5-yl)ethyl)phosphoramidate (compound 029)

*O*,*O*-bis(2-decyltetradecyl)phosphite (0.5 g, 0.64 mmol, 1 eq.), histamine dihydrochloride (0.153 g, 0.83 mmol, 1.3 eq.). The crude compound was purified by chromatography on silica gel CH₂Cl₂/MeOH from 100/0.5 to 100/7 to produce 185 mg of colorless viscous oil (Yield: 34%). R_{f} (CH₂Cl₂/MeOH: 100/7 (v/v)): 0.40; ¹H NMR (CDCl₃, 400.0 MHz): δ = 0.96-0.99 (t, ³J_{HH}= 6.8 Hz, CH₃, 12H), 1.35 (broad, 80H), 1.70 (broad, CH, 2H), 2.87-2.89 (t, ³J_{HH} = 6.2 Hz, CH₂-C_{Ar}, 2H), 3.23-3.29 (m, CH₂-NH, 2H), 3.43 (s broad, NH, 1H), 3.88-4.00 (m, CH₂-O, 4H), 6.87 (s, CH_{Ar}, 1H), 7.62 (s, CH_{Ar}, 1H); ³¹P{¹H} (CDCl₃, 202.4 MHz): δ = 9.84; ¹³C (CDCl₃, 125.7 MHz): δ = 14.59 (CH₃), 22.69 (CH₂), 26.71 (CH₂), 29.38 (CH₂), 29.68 (CH₂), 29.73 (CH₂), 30.04 (CH₂), 30.82 (d, ³J_{CP} = 6.1 Hz, CH₂), 31.94 (CH₂), 38.64 (d, ³J_{CP} = 7.3 Hz, CH), 41.20 (CH₂-NH), 68.96 (d, ²J_{CP} = 6.2 Hz, CH₂-O), 134.99 (CH_{Ar}); IR (ATR, neat): 3155.32 (v N-H), 2920.82, 2852.07, 1465.21, 1223.68 (v P=O), 1005.57, 720.88; HRMS m/z calcd for [C₅₃H₁₀₇N₃O₃P]⁺: 864.8045, found: 864.8043, [M + H], 528.4291 = fragmentation (-C24).

### O,O-bis(3,7,11,15-tetramethylhexadecyl)-N-(2-(dimethylamino)ethyl)phosphoramidate (compound 030)

*O*,*O*-bis(3,7,11,15tetramethylhexadecyl) phosphonate (0.500 g, 7.78.10⁻¹ mmol, 1 eq.), *N*,*N*-dimethylethane-1,2-diamine (0.089 g, 1,01 mmol, 1.3 eq.). The crude compound was purified by chromatography on silica gel CH₂Cl₂/MeOH from 100/0.5 to 100/5 to produce 415 mg of colorless oil (Yield: 73%). R_{f} (CH₂Cl₂/MeOH 100/7 (v/v)): 0.33; ¹H NMR (CDCl₃, 500.0 MHz): δ = 0.82-0.89 (m, CH₃, 30H), 1.04-1.35 (broad, CH₂, 40H), 1.46-1.70 (m, CH, 8H), 2.20 (s, CH₃-N, 6H), 2.35-2.37 (m, CH₂-NMe₂, 2H), 2.93-2.96 (m, CH₂-NH, 2H), 3.21-3.23 (m, NH, 1H), 3.98-4.03 (m, CH₂-O, 4H); ³¹P{¹H} (CDCl₃, 202.4 MHz): δ = 10.07; ¹³C (CDCl₃, 125.7 MHz): δ = 22.15 (CH₃), 22.38 (CH₃), 22.45 (CH₃), 25.33 (CH₃), 25.42 (CH₃), 27.04 (CH₂), 27.19 (CH₂), 27.50 (CH₂), 30.67 (CH), 32.18 (CH), 35.49 (CH), 40.00 (CH₂), 40.11 (CH₂), 40.16 (CH₂), 41.36 (CH₂), 42.07 (CH₂-NMe₂), 47.80 (CH₃-N), 62.30 (d, ³J_{CP} = 7.1 Hz, CH₂-NH), 67.39 (d, ³J_{CP} = 5.1 Hz, CH₂-O); IR (ATR, neat): 3217.21 (NH), 2924.12, 2867.46, 1461.16, 1377.59, 1233.56 (v P=O), 981.36, 511.23; HRMS m/z calcd for [C₄₄H₉₄N₂O₃P]⁺: 729.6997, found: 729.6993.

### O,O-bis(3,7,11,15-tetramethylhexadecyl)-N-(3-(dimethylamino)propyl)phosphoramidate (compound 031)

*O,O*-bis(3,7,11,15tetramethylhexadecyl) phosphonate (0.500 g, 7.78.10⁻¹ mmol, 1 eq.), *N*,*N*-dimethylpropane-1,3-diamine (0.103 g, 1,01 mmol, 1.3 eq.). The crude compound was purified by chromatography on silica gel CH₂Cl₂/MeOH from 100/0.5 to 100/10 to produce 346 mg of colorless oil (Yield: 60%). R_{f} (CH₂Cl₂/MeOH) : 100/7 (v/v)): 0.31 ; ¹H NMR (CDCl₃, 500.0 MHz): δ = 0.83-0.89 (m, CH₃, 30H), 1.03-1.35 (broad, CH₂, 40H), 1.50-1.53 (m, CH, 6H), 1.67-1.69 (m, CH + CH₂, 4H), 2.28 (s, CH₃-N, 6H), 2.43-2.46 (m, CH₂-NMe₂, 2H), 2.98-3.01 (m, CH₂-NH, 2H), 3.47-3.53 (m, NH, 1H), 3.98-4.02 (m, CH₂-O, 4H); ³¹P{¹H} (CDCl₃, 202.4 MHz): δ = 9.80; ¹³C (CDCl₃, 125.7 MHz): δ = 19.42 (CH₃), 19.59 (CH₃), 19.73 (CH₃), 22.61 (CH₃), 22.70 (CH₃), 24.33 (CH₂), 24.46 (CH₂), 24.78 (CH₂), 27.94 (CH), 28.25 (d, ³J_{CP} = 5.5 Hz, CH₂), 29.48 (CH), 32.77 (CH), 37.28 (CH₂), 37.40 (CH₂), 39.35 (CH₂), 40.40 (CH₂-NMe₂), 45.13 (CH₃-N), 57.76 (CH₂-NH), 64.66 (d, ³J_{CP} = 5.5 Hz, CH₂-O); IR (ATR, neat): 3210.73 (NH), 2924.12, 2867.39, 1461.03, 1377.56, 1233.78 (v P=O), 981.86, 528.24; HRMS m/z calcd for [C₄₅H₉₆N₂O₃P]⁺: 743.7153, found: 743.7150.

### O,O-di(heptadecan-9-yl) (2-(pyrrolidin-1-yl)ethyl)phosphoramidate (compound 032)

*N*-(2-aminoethyl)pyrrolidine (0.13 g, 1.16 mmol, 1.3 eq.), *O,O*-bis(heptadecan-9-yl) phosphite (0.5 g, 0.89 mmol, 1 eq.), DIPEA (0.15 g, 1.16 mmol, 1.3 eq.) and bromotrichloromethane (0.23 g, 1.16 mmol, 1.3 eq.) were used. The crude compound was purified by chromatography on silica gel CH₂Cl₂/MeOH from 100/5 to 100/7 to produce 0.29 g of colorless oil (Yield: 48%). R_{f} (CH₂Cl₂/MeOH 100/10 (v/v): 0.34; ¹H NMR (CDCl₃, 400.0 MHz): δ = 0.90 (t, ³J_{HH} = 6.7 Hz, CH₃ fatty chain, 12H), 1.28-1.40 (broad, 48H), 1.58-1.64 (m, 8H), 1.78-1.83 (broad, CH₂-CH₂, 4H), 2.57-2.64 (m broad, CH₂-N, 6H), 3.04 (m, CH₂-NH, 2H), 3.24 (broad, NH, 1H), 4.32 (m, CH-O, 2H); ³¹P{¹H} (CDCl₃, 161.92 MHz): δ = 7.88; ¹³C (CDCl₃, 100.6 MHz): δ = 13.96 (CH₃), 22.53 (CH₂), 23.35 (CH₂), 24.82 (CH₂), 29.16 (CH₂), 29.19 (CH₂), 29.47 (d, J_{CP} = 3.8 Hz, CH₂), 29.59 (CH₂), 31.76 (CH₂), 34.82 (broad, CH₂), 39.71 (CH₂), 53.67 (cyclic CH₂), 56.23 (d, ³J_{CP} = 7.2 Hz, CH₂-N, CH₂), 77.74 (d, ²J_{CP} = 6.0 Hz, CH-O).

### O,O-di(henicosan-11-yl) (2-(dimethylamino)ethyl)phosphoramidate (compound 033)

*N*,*N*-dimethylethylenediamine (0.085 g, 0.97 mmol, 1.3 eq.), *O,O*-di(henicosan-11-yl)-phosphite (0.5 g, 0.74 mmol, 1 eq.), DIPEA (0.12 g, 0.97 mmol, 1.3 eq.) and bromotrichloromethane (0.19 g, 0.97 mmol, 1.3 eq.) were used. The crude compound was purified by chromatography on silica gel CH₂Cl₂/MeOH from 100/1 to 100/7 to produce 0.51g of colorless oil (Yield: 90%). R_{f} (CH₂Cl₂/MeOH 100/10 (v/v) : 0.46; ¹H NMR (CDCl₃, 400.0 MHz): δ = 0.90 (t, ³J_{HH} = 6.8 Hz, CH₃ fatty chain, 12H), 1.28-1.34 (broad, 64H), 1.58-1.67 (m, 8H), 2.23 (s, N-CH₃, 6H), 2.39 (t, ³J_{HH}= 6.0 Hz, CH₂-N, 2H), 2.97 (dq, ³J_{HH} ≈ ³J_{HH} = 6.0 Hz, ³J_{HP} = 13.7 Hz, CH₂-NH, 2H), 3.15 (dt, ³J_{HH} = 6.0 Hz, ²J_{HP} = 10.8 Hz, NH, 1H), 4.33 (m, CH-O, 2H); ³¹P{¹H} (CDCl₃, 161.92 MHz): δ = 7.57; ¹³C (CDCl₃, 100.6 MHz): δ = 13.97 (CH₃), 22.55 (CH₂), 24.82 (CH₂), 29.21 (CH₂), 29.24 (CH₂), 29.50 (CH₂), 29.54 (CH₂), 29.59 (CH₂), 31.79 (CH₂), 34.83 (CH₂), 34.83 (d, ²J_{CP} = 7.7 Hz, CH₂-NH), 38.68 (CH₂), 44.98 (N-CH₃), 55.48 (d, ³J_{CP} = 7.7 Hz, CH₂-N, CH₂), 77.70 (d, ²J_{CP} = 6.2 Hz, CH-O).

### O,O-bis(2-octyldodecyl)-N-(4-(dimethylamino)butyl)phosphoramidate (compound 034)

*O,O*-bis(2-octyldodecyl)phosphite (1 g, 1.55 mmol, 1 eq.), *N*,*N*-dimethylbutane-1,4-diamine (0.235 g, 2.02 mmol, 1.3 eq.), N,N-diisopropylethylamine (0.352 µL, 2.02 mmol, 1.3 eq) and bromotrichloromethane (199 µL, 2.02 mmol, 1.3 eq) were used. The crude compound was purified by chromatography on silica gel CH₂Cl₂/MeOH from 100/1 to 100/7 to produce 261 mg of colorless oil (Yield: 22%). Rf (CH₂Cl₂/MeOH) : 100/7 (v/v)): 0.30; ¹H NMR (CDCl₃, 400.0 MHz): δ = 0.88-0.92 (t, ³J_{HH} = 6.4 Hz, CH₃, 12H), 1.28 (broad, CH₂, 64H), 1.57-1.62 (m, CH, CH₂ polar head, 6H), 2.33 (s, CH₃-N, 6H), 2.40-2.43 (m, CH₂-NMe₂, 2H), 2.91-2.94 (m, CH₂-NH, 2H), 3.83-3.92 (m, CH₂-O, 4H); ³¹P{¹H} (CDCl₃, 202.4 MHz): δ = 9.78; IR (ATR, neat): 3204(NH), 2920, 2847, 1462, 1232 (v P=O), 1001, 882, 720.

### O,O-bis(2-octyldodecyl)-N-(2-(pyrrolidin-1-yl)ethyl)phosphoramidate (compound 035)

*O,O*-bis(2-octyldodecyl)phosphite (1 g, 1.55 mmol, 1 eq.), N-(2-Aminoethyl)pyrrolidine (0.178 g, 2.02 mmol, 1.3 eq.), N,N-diisopropylethylamine (0.352 µL, 2.02 mmol, 1.3 eq) bromotrichloromethane (199 µL, 2.02 mmol, 1.3 eq) were used. The crude compound was purified by flash chromatography CH₂Cl₂/MeOH from 100/0 to 95/5 to produce 311 mg of colorless oil (Yield: 26%). Rf (CH₂Cl₂/MeOH) : 100/7 (v/v)): 0.36; ¹H NMR (CDCl₃, 400.0 MHz): δ = 0.89-0.92 (t, ³J_{HH}= 6.8 Hz, CH₃, 12H), 1.28 (broad, CH₂, 64H), 1.62-1.63 (m, CH, 2H), 1.82 (m, CH₂ pyrrolidine, 4H), 2.60-2.66 (m, CH₂-N, 6H), 3.05 (m, CH₂-NH, 2H), 3.36 (s broad, NH, 1H), 3.85-3.94 (m, CH₂-O, 4H); ³¹P{¹H} (CDCl₃, 202.4 MHz): δ = 9.90; ¹³C (CDCl₃, 101 MHz): δ = 14.10 (CH₃), 22.69 (CH₂), 23.46 (CH₂), 26.72 (CH₂), 29.37 (CH₂), 29.62 (CH₂), 29.67 (CH₂), 29.71 (CH₂), 30.85 (CH₂), 30.91 (CH₂), 31.92 (CH₂), 38.66 (d, ³J_{CP} = 7.5 Hz, CH), 39.64 (CH₂-NH), 53.87 (CH₂-pyrrolidine), 56.51 (d, ³J_{CP} = 6.3 Hz, CH₂-N), 68.77 (d, ²J_{CP} = 6.0 Hz, CH₂-O); IR (ATR, neat): 3214 (NH), 2920, 2847, 1462, 1233 (v P=O), 999, 876, 720.

### O,O-bis(2-octyldodecyl)-N-(3-(pyrrolidin-1-yl)propyl)phosphoramidate (compound 036)

*O,O*-bis(2-octyldodecyl)phosphite (1 g, 1.55 mmol, 1 eq.), N-(3-Aminopropyl)pyrrolidine (0.199 g, 2.02 mmol, 1.3 eq.), *N*,*N*-diisopropylethylamine (0.352 µL, 2.02 mmol, 1.3 eq) and bromotrichloromethane (199 µL, 2.02 mmol, 1.3 eq) were used. The crude compound was purified by flash chromatography CH₂Cl₂/MeOH from 100/0 to 95/5 to produce 250 mg of colorless oil (Yield: 21%). Rf (CH₂Cl₂/MeOH) : 100/7 (v/v)): 0.30; ¹H NMR (CDCl₃, 400.0 MHz): δ = 0.88-0.92 (t, ³J_{HH}= 6.8 Hz, CH₃, 12H), 1.28 (broad, CH₂, 64H), 1.61-1.62 (m, CH, 2H), 1.80-1.83 (m, CH₂ polar head, 2H), 1.88-1.91 (m, CH₂ pyrrolidine, 4H), 2.75 (m, CH₂-N, 6H), 3.04-3.05 (m, CH₂-NH, 2H), 3.45 (s broad, NH, 1H), 3.83-3.93 (m, CH₂-O, 4H); ³¹P{¹H} (CDCl₃, 202.4 MHz): δ = 9.74; ¹³C (CDCl₃, 101 MHz): δ = 14.10 (CH₃), 22.68 (CH₂), 23.39 (CH₂), 26.72 (CH₂), 29.37 (CH₂), 29.62 (CH₂), 29.67 (CH₂), 29.72 (CH₂), 30.05 (CH₂), 30.83 (CH₂), 30.89 (CH₂), 31.92 (CH₂), 38.67 (d, ³J_{CP} = 7.5 Hz, CH), 40.10 (CH₂-NH), 54.07 (CH₂-pyrrolidine), 54.17 (CH₂-N), 68.79 (d, ²J_{CP} = 6.1 Hz, CH₂-O); IR (ATR, neat): 3210 (NH), 2920, 2847, 1462, 1232 (v P=O), 1001, 874, 720.

### O,O-di(heptadecan-9-yl)-N-(2-(dimethylamino)ethyl)phosphoramidate (compound 037)

*O*,*O*-di(heptadecan-9-yl) phosphonate (0.564 g, 1.01 mmol, 1 eq.), *N*,*N*-dimethylethane-1,2-diamine (143 µL, 1.31 mmol, 1.3 eq.), *N*,*N*-diisopropylethylamine (229 µL, 1.31 mmol, 1.3 eq) and bromotrichloromethane (129 µL, 1.31 mmol, 1.3 eq) were used. The crude compound was purified by flash chromatography CH₂Cl₂/MeOH from 99/1 to 97/3 to produce 0.133 g of colorless oil (Yield: 20%). Rf (CH₂Cl₂/MeOH) : 100/7 (v/v)): 0.42; ¹H NMR (CDCl₃, 400.0 MHz): δ = 0.88-0.91 (t, ³J_{HH} = 6.7 Hz, CH₃, 12H), 1.28 (broad, CH₂, 48H), 1.60-1.63 (m, CH₂ β-O, 8H), 2.25 (s, CH₃-N, 6H), 2.40-2.43 (t, ³J_{HH}= 6.0 Hz, CH₂-NMe₂, 2H), 2.96-3.02 (m, CH₂-NH, 2H), 3.15-3.21 (m, NH, 1H), 4.28-4.36 (m, CH₂ α-O, 4H); ³¹P{¹H} (CDCl₃, 202.4 MHz): δ = 7.96; ¹³C (CDCl₃, 125.7 MHz): δ = 16.81 (CH₃), 25.38 (CH₂), 25.40 (CH₂), 27.67 (CH₂), 32.01 (CH₂), 32.05 (CH₂), 32.29 (CH₂), 32.34 (CH₂), 32.44 (CH₂), 34.60 (CH₂), 34.62 (CH₂), 37.68 (CH₂), 41.47 (CH₂-NH), 47.78 (CH₃-N), 62.35 (d, ³J_{CP} = 7.6 Hz, CH₂-N), 80.60 (d, ²J_{CP} = 6.1 Hz, CH-O); IR (ATR, neat): 3196 (NH), 2916, 2848, 1455, 1230 (v P=O), 975, 720.

### O,O-tetrakis(2-decyltetradecyl)-N-((methylazanediyl)bis(propane-3,1-diyl))bis(phosphoramidate) (compound 38)

*O,O*-bis(2-decyltetradecyl)phosphite (1.23 g, 1.57 mmol, 2.3 eq.), 3,3'-Diamino-*N-*methyldipropylamine (0.113 g, 0.68 mmol, 1 eq.). The crude compound was purified by chromatography on silica gel CH₂Cl₂/MeOH/NH₄OH from 100/10/0.2 to 100/10/0.5 to produce 850 mg of colorless oil (Yield: 75%). R_{f} (CH₂Cl₂/MeOH/NH₄OH (25% in water) 100/10/1 (v/v/v)): 0.44; ¹H NMR (CDCl₃, 500.0 MHz): δ = 0.85-0.88 (t, ³J_{HH} = 6.9 Hz, CH₃, 24H), 1.24 (broad, 168H), 1.58-1.64 (m, CH + CH₂, 8H), 2.17 (s, CH₃-N, 3H), 2.36-2.37 (m, CH₂-NMe, 4H), 2.91-2.94 (m, CH₂-NH, 4H), 3.25-3.29 (m, NH, 2H), 3.79-3.89 (m, CH₂-O, 8H); ³¹P{¹H} (CDCl₃, 202.4 MHz): δ = 9.78; ¹³C (CDCl₃, 125.7 MHz): δ = 16.82 (CH₃), 25.41 (CH₂), 29.44 (CH₂), 31.35 (d, ³J_{CP} = 4.4 Hz, CH₂), 32.10 (CH₂), 32.41 (CH₂), 32.45 (CH₂), 32.79 (CH₂), 33.59 (d, ³J_{CP} = 6.4 Hz, CH₂), 34.65 (CH₂), 41.37 (d, ³J_{CP} = 7.5 Hz, CH), 43.20 (CH₂-NMe), 44.69 (CH₃-N), 58.86 (CH₂-NH), 71.41 (d, ²J_{CP} = 5.4 Hz, CH₂-O); IR (ATR, neat): 3209.67, 2920.66, 2852.03, 1464.69, 1235.16 (v P=O), 1004.45, 721.02; HRMS m/z calcd for [C₁₀₃H₂₁₄N₃O₆P₂]⁺: 1651.6002, found: 1651.6020; calcd for [C₁₀₃H₂₁₅N₃O₆P₂]²⁺: 826.3037, found: 826.3034).

### O,O-tetrakis(2-hexyldecyl)-N-((methylazanediyl)bis(propane-3,1-diyl))bis(phosphoramidate) (compound 039)

*O,O*-bis(2-decyltetradecyl)phosphite (0.620 g, 1.17 mmol, 2.3 eq.), 3,3'-Diamino-N-methyldipropylamine (0.84 g, 5.08.10⁻¹ mmol, 1 eq.). The crude compound was purified by chromatography on silica gel CH₂Cl₂/MeOH/NH₄OH from 100/10/0.2 to 100/10/0.4 to produce 503 mg of colorless oil (Yield: 82%). R_{f} (CH₂Cl₂/MeOH/NH₄OH (25% in water) 100/10/1 (v/v/v)): 0.47; ¹H NMR (CDCl₃, 500.0 MHz): δ = 0.87-0.90 (m, CH₃, 24H), 1.27 (broad, 96H), 1.62-1.67 (m, 8H, CH + CH₂), 2.20 (s, CH₃-N, 3H), 2.38-2.42 (t, ³J_{HH} = 6.9 Hz, CH₂-NMe₂, 4H), 2.93-2.98 (m, CH₂-NH, 4H), 3.30 (s broad, NH, 2H), 3.83-3.91 (m, CH₂-O, 8H); ³¹P{¹H} (CDCl₃, 202.4 MHz): δ = 9.76; ¹³C (CDCl₃, 125.7 MHz): δ = 16.26 (CH₃), 24.85 (CH₂), 28.84 (CH₂), 28.89 (CH₂), 31.82 (d, ³J_{CP} = 4.9 Hz, CH₂), 31.54 (CH₂), 31.79 (CH₂), 31.87 (CH₂), 32.22 (CH₂), 33.05 (CH₂), 33.10 (CH₂), 34.03 (CH₂), 34.10 (CH₂), 40.85 (d, ³J_{CP} = 7.4 Hz, CH), 42.65 (CH₂-NMe₂), 44.13 (CH₃-N), 58.30 (CH₂-NH), 70.81 (d, ²J_{CP} = 6.3 Hz, CH₂-O); IR (ATR, neat): 3209.67, 2920.66, 2852.03, 1464.69, 1235.16 (v P=O), 1004.45, 721.02; HRMS m/z calcd for [C₇₁H₁₅₀N₃O₆P₂]⁺: 1203.0994, found: 1203.1001; calcd for [C₇₁H₁₅₁N₃O₆P₂]²⁺: 602.0533, found: 602.0529; 489.9278 (2+) = 978.8479 : fragmentation (-C16) -> 377.8025 (2+) = 754.5980 (-C16).

### O,O-bis(2-decyltetradecyl)-N-(3-(1-(dimethylamino)ethyl)phenyl) phosphate (compound 040)

*O,O*-bis(2-decyltetradecyl)phosphite (0.500 g, 6.38.10⁻¹ mmol, 1 eq.), 3-(1-(dimethylamino)ethyl) phenol (0.137 g, 8.298.10⁻¹ mmol, 1.3 eq.), *N,N-*diisopropylethylamine (0.107 g, 8.298.10⁻¹ mmol, 1.3 eq) were added at a 25 mL round bottom flask and bromotrichloromethane (0.165 g, 8.298.10⁻¹ mmol, 1.3 eq) was added drop by drop at 0°C. The reaction mixture was stirred during 10 minutes at 0°C and 24h at room temperature. The solvents were removed under vacuum and the residue was diluted in diethyl ether. The solution was filtered over a pad of celite and concentrated under vacuum. The organic layer was diluted in CH₂Cl₂, washed with 3x50 mL of water, dried over MgSO₄, filtered and concentrated under vacuum. The crude compound was purified by chromatography on silica gel CH₂Cl₂/MeOH from 100/0.5 to 100/2 to produce 284 mg of colorless oil (Yield: 48%). R_{f} (CH₂Cl₂/MeOH 100/7 (v/v)): 0.43; ¹H NMR (CDCl₃, 500.0 MHz): δ = 0.89-0.93 (t, ³J_{HH}= 6.8 Hz, CH₃, 12H), 1.28 (broad, 80H), 1.36-1.38 (d, ³J_{HH}= 6.7 Hz, CH₃, 3H), 1.64 (s broad, CH, 1H), 1.75 (s broad, CH, 1H), 2.22 (s, CH₃-N, 6H), 3.25-3.30 (q, ³J_{HH}= 6.7 Hz, CH-N, 1H), 4.03-4.08 (m, CH₂-O, 4H), 7.13-7.17 (m, CH_{Ar}, 3H), 7.27-7.29 (m, CH_{Ar}, 2H); ³¹P{¹H} (CDCl₃, 202.4 MHz): δ = 5.53; ¹³C (CDCl₃, 125.7 MHz): δ = 14.10 (CH₃), 20.12 (CH₃), 22.69 (CH₂), 26.66 (CH₂), 29.37 (CH₂), 29.64 (CH₂), 29.70 (CH₂), 29.97 (CH₂), 30.67 (CH₂), 31.93 (CH₂), 38.65 (d, ³J_{CP} = 7.30 Hz, CH), 43.11 (CH₃-N), 65.57 (CH), 70.82 (d, ³J_{CP} = 5.3 Hz, CH₂-O), 118.34 (CH_{Ar}), 119.09 (CH_{Ar}), 123.89 (CH_{Ar}), 129.33 (CH_{Ar}), 146.31 (C_{Ar}), 150.89 (C_{Ar}-O); IR (ATR, neat): 2921.55, 2852.48, 1457.79, 1278.56 (v P=O), 1016.35, 720.16, 521.83; HRMS ESI : m/z for [C₅₉H₁₁₅N₂O₃P] : calcd 918.51, found 918.8470;

### Synthesis of phosphonate

### O,O-bis(2-decyltetradecyl)-N-(((3-(1H-imidazol-1-yl)propyl)amino)(1H-imidazol-2-yl)methyl)phosphonate (compound 041)

The crude (*Z*)-*N*-(3-(1*H*-imidazol-1-yl)propyl)-1-(1*H*-imidazol-2-yl)methanimine (259 mg, 1.28 mmol, 1 eq.) and *O,O*-bis(2-decyltetradecyl) phosphite (1 g, 1.28 mmol, 1 eq.) were combined in a flask. The reaction was stirred at 100°C under nitrogen atmosphere for 20h. The reaction course was monitored by ³¹P NMR. After completion, the crude compound was purified by chromatography on silica gel CH₂Cl₂/MeOH/NH₄OH (25% in water) from 100/10/0.1 to 100/10/0.5 to give a brown oil (558 mg, 45% yield). R_{f} (CH₂Cl₂/MeOH/NH₄OH (25% in water) 100/10/1 (v/v/v)): 0.47; ¹H NMR (CDCl₃, 400.0 MHz): δ = 0.83-0.86 (t, ³J_{HH}= 6.9 Hz, CH₃, 12H), 1.22 (broad, CH₂, 80H), 1.44 (s broad, CH, 1H), 1.59 (s broad, CH, 1H), 1.80-1.85 (p, ³J_{HH} = 6.7 Hz, CH₂, 2H), 2.47-2.60 (m, CH₂-NH, 2H), 3.59 (m, CH₂-N_{Ar}, 1H), 3.79 (m, CH₂-N_{Ar}, 1H), 3.94-3.96 (t, ³J_{HH} = 6.3 Hz, CH₂-O, 4H), 4.24 (d, ³J_{HH}= 21.1 Hz, CH, 1H), 6.82 (s, CH_{Ar}, 1H), 6.95 (s, CH_{Ar}, 2H), 6.98 (s, CH_{Ar}, 1H), 7.39 (s, CH_{Ar}, 1H); ³¹P{¹H} (CDCl₃, 202.4 MHz): δ = 21.93; ¹³C (CDCl₃, 125.7 MHz): δ = 16.82 (CH₃), 25.40 (CH₂), 29.37 (CH₂), 32.08 (CH₂), 32.38 (CH₂), 32.43 (CH₂), 32.70 (CH₂), 32.74 (CH₂), 33.22 (d, ³J_{CP} = 9.1 Hz, CH₂), 33.40 (d, ³J_{CP} = 7.0 Hz, CH₂), 33.71 (CH₂), 34.64 (CH₂), 41.46 (dd, ³J_{CP} = 20.9 Hz, ³J_{CP} = 6.0 Hz, CH), 47.02 (CH₂-N_{Ar}), 47.66 (d, ³J_{CP} = 16.3 Hz, CH₂-NH), 57.36 (d, ³J_{CP} = 158.0 Hz, CH), 72.55 (dd, ³J_{CP} = 53.9 Hz, ³J_{CP} = 7.6 Hz, CH₂-O), 121.51 (CH_{Ar}), 132.06 (CH_{Ar}), 139.84 (CH_{Ar}), 145.84 (C); IR (ATR, neat): 3155.32 (v N-H), 2921.17, 2852.24, 1465.05, 1231.51 (v P=O), 1005.17, 723.75; HRMS m/z calcd for [C₅₈H₁₁₃N₅O₃P]⁺: 958.8576, found: 958.8590; calcd for [C₅₈H₁₁₄N₅O₃P]²⁺: 479.9324, found: 479.9323; 622.4819: fragmentation (-C24) -> 143.5565 (2+) = 286.1065 (-C24).

### O,O-bis(2-decyltetradecyl)-N-(((3-(dimethylamino)propyl)amino)(1H-imidazol-2-yl)methyl)phosphonate (compound 042)

The crude (*Z*)-3-(((1*H*-imidazol-2-yl)methylene)amino)-N,N-dimethylpropan-1-amine (259 mg, 1.28 mmol, 1 eq.) and *O,O*-bis(2-decyltetradecyl) phosphite (1 g, 1.28 mmol, 1 eq.) were combined in a flask. The reaction was stirred at 100°C under nitrogen atmosphere for 1h30. The reaction course was monitored by ³¹P NMR. After completion, the crude compound was purified by chromatography on silica gel CH₂Cl₂/MeOH/NH₄OH (25% in water) from 100/10/0.1 to 100/10/0.5 to give a brown oil (783 mg, 66% yield). R_{f} (CH₂Cl₂/MeOH/NH₄OH (25% in water) 100/10/1 (v/v/v)): 0.33; ¹H NMR (CDCl₃, 500.0 MHz) : δ = 0.84-0.87 (t, ³J_{HH} = 7.2 Hz, CH₃, 12H), 1.23 (broad, CH₂, 80H), 1.45 (s broad, CH, 1H), 1.61-1.64 (s broad, CH + CH₂, 3H), 2.24 (s, CH₃-N, 6H), 2.31-2.34 (m, CH₂-NMe₂, 1H), 2.45-2.47 (m, CH₂-NMe₂, 1H), 2.58-2.62 (m, CH₂-NH, 1H), 2.65-2.67 (m, CH₂-NH, 1H), 3.67-3.71 (m, CH₂-O, 1H), 3.77-3.81 (m, CH₂-O, 1H), 3.95-3.98 (m, CH₂-O, 2H), 4.32 (d, ³J_{HH}= 19.5 Hz, CH, 1H), 6.95 (s, CH_{Ar}, 2H); ³¹P{¹H} (CDCl₃, 202.4 MHz): δ = 21.83; ¹³C (CDCl₃, 125.7 MHz): δ = 16.82 (CH₃), 25.40 (CH₂), 29.30 (CH₂), 29.36 (CH₂), 29.41 (CH₂), 29.66 (CH₂), 32.09 (CH₂), 32.39 (CH₂), 32.44 (CH₂), 32.72 (CH₂), 32.77 (CH₂), 33.23 (CH₂), 33.30 (CH₂), 33.35 (CH₂), 33.40 (CH₂), 34.64 (CH₂), 41.46 (dd, ³J_{CP} = 14.3 Hz, ³J_{CP} = 5.9 Hz, CH), 47.76 (CH₃-N), 49.00 (d, ³J_{CP} = 15.7 Hz,-NMe₂), 57.80 (d, ³J_{CP} = 154.4 Hz, CH), 60.06 (CH₂-NH), 72.36 (dd, ³J_{CP} = 61.1 Hz, ³J_{CP} = 7.4 Hz, CH₂-O), 146.46 (d, ³J_{CP} = 3.6 Hz, C); IR (ATR, neat): 2921.22, 2852.36, 1464.06, 1229.01 (v P=O), 1009.19, 734.83; HRMS m/z calcd for [C₅₇H₁₁₆N₂O₃P]⁺: 935.8780, found: 935.8784; calcd for [C₅₇H₁₁₇N₄O₃P]²⁺: 468.4426, found: 468.4427; 599.5022: fragmentation (-C24) -> 132.0668 (2+) = 263.1267 (-C24).

### O,O-bis(2-decyltetradecyl)-N-(((2-(dimethylamino)ethyl)amino)(phenyl)methyl)phosphonate (compound 043)

2-(benzylideneamino)-*N*,*N*-dimethylethan-1-amine and *O,O*-bis(2-decyltetradecyl)phosphite were heated 6h at 100°C. The crude compound was purified by chromatography on silica gel CH₂Cl₂/MeOH from 100/2 to 100/5 to produce 952 mg of colorless oil (Yield: 80%). R_{f} (CH₂Cl₂/MeOH 100/7 (v/v) : 0.27; ¹H NMR (CDCl₃, 500.0 MHz): δ = 0.84-0.87 (t, ³J_{HH}= 6.7 Hz, CH, 12H), 1.24 (broad, 80H), 1.41 (s broad, C, 1H), 1.53 (s broad, CH, 1H), 2.14 (s, CH₃-N, 6H), 2.32-2.41 (m, CH₂-NMe₂, 2H), 2.50-2.62 (m, CH₂-NH, 2H), 2.77 (s broad, NH, 1H), 3.65-3.66 (m, CH₂-O, 1H), 3.79-3.82 (m, CH₂-O, 1H), 3.84-3.88 (m, CH₂-O, 2H), 4.01 (d, ³J_{HH}= 19.6 Hz, CH, 1H), 7.21-7.30 (m, CH_{Ar}, 3H), 7.37-7.39 (m, CH_{Ar}, 2H); ³¹P{¹H} (CDCl₃, 202.4 MHz): δ = 23.63; ¹³C (CDC1₃, 125.7 MHz): δ = 16.82 (CH₃), 25.40 (CH₂), 29.35 (CH₂), 33.09 (CH₂), 32.39 (CH₂), 32.44 (CH₂), 32.73 (CH₂), 33.34 (CH₂), 33.39 (CH₂), 33.48 (CH₂), 34.65 (CH₂), 41.46 (dd, ³J_{CP} = 6.6 Hz, ³J_{CP} = 8.9 Hz, CH), 48.06 (CH₃-N), 48.33 (d, ³J_{CP} = 16.4 Hz, CH₂-NMe₂), 61.5 (CH₂-NH), 64.01 (d, ³J_{CP} = 153.5 Hz, CH), 71.66 (dd, ²J_{CP} = 7.5 Hz, ²J_{CP} = 25.2 Hz, CH₂-O), 130.34 (CH_{Ar}), 130.96 (CH_{Ar}), 131.22 (CH_{Ar}), 131.27 (CH_{Ar}), 139.25 (C); IR (ATR, neat): 2921.11, 2852.10, 1457.39, 1246.04 (v P=O), 1001.02, 697.72, 550.40; HRMS m/z calcd for [C₅₉H₁₁₆N₂O₃P]⁺: 931.8718, found: 931.8707; 595.4955: fragmentation (-C24) -> 259.1204 (-C24).

### O,O-bis(2-decyltetradecyl)-hydroxy-(pyridine-4-yl)phosphonate (compound 044)

Bis(2-decyltetradecyl)phosphite (0.5 g, 0.66 mmol, 1 eq.) and isonicotinaldehyde (0.14 g, 0.99 mmol, 1.5 eq.) were placed under nitrogen in a round-bottom flask. The mixture was heated at 120°C for 2 hours. The product was purified by chromatography on silica gel CH₂Cl₂/MeOH from 100/1 to 100/7 to produce 0.16 g of colorless viscous oil (Yield: 28%). R_{f} (CH₂Cl₂/MeOH: 100/10 (v/v)): 0.43; ¹H NMR (CDC1₃, 500.0 MHz): δ = 0.87 (t, ³J_{HH} = 6.6 Hz, CH₃, 12H), 1.25 (broad, 80H), 1.51 (broad, CH, 2H), 3.91 (m, CH₂-O, 4H), 4.46(s broad, OH, 1H), 5.06 (d, ²J_{HP} = 12.8 Hz, CH, 2H), 7.42 (s broad, C_{Ar}-H, 2H), 8.55 (s broad, C_{Ar}-H, 1H); ³¹P{¹H} (CDCl₃, 202.4 MHz): δ = 19.96 ; ¹³C (CDC1₃, 125.7 MHz): δ = 14.12 (CH₃), 22.71 (CH₂), 26.64 (CH₂), 29.39 (CH₂), 29.69 (CH₂), 29.74 (CH₂), 30.00 (CH₂), 30.63 (CH₂), 30.71 (CH₂), 31.95 (CH₂), 38.78 (m, CH), 69.46 and 70.20 (2d, ²J_{CP} = 6.9 and 7.6 Hz, CH₂-O), 69.64 (d, ¹J_{CP} = 157.7 Hz, CH-P), 121.81 (C_{Ar}-H), 146.83 (C_{Ar}), 148.99 (C_{Ar}-H); IR (neat, ): 3880.30, 2921.03, 2852.13, 1597.03, 1465.37, 1244.96 & 1220.63 (v P=O), 1003.26, 721.02;

### O,O-ditetradecyl- (((2-(dimethylamino)ethyl)(methyl)amino)methyl)phosphonate (compound 045)

Ditetradecylphosphite (0.5 g, 1.03 mmol, 1 eq.), paraformaldehyde (0.06g, 2.1 mmol, 2 eq.), and N,N,N'-trimethylethylenediamine (0.42 g, 4.12 mmol, 4 eq.) were placed under nitrogen in a round-bottom flask. The mixture was heated at 120°C for 4 hours. CH₂Cl₂ (70 mL) was added and the solution was washed with water (3x25 mL), dried over MgSO₄, filtrated and concentrated. The residue was purified by chromatography on silica gel with CH₂Cl₂/MeOH/NH₄OH (25% in water) as eluent (from 100/3/0.3 to 100/10/1) to produce 0.39 g of colorless viscous oil (Yield: 64%). R_{f} (CH₂Cl₂/MeOH: 100/10/NH₄OH (25%) (v/v/v)): 0.59; ¹H NMR (CDCl₃, 500.0 MHz): δ = 0.98 (t, ³J_{HH} = 6.9 Hz, CH₃, 6H), 1.40 (broad, 44H), 1.76 (q, ³J_{HH} = 7.0 Hz, CH₂, 4H), 2.40 (s, N-Me₂, 6H), 2.55 (s, N-CH₃, 3H), 2.58 (m, CH₂-N, 2H), 2.80 (t, ³J_{HH} = 6.7 Hz, CH₂-N, 2H), 2.98 (d, ²J_{HP} = 10.5 Hz, CH₂-P, 2H), 4.16 (m, CH₂-O, 4H); ³¹P{¹H} (CDC1₃, 202.4 MHz): δ = 25.83 ; ¹³C (CDC1₃, 125.7 MHz): δ = 16.82 (CH₃), 25.39 (CH₂), 28.27 (CH₂), 31.91 (CH₂), 32.06 (CH₂), 32.26 (CH₂), 32.29 (CH₂), 32.36 (CH₂), 32.38 (CH₂), 33.35 (d, ³J_{CP} = 5.7 Hz, CH₂), 34.63 (CH₂), 47.08 (d, ³J_{CP} = 6.4 Hz, N-CH₃), 48.28 (NMe₂), 55.81 (d, ¹J_{CP} = 159.5 Hz, CH₂-P), 59.22 (d, ³J_{CP} = 10.8 Hz, CH₂-N), 59.87 (N-CH₂), 68.65 (d, ²J_{CP} = 6.9 Hz, CH₂-O); IR (neat, ): 2920.61, 2851.71, 1465.18, 1259.84 & 1240.66 (v P=O), 988.64, 721.06

### O,O-bis(2-decyltetradecyl)-(((2-(dimethylamino)ethyl)(methyl)amino)methyl)phosphonate (compound 046)

Bis(2-decyltetradecyl)phosphite (0.5 g, 0.66 mmol, 1 eq.), paraformaldehyde (0.08g, 2.6 mmol, 4 eq.), and N,N,N'-trimethylethylenediamine (0.27 g, 2.65 mmol, 4 eq.) were placed under nitrogen in a round-bottom flask. The mixture was heated at 120°C for 2 hours. CH₂Cl₂ (60 mL) was added and the solution was washed with water (3x25 mL), dried over MgSO₄, filtrated and concentrated. The residue was purified by chromatography on silica gel with CH₂Cl₂/MeOH (100/3) to CH₂Cl₂/MeOH/NH4OH (25% in water) (100/10/0.5) as eluent to produce 0.17 g of colorless viscous oil (Yield: 30%). R_{f} (CH₂Cl₂/MeOH: 100/20 (v/v)): 0.86; ¹H NMR (CDC1₃, 500.0 MHz): δ = 0.86 (t, ³J_{HH} = 6.9 Hz, CH₃, 12H), 1.24 (broad, 80H), 1.59 (s broad, CH, 2H), 2.32 (s, N-Me₂, 6H), 2.43 (s, N-CH₃, 3H), 2.51 (m, CH₂-N, 2H), 2.70 (t, ³J_{HH} = 6.5 Hz, CH₂-N, 2H), 2.87 (d, ²J_{HP} = 10.4 Hz, CH₂-P, 2H), 3.93 (m, CH₂-O, 4H); ³¹P{¹H} (CDC1₃, 202.4 MHz): δ = 25.89 ; ¹³C (CDC1₃, 125.7 MHz): δ = 16.79 (CH₃), 25.38 (CH₂), 29.39 (CH₂), 32.06 (CH₂), 32.36 (CH₂), 32.41 (CH₂), 32.73 (CH₂), 32.48 (CH₂), 33.51 (CH₂), 34.62 (CH₂), 41.53 (d, ³J_{CP} = 6.1 Hz, CH), 46.98 (d, ³J_{CP} = 6.2 Hz, N-CH₃), 48.11 (NMe₂), 55.66 (d, ¹J_{CP} = 158.6 Hz, CH₂-P), 58.59 (d, ³J_{CP} = 10.4 Hz, CH₂-N), 59.73 (N-CH₂), 71.09 (d, ²J_{CP} = 7.1 Hz, CH₂-O); IR (neat, ): 2921.22, 2852.36, 1464.41, 1263.66 & 1240.38 (v P=O), 1033.45, 999.76, 725.86; HRMS (ESI-qTOF), m/z calcd for C₅₄H₁₁₃N₂O₃P+H [M+H]⁺= 869.8567 ; observed [M+H]⁺=869.8556;

### Synthesis from POCl3

### O,O-bis(2-decyltetradecyl)-O-(N,N-dimethylaminoeth-2-yl)phosphoramidate (compound 047)

The protocol was adapted from a protocol reported by S. Le Corre et al (S. S. Le Corre, M. Berchel, T. Le Gall, J.P. Haelters, P. Lehn, T. Montier, P.A. Jaffrès, Eur. J. Org. Chem., 2014, 8041-8048.). Briefly, POCl₃ (0.8 g, 5.21 mmol) were placed in a three neck round-bottom flask under N₂. 40 mL of CH₂Cl₂ were added and the solution was cooled with an ice bath at 3-5°C. a mixture of 2-decyltetradecanol (3.68 g, 10.4 mmol, 2 eq.) and triethylamine (1.31 g, 13 mmol, 2.5 eq.) in CH₂Cl₂ (40 mL) was placed in an additional funnel. This solution was added at 3-5°C. At the end of the addition the temperature was slowly raised up to 20°C and the solution was stirred for 3 days at room temperature (³¹P NMR of the solution indicated one peak at 4.72 ppm). *N*,*N*-dimethylaminoethanol (1.2 g, 13.4 mmol), triethylamine (2.02 g, 10.1 mmol) and CH₂Cl₂ (40 mL) were place in the additional funnel. This solution was added at 3-5°C. Then the temperature was raised to room temperature (RT) and stirred for 5 days at RT (³¹P NMR of the solution revealed the absence of the peak at 4.7 ppm). The solution was concentrated under vacuum. To the residue, Et₂O (80 mL) was added. The solution was filtrated on celite. The filtrate was concentrated and purified by chromatography on silica gel (CH₂Cl₂/MeOH 100/0.5 to 100/4) to produce the expected compound as a colorless oil (1.27 g, 29 % yield). R_{f} (CH₂Cl₂/MeOH : 100/10 (v/v)): 0.52; ¹H NMR (CDC1₃, 500.0 MHz): δ = 0.87 (t, ³J_{HH} = 6.6 Hz, CH₃, 12H), 1.24 (broad, 80H), 1.60 (broad, CH, 2H), 2.29 (s, NMe₂, 6H), 2.62 (m, CH₂-N, 2H), 3.91 (m, CH₂-O, 4H), 4.12 (q, ³J_{HH} ≈ ³J_{HP} ≈ 7.2 Hz, CH₂-O, 2H); ³¹P{¹H} (CDC1₃, 202.4 MHz): δ = 0.04 ; ¹³C (CDC1₃, 125.7 MHz): δ = 14.10 (CH₃), 22.69 (CH₂), 26.68 (CH₂), 29.37 (CH₂), 29.66 (CH₂), 29.71 (CH₂), 30.00 (CH₂), 30.56 (CH₂), 31.93 (CH₂), 38.66 (d, ³J_{CP} = 7.4 Hz, CH), 45.69 (CH₃-N), 58.90 (d, ³J_{CP} = 6.2 Hz, CH₂-N), 65.20 (CH₂-O), 70.19 (d, ²J_{CP} = 6.1 Hz, CH₂-O); IR (neat, ): 2921.04, 2852.25, 1465.10, 1267.63 (v P=O), 1013.92, 721.05

### O,O-bis(2-decyltetradecyl)-O-(N,N-dimethylaminoprop-3-yl)phosphoramidate (compound 048)

POCl₃ (0.8 g, 5.21 mmol) were placed in a three neck round-bottom flask under N₂. 40 mL of CH₂Cl₂ were added and the solution was cooled with an ice bath at 3-5°C. a mixture of 2-decyltetradecanol (3.68 g, 10.4 mmol, 2 eq.) and triethylamine (1.31 g, 11.3 mmol, 2.5 eq.) in CH₂Cl₂ (40 mL) was placed in an additional funnel. This solution was added at 3-5°C. At the end of the addition the temperature was slowly raised up to 20°C and the solution was stirred for 3 days at room temperature (³¹P NMR of the solution indicated one peak at 4.11 ppm). N,N-dimethylaminopropanol (1.07 g, 10.4 mmol), triethylamine (1.31 g, 11.3 mmol) and CH₂Cl₂ (40 mL) were place in the additional funnel. This solution was added at 3-5°C. Then the temperature was raised to room temperature (RT) and stirred for 2 days at RT (³¹P NMR of the solution revealed the absence of the peak at 4.11 ppm). The solution was concentrated under vacuum. To the residue, Et₂O (80 mL) was added. This solution was washed with water (3x50 mL), dried over MgSO₄, filtrated and concentrated. The residue was purified by chromatography on silica gel (CH₂Cl₂/MeOH 100/0.5 to 100/2) to produce the expected compound as a colorless oil (0.84 g, 19 % yield). R_{f} (CH₂Cl₂/MeOH : 100/10 (v/v)): 0.50; ¹H NMR (CDC1₃, 500.0 MHz): δ = 0.87 (t, ³J_{HH} = 6.6 Hz, CH₃, 12H), 1.25 (broad, 80H), 1.61 (broad, CH, 2H), 1.85 (q, ³J_{HH} =6.9 Hz, CH₂, 2H), 2.24 (s, NMe₂, 6H), 2.39 (m, CH₂-N, 2H), 3.91 (m, CH₂-O, 4H), 4.07 (q, ³J_{HH} ≈ ³J_{HP} ≈ 6.7 Hz, CH₂-O, 2H); ³¹P{¹H} (CDCl₃, 202.4 MHz): δ = - 0.05 ; ¹³C (CDCl₃, 125.7 MHz): δ = 16.82 (CH₃), 25.40 (CH₂), 29.39 (CH₂), 31.20 (d, ³J_{CP} = 5.8 Hz, CH₂), 32.08 (CH₂), 32.38 (CH₂), 32.42 (CH₂), 32.72 (CH₂), 33.42 (CH₂), 34.64 (CH₂), 41.38 (d, ³J_{CP} = 7.3 Hz, CH), 48.09 (CH₃-N), 58.50 (CH₂-N), 68.56 (d, ²J_{CP} = 5.7 Hz, CH₂-O), 72.83 (d, ²J_{CP} = 6.0 Hz, CH₂-O); IR (neat, ): 2921.27, 2852.52, 1464.02, 1283.53 & 1267.83 (v P=O), 1010.76, 721.42; HRMS (ESI-qTOF), m/z calcd for C₅₃H₁₁₀NO₄P+H [M+H]⁺= 856.8251 ; observed [M+H]⁺=856.8320;

### O,O-dodecyl(2-hexyldecyl)-N-(2-(dimethylamino)ethyl)phosphoramidate (compound 049)

Phosphoryl trichloride (0.511 g, 3.3 mmol, 1 eq.) was placed in a round-bottom flask under N₂. 5 mL of Et₂O was added and the solution was cooled with an ice bath at -5°C. Triethylamine (460 µL, 3.3 mmol, 1 eq.) was added followed by 2-hexyl-1-decanol (0.808 g, 3.3 mmol, 1 eq.) added dropwise. The solution was stirred 1h30 and temperature was raised up to 0°C. Then, a mixture of triethylamine (506 µL, 3.63 mmol, 1.1 eq.) and dodecan-1-ol (0.615 g, 3.3 mmol, 1 eq.) in Et₂O (5 mL) was added dropwise. The solution was stirred 4h and slowly raised to room temperature. Finally, a solution containing triethylamine (460 µL, 3.3 mmol, 1 eq.) and N,N-dimethylethylene diamine (0.291 g, 3.3 mmol, 1 eq.) in Et₂O (5 mL) was added. After 1h, 15 mL of Et₂O was added and the organic layer was washed twice with a saturated solution of NaCl, dried over MgSO₄, filtered and concentrated under vacuum. The crude product was purified by chromatography on silica gel (CH₂Cl₂/MeOH 100/2 to 100/5) to produce the expected compound as a colorless oil (0.380 g, 20 % yield). R_{f} (CH₂Cl₂/MeOH 100/7 (v/v)): 0.20 ; ¹H NMR (CDC1₃, 400.0 MHz): δ = 0.88-0.92 (t, ³J_{HH}= 6.8 Hz, CH₃, 9H), 1.28 (broad, 40H), 1.61-1.70 (m, CH, CH₂ β-O, 3H), 1.79-1.81 (m, CH₂, 2H), 2.41 (s, CH₃-N, 6H), 2.61-2.63 (m, CH₂-NMe₂, 2H), 3.04-3.07 (m, CH₂-NH, 2H), 3.51-3.54 (s broad, NH, 1H), 3.84-3.93 (m, CH₂ α-O, 2H), 3.95-4.03 (m, CH₂ α-O, 2H); ³¹P{¹H} (CDC1₃, 202.4 MHz): δ = 9.7; ¹³C (CDC1₃, 101 MHz): δ = 13.90 (CH₃), 22.48 (CH₂), 25.44 (CH₂), 26.46 (CH₂), 26.50 (CH₂), 29.05 (CH₂), 29.15 (CH₂), 29.41 (CH₂), 29.47 (CH₂), 29.81 (CH₂), 30.20 (CH₂), 30.27 (CH₂), 30.65 (CH₂), 30.71 (CH₂), 31.65 (CH₂), 31.72 (CH₂), 38.44 (CH₂-NH), 38.51 (CH), 44.86 (CH₃-N), 59.46 (d, ³J_{CP} = 7.0 Hz, CH₂-NMe₂), 66.14 (d, ²J_{CP} = 5.8 Hz, CH₂-O), 68.49 (d, ²J_{CP} = 6.1 Hz, CH₂-O); IR (ATR, neat): 3210 (NH), 2916, 2848,

### O,O-dodecyl (2-hexyldecyl)-N-(3-(dimethylamino)propyl)phosphoramidate (compound 050)

Phosphoryl trichloride (0.506 g, 3.3 mmol, 1 eq.) was placed in a round-bottom flask under N₂. 10 mL of Et₂O was added and the solution was cooled with an ice bath at -5°C. Triethylamine (466 µL, 3.3 mmol, 1 eq.) was added followed by 2-hexyl-1-decanol (0.800 g, 3.3 mmol, 1 eq.) added dropwise. The solution was stirred 1h30 and temperature was raised up to 0°C. Then, a mixture of triethylamine (506 µL, 3.63 mmol, 1.1 eq.) and dodecan-1-ol (0.334 g, 3.3 mmol, 1 eq.) in Et₂O (5 mL) was added dropwise. The solution was stirred 4h and slowly raised to room temperature. Finally, a solution containing triethylamine (466 µL, 3.28 mmol, 1 eq.) and N,N-dimethylpropane-1,3-diamine (0.337 g, 3.3 mmol, 1 eq.) in Et₂O (5 mL) was added. After 1h, 15 mL of Et₂O was added and the organic layer was washed twice with a saturated solution of NaCl, dried over MgSO₄, filtered and concentrated under vacuum. The crude product was purified by chromatography on silica gel (CH₂Cl₂/MeOH 100/2 to 100/5) to produce the expected compound as a colorless oil (0.230 g, 11 % yield). Rf (CH₂Cl₂/MeOH 100/7 (v/v)): 0.16; ¹H NMR (CDC1₃, 400.0 MHz): δ = 0.88-0.91 (t, ³J_{HH} = 6.7 Hz, CH₃, 9H), 1.27 (broad, 42H), 1.61-1.69 (m, CH, CH₂ β-O, 3H), 1.75-1.81 (q, ³J_{HH}= 6.1 Hz, CH₂ polar head), 2.40 (s, CH₃-N, 6H), 2.59-2.61 (m, CH₂-NMe₂, 2H), 3.00-3.08 (m, CH₂-NH, 2H), 3.55-3.58 (m, NH, 1H), 3.83-3.92 (m, CH₂ α-O, 2H), 3.93-4.04 (m, CH₂ α-O, 2H); ³¹P{¹H} (CDC1₃, 162 MHz): δ = 9.69; ¹³C (CDC1₃, 101 MHz): δ = 13.96 (CH₃), 22.53 (CH₂), 25.50 (CH₂), 26.51 (CH₂), 27.75 (CH₂), 29.12 (CH₂), 29.20 (CH₂), 29.46 (CH₂), 29.52 (CH₂), 29.87 (CH₂), 30.27 (CH₂), 30.34 (CH₂), 30.67 (CH₂), 30.73 (CH₂), 31.70 (CH₂), 31.77 (CH₂), 38.52 (d, ³J_{CP} = 7.6 Hz, CH), 39.81 (CH₂-NH), 44.58 (CH₃-N), 57.19 (CH₂-NMe₂), 66.26 (d, ²J_{CP} = 5.9 Hz, CH₂-O), 68.64 (d, ²J_{CP} = 6.1 Hz, CH₂-O); IR (ATR, neat): 3221 (NH), 2921, 2848, 1462, 1229 (v P=O), 1002, 865, 720.

### O,O-(Z)-octadec-9-en-1-yl (2-octyldodecyl)-N-(2-(dimethylamino)ethyl)phosphoramidate (compound 51)

Phosphoryl trichloride (0.618 g, 4.03 mmol, 1 eq.) was placed in a round-bottom flask under N₂. 5 mL of Et₂O was added and the solution was cooled with an ice bath at -5°C. Triethylamine (531 µL, 3.81 mmol, 1 eq.) was added followed by 2-octyldecanol (1.20 g, 4.03 mmol, 1 eq.) added dropwise. The solution was stirred 1h30 and temperature was raised up to 0°C. Then, a mixture of triethylamine (618 µL, 4.43 mmol, 1.1 eq.) and oleic dodecanol (0.751 g, 4.03 mmol, 1 eq.) in Et₂O (5 mL) was added dropwise. The solution was stirred 4h and slowly raised to room temperature. Finally, a solution containing triethylamine (562 µL, 4.03 mmol, 1 eq.) and N,N-dimethylethylene diamine (0.319 g, 4.03 mmol, 1 eq.) in Et₂O (5 mL) was added. After 1h, 15 mL of Et₂O was added and the organic layer was washed twice with a saturated solution of NaCl, dried over MgSO₄, filtered and concentrated under vacuum. The crude product was purified by flash chromatography (CH₂Cl₂/MeOH 100/0 to 97/3) to produce the expected compound as a brown oil (0.396 g, 20 % yield). Rf (CH₂Cl₂/MeOH 100/7 (v/v)): 0.43; ¹H NMR (CDCl₃, 400.0 MHz): δ = 0.87-0.90 (t, ³J_{HH}= 6.7 Hz, CH₃, 9H), 1.26 (broad, 50H), 1.60-1.68 (m, CH, CH₂ β-O, 3H), 2.25 (s, CH₃-N, 6H), 2.40-2.43 (t, ³J_{HP} = 6.0 Hz, CH₂-NMe₂, 2H), 2.95-3.02 (dq, J = 8.4, 6.0 Hz, CH-NH, 2H), 3.28-3.34 (dt, J = 11.6, 6.0 Hz, NH, 1H), 3.83-3.93 (m, CH₂ α-O, 2H), 3.94-4.03 (m, CH₂ α-O, 2H); ³¹P{¹H} (CDC1₃, 162 MHz): δ = 9.96; ¹³C (CDC1₃, 101 MHz): δ = 14.10 (CH₃), 22.68 (CH₂), 25.63 (CH₂), 26.69 (CH₂), 29.24 (CH₂), 29.35 (CH₂), 29.60 (CH₂), 29.64 (CH₂), 30.00 (CH₂), 30.39 (CH₂), 30.46 (CH₂), 30.83 (CH₂), 30.88 (CH₂), 31.91 (CH₂), 38.62 (CH₂-NH), 38.69 (CH), 45.06 (CH₃-N), 59.63 (d, ³J_{CP} = 6.9 Hz, CH₂-NMe₂), 66.35 (d, ²J_{CP} = 5.8 Hz, CH₂-O), 68.72 (d, ²J_{CP} = 6.0 Hz, CH₂-O); IR (ATR, neat): 3218 (NH), 2921, 2847, 1462, 1231 (v P=O), 1000, 853, 720.

Compounds prepared by the Atherton-Todd reaction.

### di(pentadecan-8-yl) (2-(dimethylamino)ethyl)phosphoramidate (compound 52)

Protocol B: *O*,*O*-bis(pentadecane-8-yl) phosphite (0,5 g, 1.0 mmol, 1 eq.), *N,N-*dimethylethylenediamine (0.115 g, 1.3 mmol, 1.3 eq), *N*,*N*-diisopropylethylamine (0.168 g, 1.3 mmol, 1.3 eq.) and bromotrichloromethane (0.258 g, 1.3 mmol, 1.3 eq) were used. The crude compound was purified by chromatography on silica gel (CH₂Cl₂/MeOH 100/1 to 100/4) to produce 230 mg of colorless oil (Yield: 39%). R_{f} (CH₂Cl₂/MeOH 100/10 (v/v)): 0.62; ¹H NMR (CDC1₃, 400.0 MHz): δ = 0.81 (t, ³J_{HH} = 6.7 Hz, CH₃, 12H), δ = 1.19-1.22 (m, CH₂, 40H), 1.51-1.55 (m, CH₂ β-O, 8H), 2.14 (s, CH₃-N, 6H), 2.30 (t, ³J_{HH} = 6.0 Hz, CH₂-NMe₂, 2H), 2.85-2.92 (m, CH₂-NH, 2H), 3.05 (broad, NH, 1H), 4.19-4.27 (m, CH-O, 2H); ³¹P{¹H} (CDC1₃, 202.4 MHz): δ = 7.58; ¹³C (CDC1₃, 125.7 MHz): δ = 14.23 (CH₃), 22.80 (CH₂), 25.10 (CH₂), 29.42 (CH₂), 29.46 (CH₂), 29.81 (CH₂), 31.99 (d, ³J_{CP} = 2.9 Hz, CH₂), 35.11 (CH₂), 38.96 (CH₂), 45.26 (CH₃-N), 59.75 (d, ²J_{CP} = 7.7 Hz, CH₂-NMe₂), 77.97 (d, ²J_{CP} = 6.2 Hz, CH-O); IR (ATR, neat): 3195 (v N-H), 2917, 2849, 1455, 1230 (v P=O), 975 (v C-C).

*di(pentadecan-8-yl) (2-(pyrrolidin-1-yl)ethyl)phosphoramidate (compound 53)* Protocol B : *O,O*-bis(pentadecane-8-yl) phosphite (0,360 g, 0,72 mmol, 1 eq.), *N-*(2-Aminoethyl)pyrrolidine (0.107 g, 0.93 mmol, 1.3 eq), *N,N*-diisopropylethylamine (0.121 g, 0.93 mmol, 1.3 eq.) and bromotrichloromethane (0.186 g, 0.93 mmol, 1.3 eq) were used. The crude compound was purified by chromatography on silica gel (CH₂Cl₂/MeOH 100/1 to 100/4) to produce 130 mg of yellow oil (Yield: 29%). R_{f}(CH₂Cl₂/MeOH 100/10 (v/v)): 0.6; ¹H NMR (CDC1₃, 400.0 MHz): δ = 0.90 (t, ³J_{HH} = 6.7 Hz, CH₃, 12H), 1.29 (broad, 40H), 1.58-1.65 (m, CH₂ β-O, 8H) 1.79-1.82 (m, CH₂ β-N, 4H), 2.59-2.64 (m, CH₂-N, 6H), 3.01-3.08 (m, CH₂-NH), 3.25 (broad, NH), 4.29-4.34 (m, CH-O, 2H); ³¹P{¹H} (CDC1₃, 202.4 MHz): δ = 7.49; ¹³C (CDC1₃, 125.7 MHz): δ = 14.10 (CH₃), 22.67 (CH₂), 23.50 (CH₂ β-N), 24.97 (CH₂), 29.32 (d, ³J_{CP} = 3.1 Hz, CH₂), 29.69 (CH₂), 31.87 (CH₂), 34.97 (CH₂), 39.82 (CH₂), 53.83 (CH₂-N), 56.39 (d, ²J_{CP} = 7.2 Hz, CH₂-N), 77.91 (d, ²J_{CP} = 6.1 Hz, CH-O); IR (ATR, neat): 3193 (v N-H), 2917, 2849, 1457, 1232 (v P=O), 976 (v C-C).

*di(nonadecan-10-yl) (2-(dimethylamino)ethyl)phosphoramidate (compound 54)* Protocol B : *O*,*O*-bis(nonadecane-10-yl) phosphite (0,5 g, 0.81 mmol, 1 eq.), *N,N-*dimethylethylenediamine (0.093 g, 1.05 mmol, 1.3 eq), N,N-diisopropylethylamine (0.136 g, 1.05 mmol, 1.3 eq.) and bromotrichloromethane (0.208 g, 1.05 mmol, 1.3 eq) were used. The crude compound was purified by chromatography on silica gel (CH₂Cl₂/MeOH 100/1 to 100/4) to produce 240 mg of colorless oil (Yield: 42%). R_{f} (CH₂Cl₂/MeOH 100/10 (v/v)): 0.72; ¹H NMR (CDC1₃, 400.0 MHz): δ = 0.81 (t, ³J_{HH} = 6.6 Hz, CH₃, 12H), δ = 1.19 (broad, 56H), 1.49-1.56 (m, CH₂ β-O, 8H), 2.14 (s, CH₃-N, 6H), 2.30 (t, ³J_{HH} = 6.0 Hz, CH₂-NMe₂, 2H), 2.87-2.90 (m, CH₂-NH, 2H), 3.07 (broad, NH, 1H), 4.21-4.26 (m, CH-O, 2H); ³¹P{¹H} (CDC1₃, 202.4 MHz): δ = 7.56; ¹³C (CDCl₃, 125.7 MHz): δ = 14.09 (CH₃), 22.68 (CH₂), 24.96 (CH₂), 29.35 (d, ³J_{CP} = 2.8 Hz, CH₂), 29.62 (CH₂), 29.70 (d, ³J_{CP} = 4.6 Hz, CH₂), 31.91 (CH₂), 34.97 (CH₂), 38.82 (CH₂), 45.11 (CH₃-N), 59.62 (d, ²J_{CP} =7.8 Hz, CH₂-NMe₂), 77.82 (d, ²J_{CP} = 6.1 Hz, CH-O).

*di(nonadecan-10-yl) (2-(pyrrolidin-1-yl)ethyl)phosphoramidate (compound 55)* Protocol B : *O*,*O*-bis(nonadecane-10-yl) phosphite (0,5 g, 0.81 mmol, 1 eq.), *N*-(2-Aminoethyl)pyrrolidine (0.120 g, 1.05 mmol, 1.3 eq), N,N-diisopropylethylamine (0.136 g, 1.05 mmol, 1.3 eq.) and bromotrichloromethane (0.208 g, 1.05 mmol, 1.3 eq) were used. The crude compound was purified by chromatography on silica gel (CH₂Cl₂/MeOH 100/1 to 100/4) to produce 270 mg of yellow-white solid (Yield: 46%). R_{f} (CH₂Cl₂/MeOH 100/10 (v/v)): 0.58; ¹H NMR (CDC1₃, 400.0 MHz): δ = 0.89 (t, ³J_{HH} = 6.8 Hz, CH₃, 12H), 1.27 (broad, 56H), 1.58-1.65 (m, CH₂ β-O, 8H) 1.78-1.80 (m, CH₂ β-N, 4H), 2.57-2.64 (m, CH₂-N, 6H), 3.00-3.06 (m, CH₂-NH, 2H) 3.23 (broad, NH), 4.28-4.35 (m, CH-O, 2H); ³¹P{¹H} (CDC1₃, 202.4 MHz): δ = 7.49; ¹³C (CDC1₃, 125.7 MHz): δ = 14.11 (CH₃), 22.69 (CH₂), 23.50 (CH₂), 24.97 (CH₂), 29.36 (d, ³J_{CP} = 2.4 Hz, CH₂), 29.64 (CH₂), 29.71 (d, ³J_{CP} = 5.0 Hz, CH₂), 31.92 (CH₂), 34.97 (CH₂), 39.86 (CH₂), 53.81 (CH₂-N), 56.38 (d, ²J_{CP} = 7.3 Hz, CH₂-N), 77.89 (d, ²J_{CP} = 6.1 Hz, CH-O).

### dihexyl 2,2'-(((((2-(dimethylamino)ethyl)amino)-phosphoryl)-bis(oxy))bis(methylene))didodecanoate (compound 56)

*N*,*N*-dimethylethylenediamine (0.085 g, 0.96 mmol, 1.3 eq.), dihexyl 2,2'-(((oxo-15-phosphanediyl)bis(oxy))bis(methylene))didodecanoate (0.5 g, 0.74 mmol, 1 eq.), DIPEA (0.12 g, 0.96 mmol, 1.3 eq.) and bromotrichloromethane (0.19 g, 0.96 mmol, 1.3 eq.) were used. The crude compound was purified by chromatography on silica gel CH₂Cl₂/MeOH from 100/10 to produce 0.28 g of colorless oil as a mixture of diastereoisomers (Yield: 50%). Rf (CH₂Cl₂/MeOH 100/15 (v/v) : 0.52 ; ¹H NMR (CDCl₃, 400.0 MHz): mixture of diastereoisomers, δ = 0.9 (m, CH₃ fatty chain, 12H), 1.27-1.38 (broad, 44H), 1.50-1.67 (m, 8H), 2.23 (s, N-CH₃, 6H), 2.38 (t, ³J_{HH}= 5.8 Hz, CH₂-N, 2H), 2.70-2.78 (m, CH-CO, 2H), 2.91-2.97 (m, CH₂-NH, 2H), 3.37 (broad, NH, 1H), 4.01-4.17 (m, CH₂-O, 8H); ³¹P{¹H} (CDC1₃, 161.92 MHz): mixture of diastereoisomers, δ = 9.41, 9.49 and 9.55; ¹³C (CDC1₃, 100.6 MHz): mixture of diastereoisomers, δ = 13.95 (CH₃), 13.96 (CH₃), 22.40 (CH₂), 22.53 (CH₂), 25.43 (CH₂), 26.80 (CH₂), 28.36 (CH₂), 28.45 (CH₂), 29.18 (CH₂), 29.28 (CH₂), 29.35 (CH₂), 29.44 (CH₂), 31.28 (CH₂), 31.76 (CH₂), 38.32 (CH₂), 44.88 (N-CH₃), 46.26-46.35 (m, CH), 59.23 (d, ³J_{CP} = 7.1 Hz, CH₂-N, CH₂), 64.58 (CH₂-OCO), 66.26 (broad, CH₂-O-P)

### Formulation.

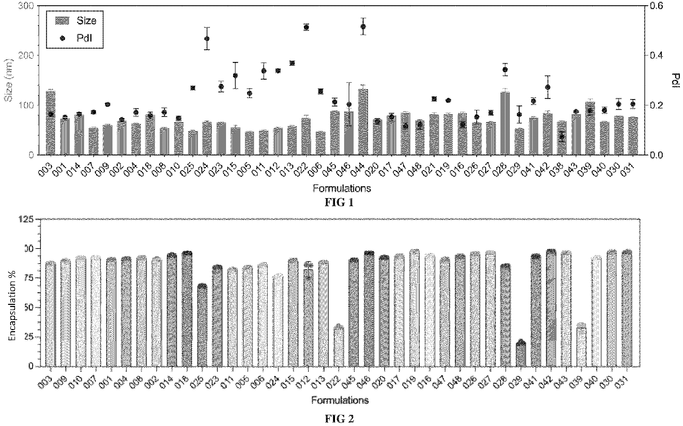

The liposomal nanoparticles were formulated using microfluidic mixing, with a 3:1 flow rate ratio between the mRNA and lipid solutions, respectively. A 10:1 weight ratio of lipid to nucleic acid was used. Ratios of lipid components tested were 35.0% ionizable lipid, 16.0% helper lipid (DOPE), 46.5% Cholesterol, and 2.5% C14-PEG_{2K} (DMPE-PEG2000). Additional ratios were tested as well, to empirically determine the amount of PEG-lipid the particles required for proper encapsulation of mRNA. The classical 50% Ionizable lipid, 38.5% Cholesterol, 10% structural lipid (DOPE), and 1.5% PEG-Lipid was also tested. All liposomal nanoparticles were assayed for size, polydispersity, and encapsulation efficiency. Hit lipid characterization (size, PdI and encapsumlation of mRNA) is shown in **Figure 1 and 2****.**

### mRNA delivery.

In these studies, Firefly Luciferase mRNA was delivered as a reporter and used to screen lipids for efficiency of transfection. Additionally, Cas9 mRNA and sgRNA were co-encapsulated for gene editing experiments. Injections were performed in balb/c or CD1 mice, at a dose of 2-50 µg / mouse. Notably, these liposomal nanoparticles are highly efficient at mRNA delivery, with equal or better delivery as compared to current FDA approved lipids (DLin-DMA-MC3, SM-102)

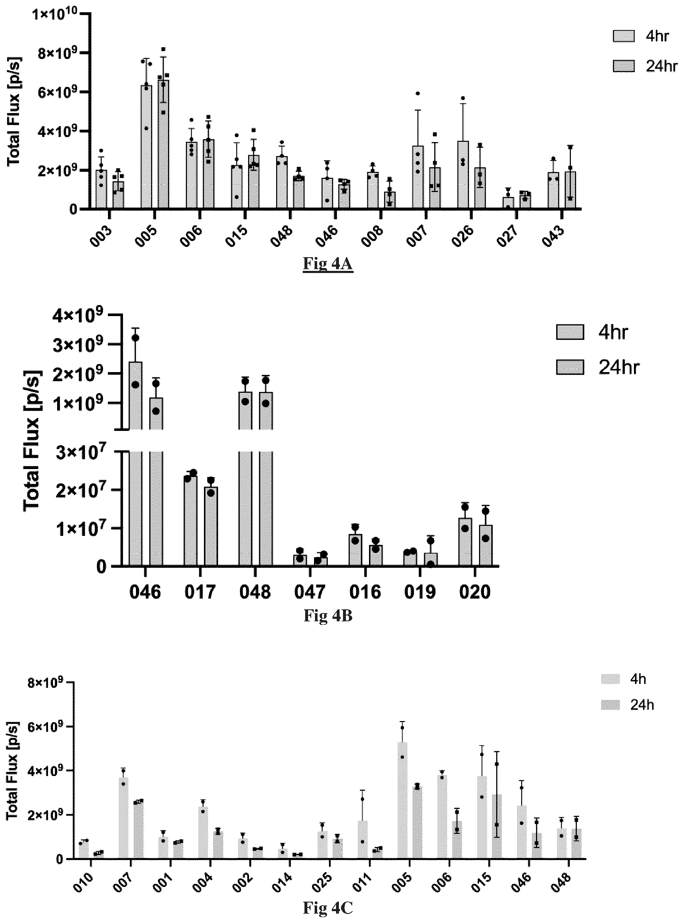

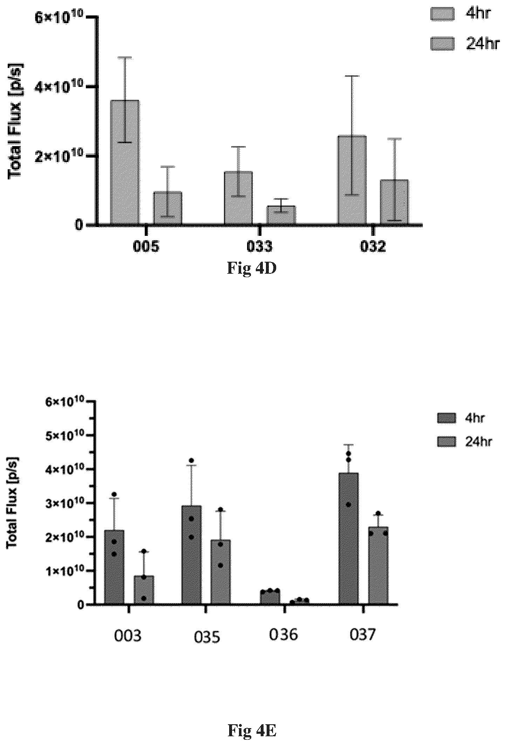

More than 40 compounds were tested in vivo. The results are presented in Figure 4A, 4B, 4C, 4D, 4E.

The compounds that produce the best efficiency of mRNA delivery in vivo (formulation with other lipids) are compounds **003, 005, 006, 007, 015, 026, 032, 033, 035, 037** and **043.**

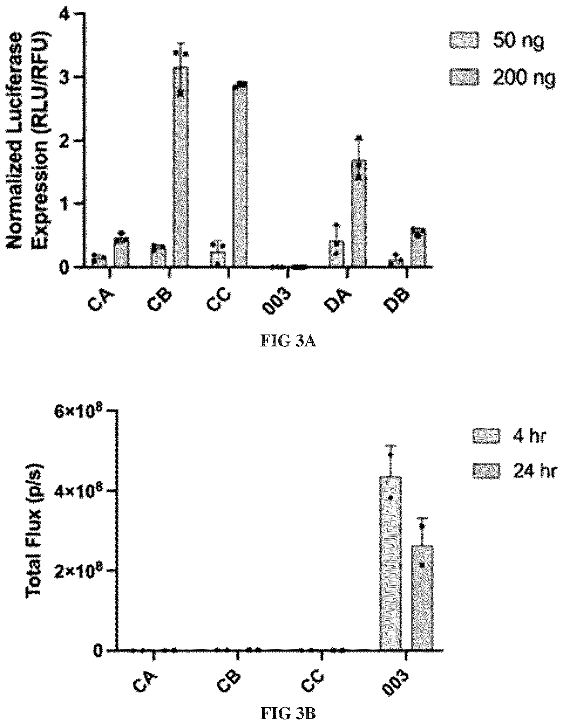

Compound **003** and compounds CA, CB, CC, DA and DB were tested in vitro and compound 003 and CA, CB, CC were tested in vivo. The results are shown in figure 3A and 3B.

| | |
|---|---|
| CA | 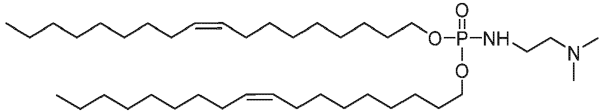 |
| CB | 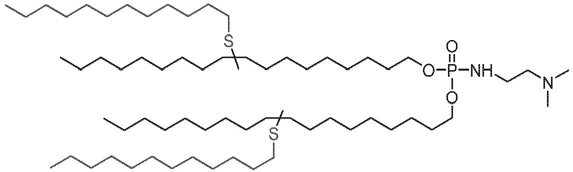 |
| CC | 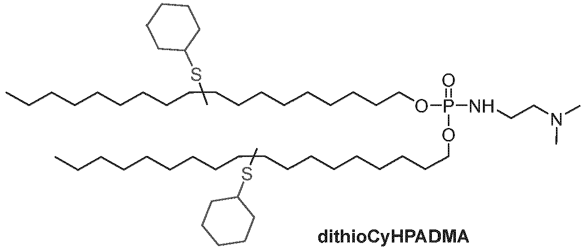 |
| 003 | 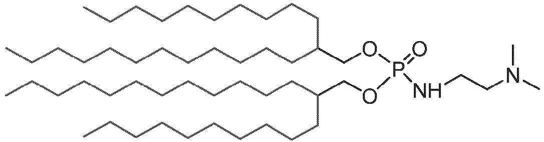 |
| DA | 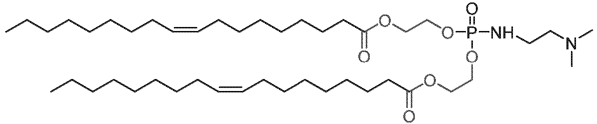 |
| DB | 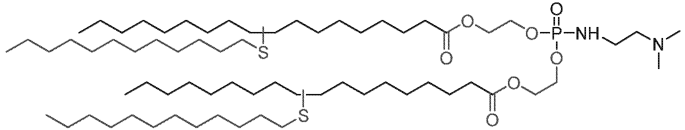 |

The initial tests *in-vitro* and *in-vivo* lead to a surprising result: the only lipid that showed transfection *in-vivo* showed no transfection *in-vitro.* Following this result, all derivatives of that lipid were directly tested *in-vivo* **(****Figure 3****).** The next compounds prepared were inspired from the structure of compound **003.**

All derivatives synthesized were screened and it was found that the most potent lipid was compound **005.** The efficiency of this lipid as compared to lipids used in FDA approved formulations was then determined. It was found that liposomal nanoparticle containing compound 006 were equal to or significantly better than both FDA approved liposomal nanoparticle formulations **(****Figure 10****).** For reference, MC3 (DLin-DMA-MC3) is used in the Onpattro formulation, and SM-102 is used in the Moderna COVID-19 vaccine formulation.

Additionally, the ability of the LNP was tested to deliver Cas9 and sgRNA to knockdown transthyretin (TTR) protein, which is a protein expressed in the liver and excreted to the systemic circulation. Cas9 mRNA and an sgRNA targeting mouse TTR or a non-targeting guide specific to GFP protein were co-encapsulated. Mice were injected with 50 µg of total RNA (25 µg Cas9, 25 µg sgRNA). After 30 days the livers were harvested and next-generation sequencing were performed to determine the editing efficiency. It was found that compound **005** delivered Cas9/sgRNA resulted in up to 62% editing in the liver with the sgRNA that targets TTR, in the control group with a non-targeting guide, that can be found no editing **(****Figure 12****).** The efficacy of the formulation with 005 is more efficient than the standard LP01 at 10 and 20µg of mRNA

## Claims

1. Compound of Formula (I), 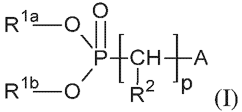 wherein:
**R^{1a}** and **R^{1b},** identical or different, represent, independently from one another, a branched or linear saturated C₅-C₃₅ alkyl chain, optionally substituted by a cyclic or acyclic C₁-C₆ alkyl or by an ester group of formula (C₆-C₁₄)-O-C(O)- , provided that at least one of **R^{1a}** and **R^{1b}** is a branched saturated C₅-C₃₅ alkyl chain;
**R²** represents H, aryl group, heteroaryl group, heterocyclyl group or hydroxyl group;
**p** represents an integer ranging from 0 to 1;
**A** represent -N(R³)(R⁴), -OR⁵ or an heteroaryl group comprising a nitrogen atom;
**R³** represent a hydrogen atom, unsubstituted or substituted alkyl group, or an unsubstituted or substituted aminoalkyl group;
**R⁴** represent a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- or -O-, and terminated by a group selected from substituted or unsubstituted amine group, substituted or unsubstituted heteroaryl group, substituted or unsubstituted heterocyclyl group and phosphoramidate of formula 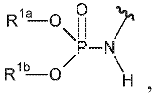 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl; and
wherein **R⁵** represents a ionizable group selected from substituted or unsubstituted heterocyclyl group comprising a nitrogen atom, substituted or unsubstituted heteroaryl group comprising a nitrogen atom, substituted or unsubstituted aryl group, or a linear or branched saturated hydrocarbon chain, the hydrocarbon chain being optionally interrupted by one or more heteroatoms chosen from N, S or O, and terminated by a group selected from substituted or unsubstituted amine group, substituted or unsubstituted heteroaryl group, and substituted or unsubstituted heterocyclyl group.

2. The compound according to claim 1 of Formula (I-A), 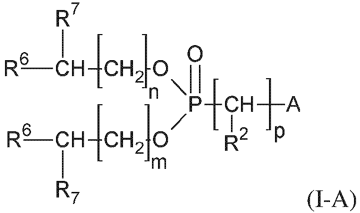 wherein:
**R²**, **R⁵** and **p** are such as defined in claim 1;
**R⁶** and **R⁷**, identical or different, represent, independently from one another, a linear or branched, saturated, C₁-C₂₄ alkyl chain, optionally substituted by a cyclic or acyclic C₁-C₆ alkyl or by an ester group of formula (C₆-C₁₄)-O-C(O)-; and
**n** and **m** are each independently an integer ranging from 0 to 4
**A** represents -N(R³)(R⁴), -OR⁵ or an heteroaryl group comprising a nitrogen atom ;
**R³** represents a hydrogen atom, unsubstituted or substituted alkyl group, or an unsubstituted or substituted aminoalkyl group;
**R⁴** represents a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- and -O-, and terminated by a group selected from substituted or unsubstituted amine group, substituted or unsubstituted heteroaryl group, substituted or unsubstituted heterocyclyl group and phosphoramidate of formula 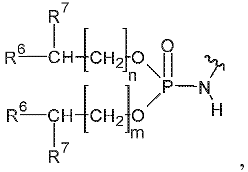 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl.

3. The compound according to claim 2 of Formula (I-B), 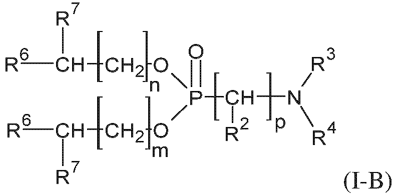 wherein **R²**, **R³, R⁴, R⁶**, **R⁷**, **n, m** and **p** are such as defined in claim **2**.

4. The compound according to any one of claims 2 or 3 of Formula (I-C), 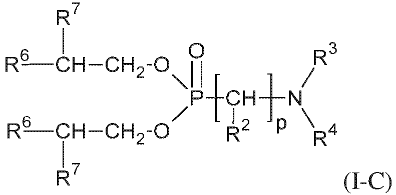 wherein **R²**, **R⁶**, **R⁷** and **p** are such as defined in claim **2** and
**R³** represents a hydrogen atom, unsubstituted or substituted alkyl group, or an unsubstituted or substituted aminoalkyl group;
**R⁴** represents a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- and -O-, and terminated by a group selected from substituted or unsubstituted amine group, substituted or unsubstituted heteroaryl group, substituted or unsubstituted heterocyclyl group and phosphoramidate of formula 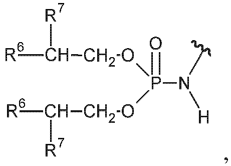 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl.

5. The compound according to any one of claims 2 to 3 of Formula (I-D), 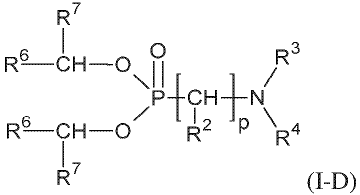
wherein **R²**, **R⁶, R⁷** and **p** are such as defined in claim **2** and
**R³** represents a hydrogen atom, unsubstituted or substituted alkyl group, or an unsubstituted or substituted aminoalkyl group;
**R⁴** represents a neutral group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- and -O-, and terminated by a group selected from substituted or unsubstituted amine group, substituted or unsubstituted heteroaryl group, substituted or unsubstituted heterocyclyl group and phosphoramidate of formula 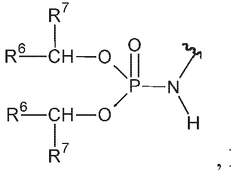 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl.

6. The compound according to any one of claims 1, selected from the group comprising the compounds :
| | |
|---|---|
| **001** | 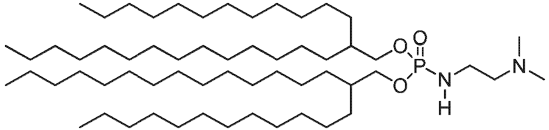 |
| **002** | 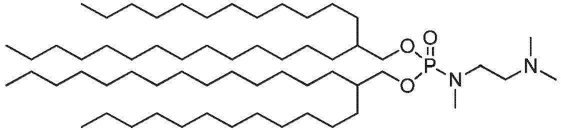 |
| **003** | 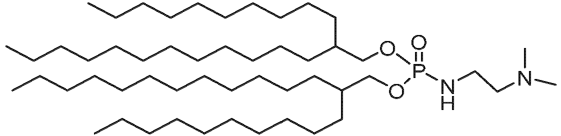 |
| **004** | 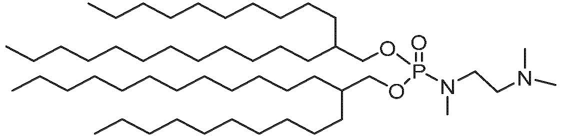 |
| **005** | 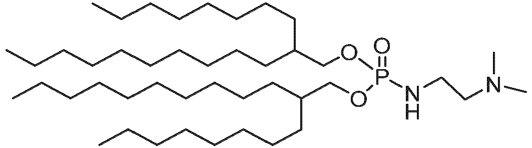 |
| **006** | 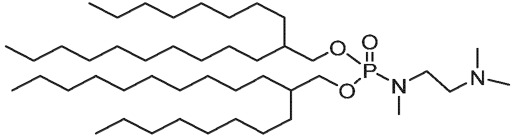 |
| **007** | 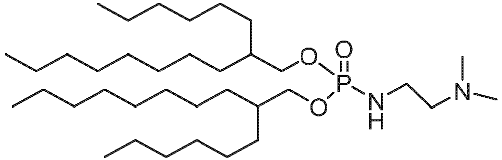 |
| **008** | 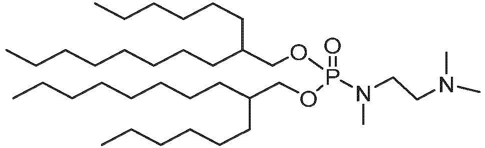 |
| **009** | 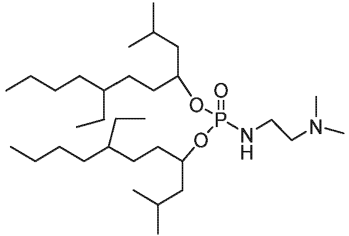 |
| **010** | 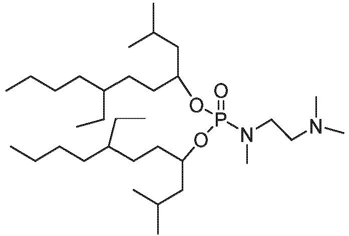 |
| **011** | 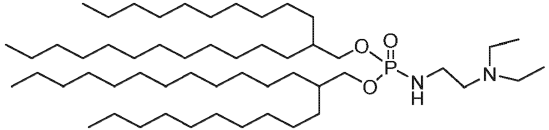 |
| **012** | 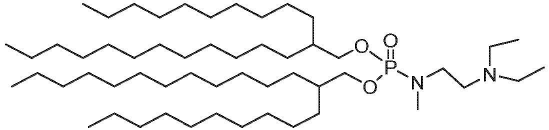 |
| **013** | 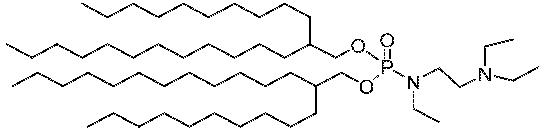 |
| **014** | 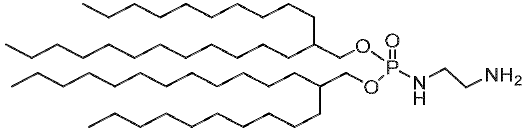 |
| **015** | 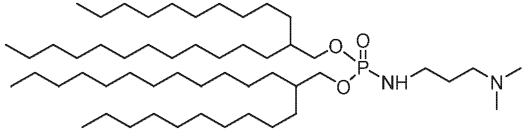 |
| **016** | 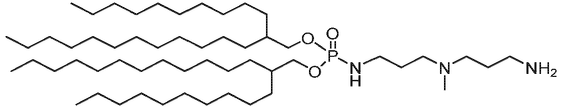 |
| **017** | 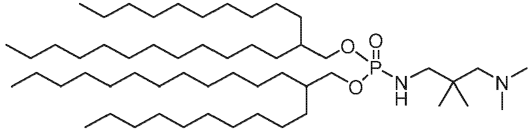 |
| **018** | 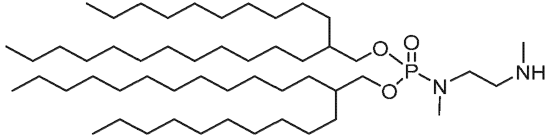 |
| **019** | 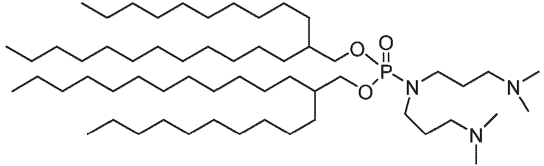 |
| **020** | 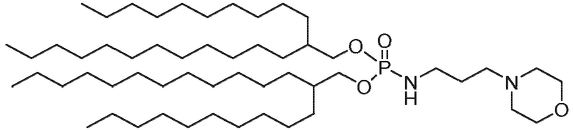 |
| **021** | 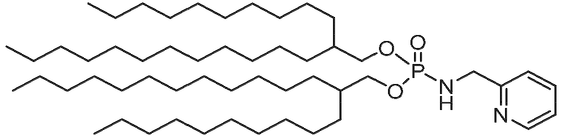 |
| **022** | 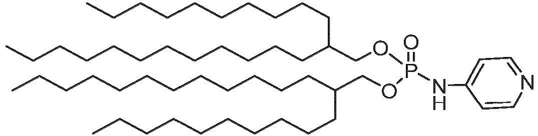 |
| **023** | 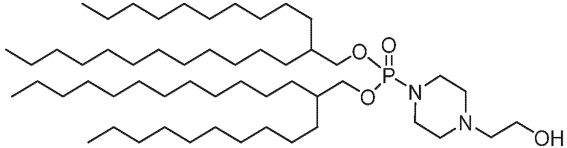 |
| **024** | 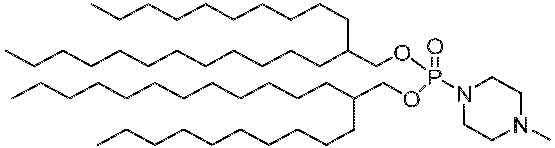 |
| **025** | 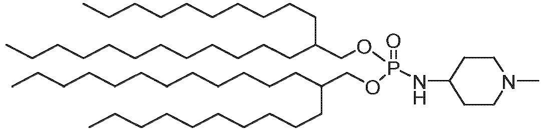 |
| **026** | 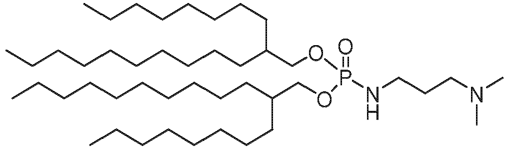 |
| **027** | 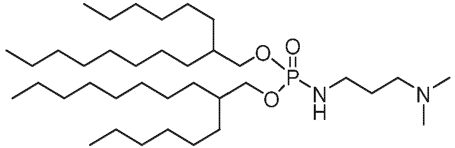 |
| **028** | 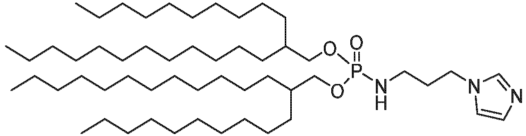 |
| **029** | 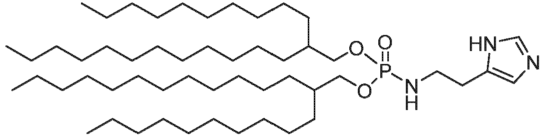 |
| **030** | 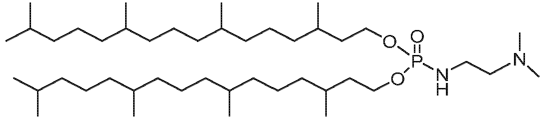 |
| **031** | 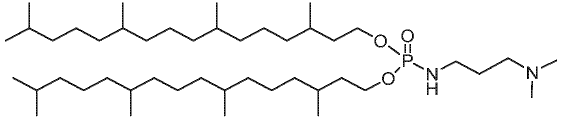 |
| **032** | 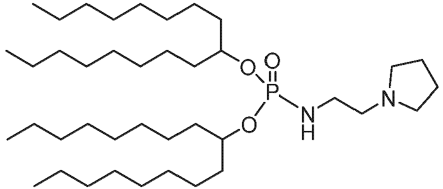 |
| **033** | 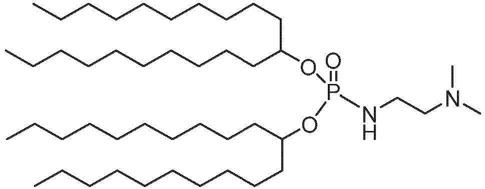 |
| **034** | 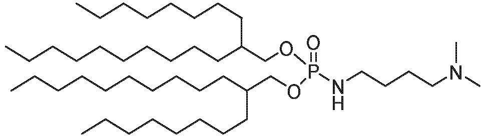 |
| **035** | 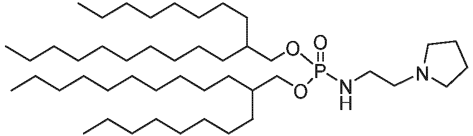 |
| **036** | 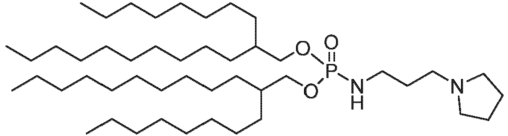 |
| **037** | 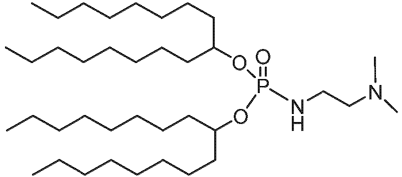 |
| **038** | 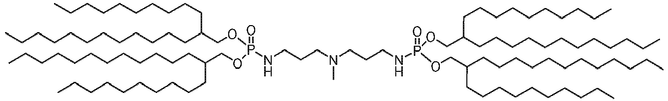 |
| **039** | 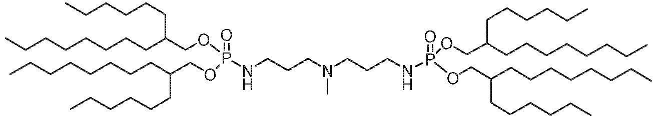 |
| **040** | 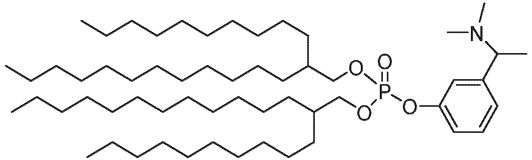 |
| **041** | 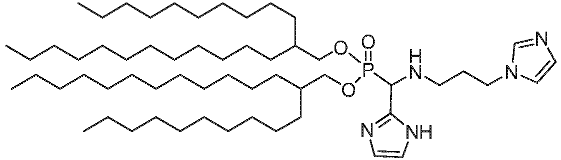 |
| **042** | 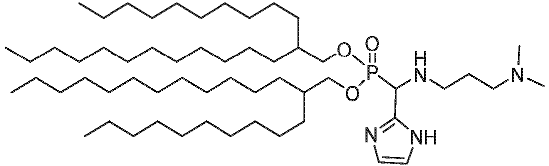 |
| **043** | 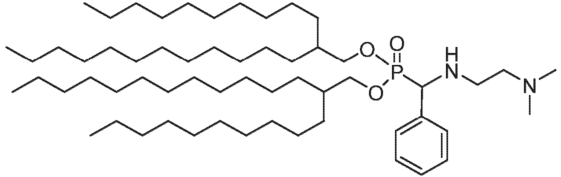 |
| **044** | 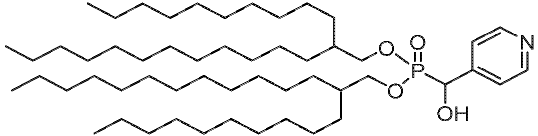 |
| **045** | 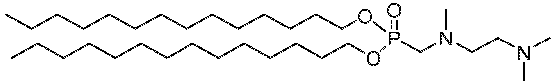 |
| **046** | 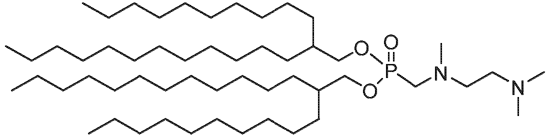 |
| **047** | 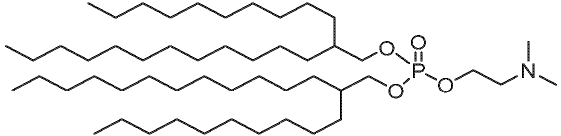 |
| **048** | 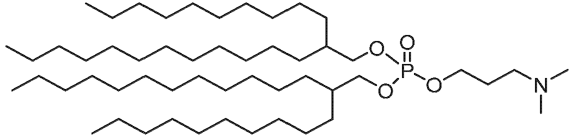 |
| **049** | 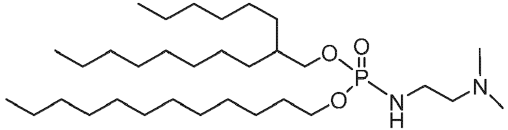 |
| **050** | 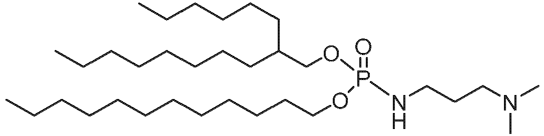 |
| **051** | 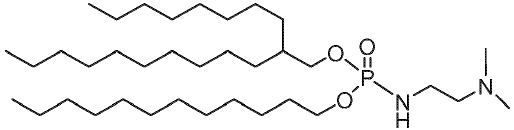 |
| **52** | 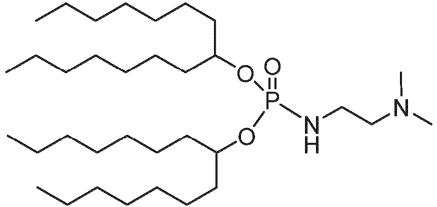 |
| ***53*** | 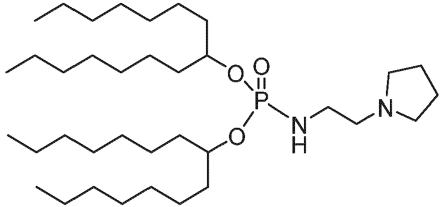 |
| **54** | 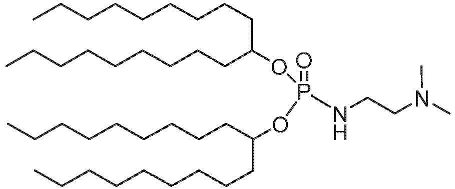 |
| **55** | 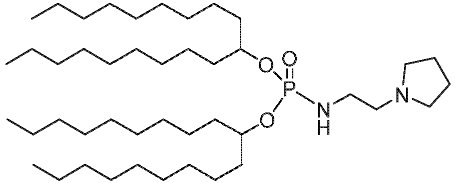 |
| **56** | 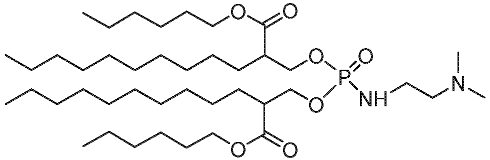 |

7. Process for manufacturing a compound of formula (I) as defined in claim 1: comprising the following step:
Reacting a compound of formula (II) : 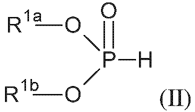
wherein **R^{1a}** and **R^{1b}** identical or different, represent, independently from one another, a branched or linear saturated C₅-C₃₅ alkyl chain, optionally substituted by a cyclic or acyclic C₁-C₆ alkyl or by an ester group of formula (C₆-C₁₄)-O-C(O)- , provided that at least one of **R^{1a}** and **R^{1b}** is a branched saturated C₅-C₃₅ alkyl chain;
with a compound of formula (III) 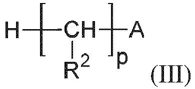 wherein
**R²** represents H, aryl group, heteroaryl group, heterocyclyl group or hydroxyl group;
**p** represents an integer ranging from 0 to 1;
**A** represent -N(R³)(R⁴), -OR⁵ or an heteroaryl group comprising a nitrogen atom;
**R³** represent a hydrogen atom, unsubstituted or substituted alkyl group, or an unsubstituted or substituted aminoalkyl group;
**R⁴** represent a ionizable group selected from substituted or unsubstituted heterocyclyl group comprising a nitrogen atom, substituted or unsubstituted heteroaryl group comprising a nitrogen atom, and a linear or branched saturated hydrocarbon chain, the hydrocarbon chain being optionally interrupted by one or more heteroatoms chosen from -N(R)-, -S- or -O-, and terminated by a group selected from substituted or unsubstituted amine group, substituted or unsubstituted heteroaryl group, substituted or unsubstituted heterocyclyl group and phosphoramidate of formula 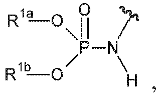 **R** being an alkyl group; or
**R³** and **R⁴** represent together, with the nitrogen atom which carries them, a heterocyclyl group, said heterocyclyl group being optionally interrupted by one or more heteroatoms chosen from N, S or O and/or optionally substituted by one or more groups chosen from alkyl, hydroxyalkyl; and
**R⁵** represent a ionizable group selected from substituted or unsubstituted heterocyclyl group comprising a nitrogen atom, substituted or unsubstituted heteroaryl group comprising a nitrogen atom, substituted or unsubstituted aryl group, or a linear or branched saturated hydrocarbon chain, the hydrocarbon chain being optionally interrupted by one or more heteroatoms chosen from N, S or O, and terminated by a group selected from substituted or unsubstituted amine group, substituted or unsubstituted heteroaryl group, and substituted or unsubstituted heterocyclyl group,
in the presence of an amine.

8. Lipid nanoparticle (LNP) comprising at least one of the compounds according to any of claims 1 to 6

9. The lipid nanoparticle (LNP) according to claim 8, further comprising at least one nucleic acid sequence and at least one PEG-lipid.

10. Process for manufacturing the lipid nanoparticle according to claims 8 or 9 comprising a step of mixing a nucleic acid sequence or a mixture of nucleic acid sequence and a compound according to any of claims 1 to 6 .

11. Pharmaceutical composition comprising a compound according to any of claims 1 to 6 or a lipid nanoparticle according to claims 8 or 9 and a physiologically acceptable vehicle.

12. Use of a compound according to any of claims 1 to 6 or a lipid nanoparticle according to claims 8 or 9 for transfection *in vitro.*

13. Compound according to any of claims 1 to 6 for the use thereof in transfection *in vivo,* for gene editing, base editing, prime editing, gene therapy, vaccination, cancer immunotherapy, topical treatments, ocular treatment or bactericidal activities.

14. Lipid nanoparticle according to claims 8 or 9 for the use thereof in transfection *in vivo,* for gene editing, base editing, gene therapy, vaccination, cancer immunotherapy, topical treatments, bactericidal activities, ex-vivo cell transfection or transfection of organoids, ex-vivo and in vitro transfection.

15. Pharmaceutical composition according to claim 11 for use in the prevention, treatment or amelioration of viral infection (e.g. Zika, HIV, Cytomegalovirus, rabies virus, COVID, influenza, respiratory syncytial virus, zoster virus), genetic disease including rare diseases, cancer (e.g. melanoma, brain cancer, lung cancer, liver cancer, pancreas cancer, prostate cancer, blood system cancer, ovarian cancer, breast cancer, digestive cancer, bone cancer), immunological diseases, metabolic diseases, ocular diseases.
